# EUROPÄISCHE PATENTSCHRIFT

(11) **EP 1 781 656 B1**
(45) Veröffentlichungstag und Bekanntmachung des Hinweises auf die Patenterteilung: **19.12.2007**
(21) Anmeldenummer: 05792575.2
(22) Anmeldetag: 16.08.2005
(51) Int. Cl.: C07D 471/04, C07D 487/08, C07D 487/04, A61K 31/407, A61K 31/437, A61K 31/4375, A61P 3/04, A61P 3/10

(54) **ARYLSUBSTITUIERTE POLYCYCLISCHE AMINE, VERFAHREN ZU IHRER HERSTELLUNG UND IHRE VERWENDUNG ALS ARZNEIMITTEL**
ARYL-SUBSTITUTED POLYCYCLIC AMINES, METHOD FOR THE PRODUCTION THEREOF, AND USE THEREOF AS A MEDICAMENT
AMINES POLYCYCLIQUES SUBSTITUES PAR ARYLE, PROCEDE DE PRODUCTION ASSOCIE ET LEUR UTILISATION EN TANT QUE MEDICAMENTS

(30) Priorität: 16.08.2004 DE 102004039789
(43) Veröffentlichungstag der Anmeldung: 09.05.2007
(73) Patentinhaber: Sanofi-Aventis Deutschland GmbH, 65929 Frankfurt am Main (DE)
(72) Erfinder: SCHWINK, Lothar, 35260 Stadtallendorf (DE); STENGELIN, Siegfried, 65817 Eppstein (DE); GOSSEL, Matthias, 65719 Hofheim (DE); HESSLER, Gerhard, 65719 Hofheim (DE); LENNIG, Petra, 55131 Mainz (DE)
(86) Internationale Anmeldenummer: PCT/EP2005/008888
(87) Internationale Veröffentlichungsnummer: WO 2006/018279

(56) Entgegenhaltungen:
- WO-A-02/10146
- WO-A-03/087044
- WO-A-20/04024702
- WO-A-20/04058736
- WO-A-20/04100946

## Beschreibung

Die Erfindung betrifft arylsubstituierte polycyclische Amine, insbesondere bicyclische Amine, sowie deren physiologisch verträgliche Salze und physiologisch funktionelle Derivate.

Es sind bereits den hier beschriebenen arylsubstituierten polycyclischen Aminen in ihrer Gesamtstruktur ähnliche Verbindungen mit pharmakologischer Wirkung im Stand der Technik beschrieben. In WO95/03302 sind Arylether bicyclischer Aminoalkohole mit Calcium-antagonistischer Wirkung offenbart. WO2002010146 beschreibt einen Amidoarylether von Hydroxychinuclidin als Antagonisten des 11CBY Rezeptors zur Behandlung der Obesitas.

Verbindungen mit MCH-antagonistischer Wirkung zur Behandlung der Obesitas sind im Stand der Technik beschrieben (Beispiele: WO2001021577, WO2003035624, WO2002089729, WO2002006245, WO2002002744, WO2002057233, WO2003045313, WO2003097047, WO2002010146, WO 2003087044).

Der Erfindung lag die Aufgabe zugrunde, Verbindungen zur Verfügung zu stellen, die eine Gewichtsreduktion bei Säugetieren bewirken und die zur Prävention und Behandlung von Obesitas und Diabetes sowie deren vielfältigen Folgeerkrankungen geeignet sind.

Überraschenderweise wurde eine Serie von Verbindungen gefunden, die die Aktivität von MCH-Rezeptoren modulieren. Insbesondere zeichnen sich die Verbindungen durch einen Antagonismus des MCH1R aus.

Die Erfindung betrifft daher Verbindungen der Formel I, 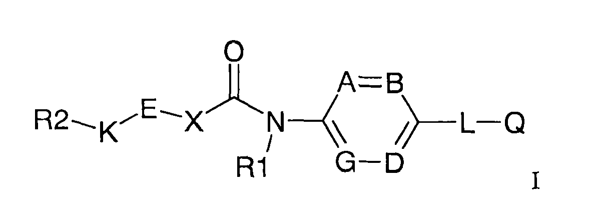 worin bedeuten
- A, B, D, G: unabhängig voneinander N, C(R3);
oder die Gruppen A und B oder die Gruppen D und G sind jeweils C(R3) und bilden gemeinsam einen 5- oder 6-gliedrigen carbocyclischen oder heterocyclischen Rest, so dass sich insgesamt ein bicyclisches System ergibt;
- R3: H, F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, (C₂-C₆)-Alkinyl, (C₀-C₈)-Alkylen-aryl, O-(C₀-C₈)-Alkylen-aryl, S-Aryl, N(R4)(R5), SO₂-CH₃, COOH, COO-(C₁-C₆)-Alkyl, CON(R6)(R7), N(R8)CO(R9), N(R10)SO₂(R11), CO(R12), (CR13R14)ₓ-O(R15);
- R4, R5, R6, R7, R8, R10: unabhängig voneinander H, (C₁-C₈)-Alkyl;
oder
- R4 und R5, R6 und R7: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R9, R11, R12: unabhängig voneinander H, (C₁-C₈)-Alkyl, Aryl;
- R13, R14: unabhängig voneinander H, (C₁-C₈)-Alkyl;
- R15: H, (C₁-C₆)-Alkyl, Aryl;
- x: 0, 1, 2, 3, 4, 5, 6;
- R1: H, (C₁-C₈)-Alkyl, (C₃-C₆)-Alkenyl, (C₃-C₆)-Alkinyl;
- X: N(R16), O, eine Bindung, (R17)C=C(R18), C=C, eine Gruppe der Formel (CR19R20)_{y}, worin eine oder zwei Gruppen (CR19R20) durch Y ersetzt sein können, wobei sich ein chemisch sinnvoller Rest ergibt;
- Y: O, S, N(R21), C=O;
- R16, R17, R18: unabhängig voneinander H, (C₁-C₈)-Alkyl;
- R19,R20: unabhängig voneinander H, (C₁-C₄)-Alkyl, wobei R19 und R20 in den y Gruppen jeweils die gleichen oder verschiedene Bedeutungen aufweisen können;
- y: 1, 2, 3, 4, 5, 6;
- R21: H, (C₁-C₈)-Alkyl;
- E: 3-14 gliedrige bivalente carbo- oder heterocyclische Ringstruktur mit 0-4 Heteroatomen aus der Gruppe N, O und S, die optional Substituenten aus der Gruppe H, F, Cl, Br, I, OH, CF₃, CN, OCF₃, Oxo, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, (C₀-C₈)-Alkylen-aryl, O-(C₀-C₈)-Alkylen-aryl, S-Aryl, N(R22)(R23), SO₂-CH₃, CON(R24)(R25), N(R26)CO(R27), N(R28)SO₂(R29), CO(R30) tragen und mono- oder bicyclisch sein kann, wobei E keine Tetrazol-5-yl-Gruppe ist;
- R22, R23, R24, R25, R26, R28: unabhängig voneinander H, (C₁-C₈)-Alkyl, Aryl;
oder
- R22 und R23, R24 und R25: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R27, R29, R30: unabhängig voneinander H, (C₁-C₈)-Alkyl, Aryl;
- K: eine Bindung, C≡C, (R31)C=C(R32), eine Gruppe der Formel (CR33R34)_{z}, worin eine oder mehrere Gruppen (CR33R34) durch Z ersetzt sein können, wobei sich ein chemisch sinnvoller Rest ergibt, bevorzugt eine Bindung, O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂;
- v: 1, 2, 3, 4;
- R31, R32, R35, R36, R37, R38, R39: unabhängig voneinander H, (C₁-C₈)-Alkyl;
- Z: O, S, N(R40), CO, SO, SO₂;
- R33, R34: unabhängig voneinander H, (C₁-C₈)-Alkyl, Hydroxy-(C₁-C₄)-alkyl, Hydroxy, (C₁-C₄)-Alkoxy-(C₁-C₄)-alkyl, wobei R38 und R39 in den z Gruppen jeweils die gleichen oder verschiedene Bedeutungen aufweisen können;
- z: 1, 2, 3, 4, 5, 6;
- R40: H, (C₁-C₈)-Alkyl;
- R2: H, (C₁-C₈)-Alkyl, (C₁-C₈)-Alkoxy-(C₁-C₄)-alkyl, (C₃-C₈)-Alkenyl, (C₃-C₈)-Alkinyl, ein 3 bis 10-gliedriger mono-, bi-, tri- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R41), CON(R42)(R43), Hydroxy, COO(R44), N(R45)CO(C₁-C₆)-Alkyl, N(R46)(R47), SO₂CH₃, SCF₃ oder S-(C₁-C₆)-Alkyl, wobei R2 keine Tetrazol-5-yl-Gruppe ist;
- R41, R42, R43, R44, R45, R46, R47: unabhängig voneinander H, (C₁-C₈)-Alkyl;
oder
- R42 und R43, R46 und R47: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
- E, K und R2: zusammen einen Tricyclus bilden, wobei die Ringe unabhängig voneinander gesättigt, teilgesättigt oder ungesättigt und jeweils 3 - 8 Ringatome enthalten können;
- L: eine Gruppe der Formel (CR48R49)ₘ, worin eine oder mehrere Gruppen (CR48R49) durch M ersetzt sein können, wobei sich ein chemisch sinnvoller Rest ergibt, wobei L in dem Fall, dass Q 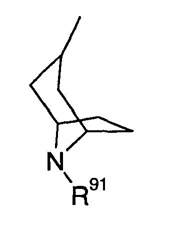 bedeutet, O ist, wobei R91 nachstehend definiert ist;
- M: O, S, N(R50), CO, SO, SO₂, bevorzugt O, S, N(R50), CO, SO₂, besonders bevorzugt O, N(R50), ganz besonders bevorzugt O;
- m: 1, 2, 3, 4, 5, 6, bevorzugt 1, 2, 3, 4, besonders bevorzugt 1, 2;
- R48, R49, R50: unabhängig voneinander H, (C₁-C₆)-Alkyl, Aryl, besonders bevorzugt H, Alkyl;
- Q: 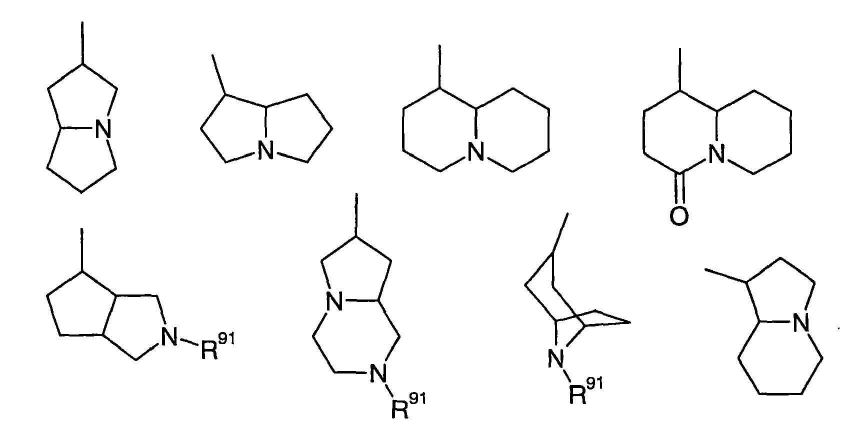
- R91: H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96;
- R92: H, (C₁-C₈)-Alkyl;
- R93, R94: unabhängig voneinander H, (C₁-C₈)-Alkyl, OH, (C₃-C₈)-Cycloalkyl, (C₁-C₄)-Alkoxy-(C₁-C₄)-alkyl;
- o', p': unabhängig voneinander 0, 1, 2, 3, 4, 5, 6;
- R95, R96: unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH;
- R97, R98, R99, R100, R103, R104: unabhängig voneinander H, (C₁-C₈)-Alkyl;
oder
- R97 und R98: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R101, R102: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
- R105, R106, R107, R108, R109, R110, R112, R113, R114, R115, R116, R117, R118, R119: unabhängig voneinander H, (C₁-C₈)-Alkyl;
oder
- R117 und R118: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q', r': unabhängig voneinander 0, 1, 2, 3, 4, 5, 6;
- R108, R111: unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
- R120, R121, R122, R123, R124, R125, R126, R127: unabhängig voneinander H, (C₁-C₈)-Alkyl;
oder
- R120 und R121, R124 und R125: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- deren: N-Oxide sowie deren physiologisch verträgliche Salze.

Die Erfindung bezieht sich auf Verbindungen der Formel I, in Form ihrer Racemate, enantiomerenangereicherten Mischungen und reinen Enantiomere sowie auf ihre Diastereomere und Mischungen davon.

Die Verbindungen der Formel I zeichnen sich dadurch aus, dass sie gegenüber strukturell ähnlichen Verbindungen eine verbesserte metabolische Stabilität bei gleichzeitiger hoher Aktivität aufweisen.

Die Alkyl-, Alkenyl- und Alkinylreste in den Substituenten R1, R2, R3, R4, R5, R6, R7, R8, R9, R10, R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44, R45, R46, R47, R48, R49, R50, R91, R92, R93, R94, R95, R96, R97, R98, R99, R100, R101, R102, R103, R104, R105, R106, R107, R108, R109, R110, R111, R112, R113, R114, R115, R116, R117, R118, R119, R120, R121, R122, R123, R124, R125, R126 und R127 können sowohl geradkettig, verzweigt und/oder optional substituiert sein mit Substituenten wie Aryl, Heteroaryl, Alkoxy oder Halogen. Dies trifft auch zu, sofern die Alkyl-, Alkenyl- und Alkinylreste Teil einer anderen Gruppe sind, z.B. Teil einer Alkoxygruppe (wie (C₁-C₄)-Alkoxy-(C₁-C₄-alkyl)). Geeignete Halogene sind Fluor, Chlor, Brom und Iod, bevorzugt Fluor, Chlor und Brom, besonders bevorzugt Fluor.

Beispiele für Alkylgruppen sind: Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise 1, 2, 3 oder 4 identische oder unterschiedliche Reste, wie Aryl, Heteroaryl, (C₁-C₄)-Alkoxy oder Halogen. Dabei können die zusätzlichen Substituenten in jeder beliebigen Position des Alkylrestes auftreten. Bevorzugt sind die Alkylreste -soweit nicht anders definiert- unsubstituiert.

Unter Cycloalkyl ist im Sinne der vorliegenden Anmeldung Cycloalkyl sowie Cycloalkylalkyl- (Alkyl, das wiederum mit Cycloalkyl substituiert ist) zu verstehen, wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt. Bevorzugt sind die Cycloalkylreste -soweit nicht anders definiert- unsubstituiert.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl.

Unter Cycloalkenyl sind im Sinne der vorliegenden Anmeldung Cycloalkenylreste sowie Cycloalkenyl-Alkylreste (Alkyl, das mit Cycloalkenyl substituiert ist) zu verstehen, die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste und Cycloalkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Für Alkinylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt. Bevorzugt sind die Alkenyl- und Alkinylreste -soweit nicht anders definiert- unsubstituiert.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Die Bezeichnung Aryl umfasst in der vorliegenden Erfindung beispielsweise auch bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl. Bevorzugt sind die Arylreste - soweit nicht anders definiert- unsubstituiert. Besonders bevorzugt ist Aryl Phenyl oder Naphthyl.

Unter Heteroarylresten sind Reste zu verstehen, die von monocyclischen oder bicyclischen Aromaten abgeleitet sind, die Ringheteroatome, bevorzugt N, O oder S, enthalten. Im Übrigen gilt für die Heteroarylreste das bezüglich der Arylreste Aufgeführte.

Unter einem "Tricyclus" werden Strukturen mit 3 Ringen verstanden, die durch mehr als eine Bindung miteinander verbunden sind. Beispiele solcher Systeme sind kondensierte Systeme mit 3 Ringen und Spirocyclen mit ankondensiertem Ringsystem.

Unter die bivalente carbo- oder heterocyclische Ringstruktur E fallen auch Strukturen, die über ein und dasselbe Atom mit den beiden benachbarten Gruppen K und X verknüpft sind.

Unter einer polycyclischen Gruppe ist im Sinne der vorliegenden Anmeldung eine Gruppe zu verstehen, die von Spiranen, kondensierten Ringsystemen oder Brücken-Ringsystemen abgeleitet ist. Die Spirane zeichnen sich dadurch aus, dass zwei Ringe nur ein Kohlenstoffatom gemeinsam aufweisen und die Ringebenen der beiden Ringe senkrecht aufeinander stehen. Bei den kondensierten Ringsystemen sind zwei Ringe so miteinander verknüpft, dass sie zwei Atome gemeinsam aufweisen. Bei dieser Art der Verknüpfung handelt es sich um eine "*ortho*-Kondensation". Bei den Brücken-Ringsystemen handelt es sich um Ringsysteme, die eine Brücke aus Kohlenstoff- und/oder Heteroatomen zwischen zwei nicht benachbarten Atomen eines Rings aufweisen.

Unter einem "chemisch sinnvollen Rest" ist im Sinne der vorliegenden Erfindung ein Rest zu verstehen, der bei Raumtemperatur und Normaldruck stabil ist. Bevorzugt sind im Sinne der vorliegenden Erfindung unter einem "chemisch sinnvollen Rest" in den Definitionen der Gruppen L, X und K in den Verbindungen der Formel (I) Gruppen der Formel (CR48R49)ₘ (in der Definition von L), (CR19R20)_{y} (in der Definition von X) bzw. (CR33R34)_{z} (in der Definition von K) zu verstehen, die keine Heteroatom-Heteroatom-Bindungen zwischen den einzelnen Gruppen (CR48R49), (CR19R20) bzw. (CR33R34) aufweisen.

Die Verbindungen der Formel I können ein oder mehrere Assymmetriezentren enthalten. Daher können die Verbindungen der Formel I in Form ihrer Racemate, Enantiomeren angereicherten Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

Können Reste oder Substituenten mehrfach in den Verbindungen der Formel I auftreten, so können sie alle unabhängig voneinander die angegebenen Bedeutungen haben und gleich oder verschieden sein.

Bevorzugt weist L in den erfindungsgemäßen Verbindungen der Formel I die folgenden Bedeutungen auf:
- L: eine Gruppe der Formel (CR48R49)ₘ, worin eine oder mehrere Gruppen (CR48R49) durch M ersetzt sein können, wobei sich ein chemisch sinnvoller Rest ergibt, wobei L in dem Fall, dass Q 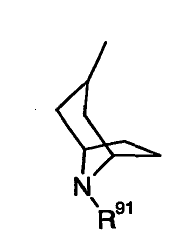 bedeutet, O ist,
- M: O, S, N(R50), CO, SO, SO₂, bevorzugt O, S,' N(R50), CO, SO₂, besonders bevorzugt O, N(R50), ganz besonders bevorzugt O;
- m: 1, 2, 3, 4, 5, 6, bevorzugt 1, 2, 3, 4, besonders bevorzugt 1, 2;
- R48, R49, R50: unabhängig voneinander H, (C₁-C₆)-Alkyl, Aryl, bevorzugt H, (C₁-C₆)-Alkyl, besonders bevorzugt H.

Bevorzugt weisen die Symbole in den Verbindungen der Formel I die folgenden Bedeutungen auf:
- A, B, D, G: unabhängig voneinander N, C(R3) oder die Gruppen A und B oder D und G sind jeweils C(R3) und bilden gemeinsam eine ortho-Phenyleneinheit, so dass sich insgesamt ein 1,4-bisubstituiertes Naphthalinsystem ergibt; bevorzugt unabhängig voneinander N oder C(R3), wobei die Gesamtzahl der Stickstoffatome in dem Ring 0 - 2, bevorzugt 0 oder 1 beträgt, besonders bevorzugt sind A, B, D und G C(R3);
- R3: H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀ - C₈)-Alkylen-aryl, N(R4)(R5), SO₂-CH₃, CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)(R14))ₓ-O(R15); ganz besonders bevorzugt H, F, (C₁-C₆)-Alkyl; weiter bevorzugt H, F, CH₃; insbesondere bevorzugt H;
- R4,R5,R6,R7,R8: unabhängig voneinander H, (C₁-C₈)-Alkyl;
oder
- R4 und R5, R6 und R7: unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R9, R12: unabhängig voneinander H, (C₁-C₈)-Alkyl;
- R13, R14: H;
- R15: H, (C₁-C₆)-Alkyl;
- x: 0, 1, 2, bevorzugt 0,1, besonders bevorzugt 1;
- R1: H, (C₁-C₈)-Alkyl;
- X: N(R16), eine Bindung, (R17)C=C(R18), C≡C, CH₂-CH₂, YCH₂, CH₂Y, bevorzugt N(R16), eine Bindung;
- Y: O, S, N(R21);
- R16, R17, R18: unabhängig voneinander H, (C₁-C₈)-Alkyl;
- R21: H, (C₁-C₈)-Alkyl;
- E: 3-8 gliedrige bivalente carbo- oder heterocyclische Ringstruktur mit 0-4 Heteroatomen aus der Gruppe N, O und S, die optional Substituenten aus der Gruppe H, F, Cl, Br, OH, CF₃, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, O-(C₃-C₈)-Cycloalkyl, (C₃-C₈)-Cycloalkenyl, (C₂-C₆)-Alkinyl, (C₀-C₈)-Alkylen-aryl, O-(C₀-C₈)-Alkylen-aryl, S-Aryl, N(R22)(R23), SO₂-CH₃, N(R26)CO(R27), N(R28)SO₂(R29), CO(R30) tragen und mono- oder bicyclisch sein kann, wobei E keine Tetrazol-5-yl-Gruppe ist;
bevorzugt 5-7 gliedrige bivalente carbo- oder heterocyclische Ringstruktur mit 0-3 Heteroatomen aus der Gruppe N, O und S, die optional Substituenten aus der Gruppe H, F, Cl, Br, OH, CF₃, CN, OCF₃, O-(C₁-C₆)-Alkyl, S-(C₁-C₆)-Alkyl, (C₁-C₆)-Alkyl, (C₂-C₆)-Alkenyl, O-(C₀-C₈)-Alkylen-aryl, S-Aryl, N(R22)(R23), SO₂-CH₃, N(R26)CO(R27), CO(R30) tragen und mono- oder bicyclisch sein kann; besonders bevorzugt 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl tragen kann; z.B. ist E ausgewählt aus der Gruppe bestehend aus 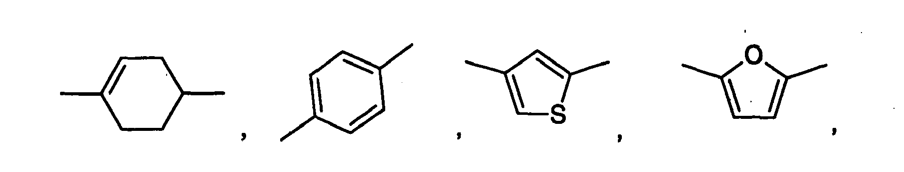 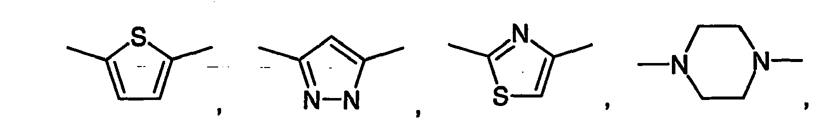 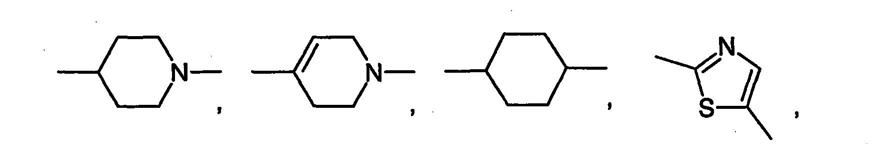 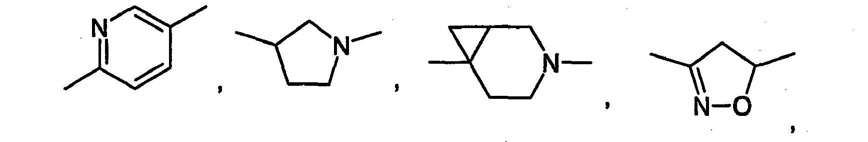 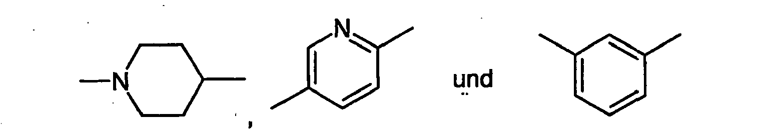
die optional Substituenten aus der Gruppe H, F, Cl, Br, OH, CF₃, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl tragen können; bevorzugt 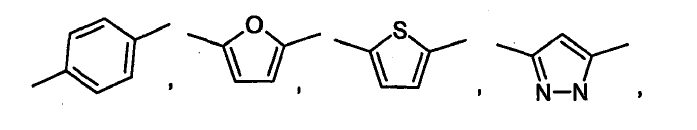 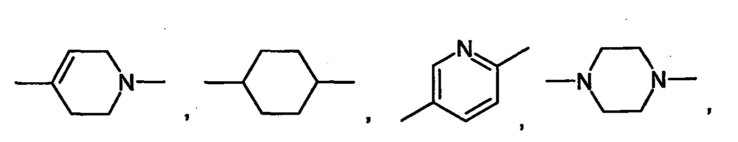 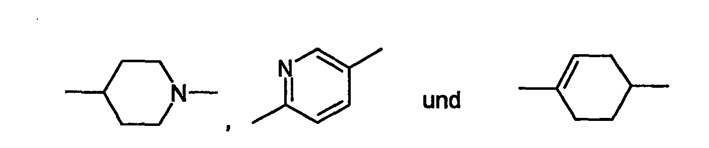 die optional die vorstehend genannten Substituenten tragen können;
- R22, R23, R26, R28: unabhängig voneinander H, (C₁-C₈)-Alkyl;
oder
- R22 und R23: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R27, R29, R30: unabhängig voneinander H, (C₁-C₈)-Alkyl;
- K: eine Bindung, O, OCH₂, CH₂O, S, SO, SO₂, N(R35), N(R36)CO, N-SO₂, CON(R37), (C(R38)(R39))ᵥ, CO, (R31)C=C(R32), C≡C, SCH₂, SO₂CH₂, bevorzugt eine Bindung, O, OCH₂, CH₂O, S, SO, SO₂, N(R35), N(R36)CO, CON(R37), (C(R38)(R39))ᵥ, CO, (R31)C=C(R32), C=C, SCH₂, SO₂CH₂, besonders bevorzugt OCH₂, CH₂O, N(R36)CO, CON(R37), (C(R38)(R39))₂, (R31)C=C(R32), C≡C, SCH₂, SO₂CH₂, ganz besonders bevorzugt OCH₂, CH₂O, CON(R37), C=C, SCH₂;
- v: 1, 2, 3, bevorzugt 2;
- R31, R32, R35, R36, R37, R38, R39: unabhängig voneinander H, (C₁-C₈)-Alkyl;
- R2: (C₁-C₈)-Alkyl, (C₁-C₄)-Alkoxy-(C₁-C₄)-alkyl, ein 3 bis 10-gliedriger mono-, bi-, tri- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, (C₀-C₂)-Alkylen-aryl, Oxo, CO(R41), CON(R42)(R43), Hydroxy, N(R45)CO(C₁-C₆)-Alkyl, N(R46)(R47) oder SO₂CH₃; bevorzugt (C₁-C₈)-Alkyl, (C₁-C₄)-Alkoxy-(C₁-C₄)-alkyl, ein 3 bis 10-gliedriger mono- oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, Oxo, CO(R41), CON(R42)(R43), N(R45)CO(C₁-C₆)-Alkyl oder SO₂CH₃;
- R41,: R42, R43, R45, R46, R47 unabhängig voneinander H, (C₁-C₈)-Alkyl;
oder
- R42 und R43, R46 und R47: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- L: eine Gruppe der Formel (C(R48)(R49))ₘ, in der 0 oder 1 Glied ersetzt sein kann durch ein Element aus der Gruppe O, N(R50), bevorzugt O, wobei L in dem Fall, dass Q 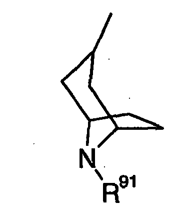 bedeutet, O ist,
- m: 1, 2, 3, 4, bevorzugt 1 oder 2;
- R48, R49: H;
- R50: H, (C₁-C₈)-Alkyl, bevorzugt H;
- Q: 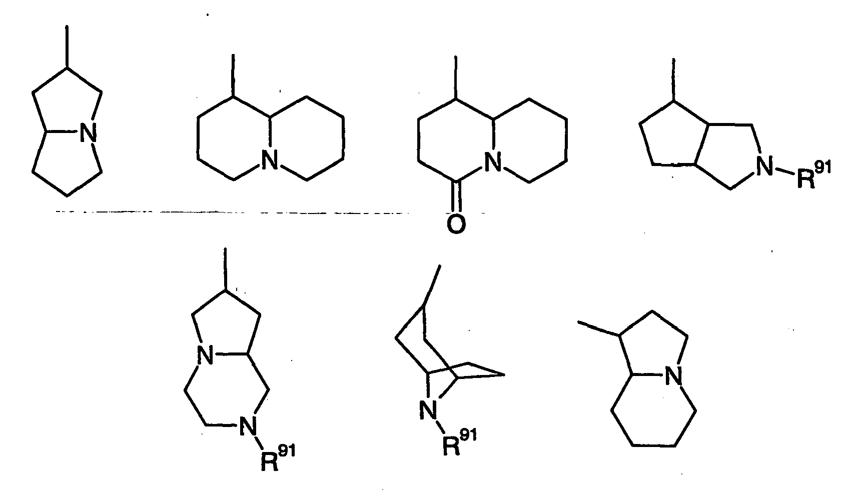
- R91: H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96; bevorzugt H, (C₁-C₆)-Alkyl, (CR93R94)_{o'}-R95;
- R92: H, (C₁-C₈)-Alkyl;
- R93, R94: unabhängig voneinander H, (C₁-C₈)-Alkyl, OH, (C₃-C₈)-Cycloalkyl, (C₁-C₄)-Alkoxy-(C₁-C₄)-alkyl, bevorzugt H, (C₁-C₈)-Alkyl;
- o', p': unabhängig voneinander 0, 1, 2, 3, 4, 5, 6, bevorzugt 0, 1, 2, 3;
- R95, R96: unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH; bevorzugt unabhängig voneinander OH, O-(C₁-C₈)-Alkyl, CON(R97)(R98), ein 4-6 gliedriges Ringsystem mit 0 bis 2 Heteroatomen ausgewählt aus der Gruppe N und O, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH, wobei das Ringsystem besonders bevorzugt unsubstituiert ist;
- R97, R98, R99, R100, R103, R104: unabhängig voneinander H, (C₁-C₈)-Alkyl;
oder
- R97 und R98: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R101, R102: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
oder
R105, R106, R107, R108, R109, R110, R112, R113, R114, R115, R116, R117, R118, R119 unabhängig voneinander H, (C₁-C₈)-Alkyl;
- R117 und R118: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q', r': unabhängig voneinander 0, 1, 2, 3, 4, 5, 6;
- R108, R111: unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
- R120, R121, R122, R123, R124, R125, R126, R127: unabhängig voneinander H, (C₁-C₈)-Alkyl;
oder
- R120 und R121, R124 und R125: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

Besonders bevorzugt sind Verbindungen der Formel I, worin
- A, B, D, G: unabhängig voneinander N oder C(R3) bedeuten und die Gesamtzahl der Stickstoffatome in diesem Ring 0-2, bevorzugt 0 oder 1, besonders bevorzugt 0 beträgt.

Die Verknüpfung zwischen der Gruppe Q und der Gruppe 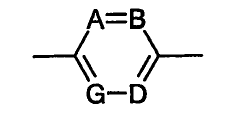 erfolgt in den Verbindungen der Formel I bevorzugt über ein Heteroatom, besonders bevorzugt O oder N, ganz besonders bevorzugt O des Linkers L.

Besonders bevorzugt weist die Gruppe Q in den Verbindungen der Formel I die folgenden Bedeutungen auf: 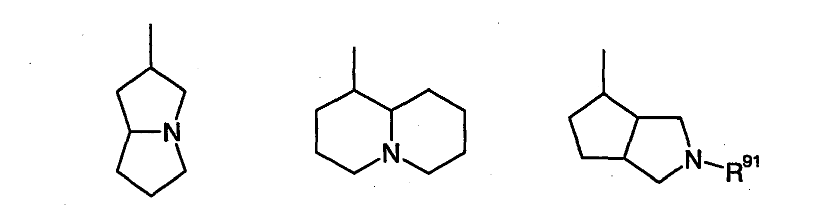 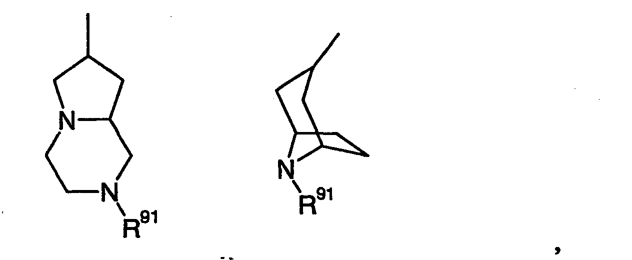 bevorzugt 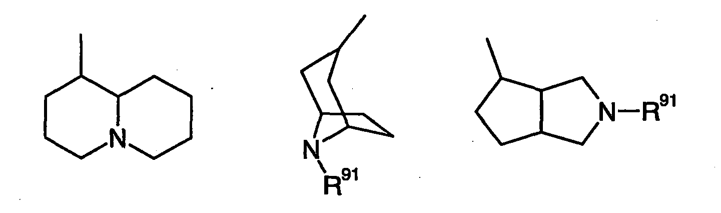
- R91: H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96; bevorzugt H, (C₁-C₆)-Alkyl, (CR93R94)_{o'}-R95;
- R92: H, (C₁-C₈)-Alkyl;
- R93, R94: unabhängig voneinander H, (C₁-C₈)-Alkyl, OH, (C₃-C₈)-Cycloalkyl, (C₁-C₄)-Alkoxy-(C₁-C₄)-alkyl, bevorzugt H, (C₁-C₈)-Alkyl;
- o', p': unabhängig voneinander 0, 1, 2, 3, 4, 5, 6, bevorzugt 0, 1, 2, 3;
- R95,: R96 unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH; bevorzugt unabhängig voneinander OH, O-(C₁-C₈)-Alkyl, CON(R97)(R98), ein 4-6 gliedriges Ringsystem mit 0 bis 2 Heteroatomen ausgewählt aus der Gruppe N und O, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH, wobei das Ringsystem besonders bevorzugt unsubstituiert ist;
- R97, R98, R99, R100, R103, R104: unabhängig voneinander H, (C₁-C₈)-Alkyl;
oder
- R97 und R98: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R101, R102: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
- R105, R106, R107, R108, R109, R110, R112, R113, R114, R115, R116, R117, R118, R119: unabhängig voneinander H, (C₁-C₈)-Alkyl;
oder
- R117 und R118: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q', r': unabhängig voneinander 0, 1, 2, 3, 4, 5, 6;
- R108, R111: unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
- R120, R121, R122, R123, R124, R125, R126, R127: unabhängig voneinander H, (C₁-C₈)-Alkyl;
oder
- R120 und R121, R124 und R125: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

Ganz besonders bevorzugt bedeutet Q in den Verbindungen der Formel I

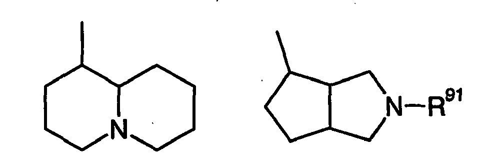

In einer weiteren ganz besonders bevorzugten Ausführungsform weist Q die folgende Bedeutung auf: 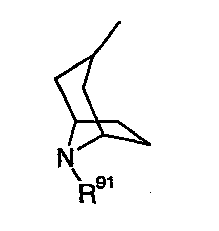 wobei R91 die vorstehend genannten Bedeutungen aufweist und L O bedeutet.

L bedeutet in den Verbindungen der Formel I bevorzugt O oder CH₂O besonders bevorzugt O, wobei wobei L in dem Fall, dass Q 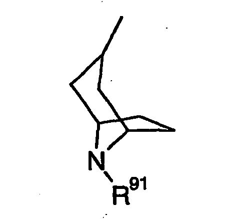 bedeutet, O ist.

Ganz besonders bevorzugt sind Verbindungen der Formel I, worin L-Q die folgenden Bedeutungen aufweist: 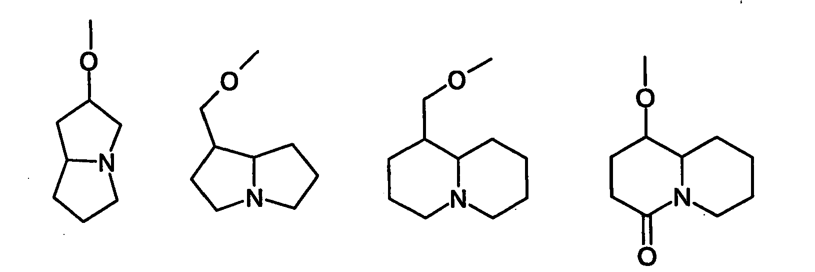 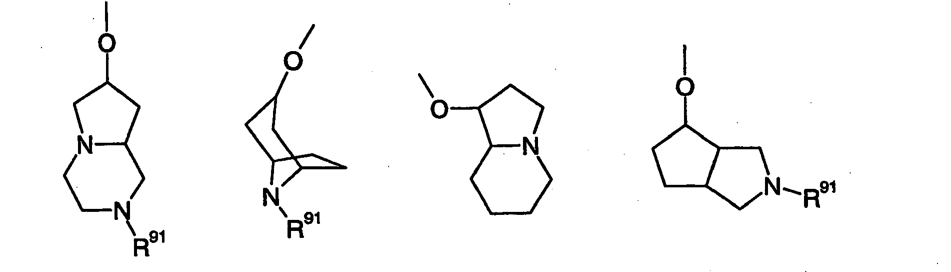 bevorzugt 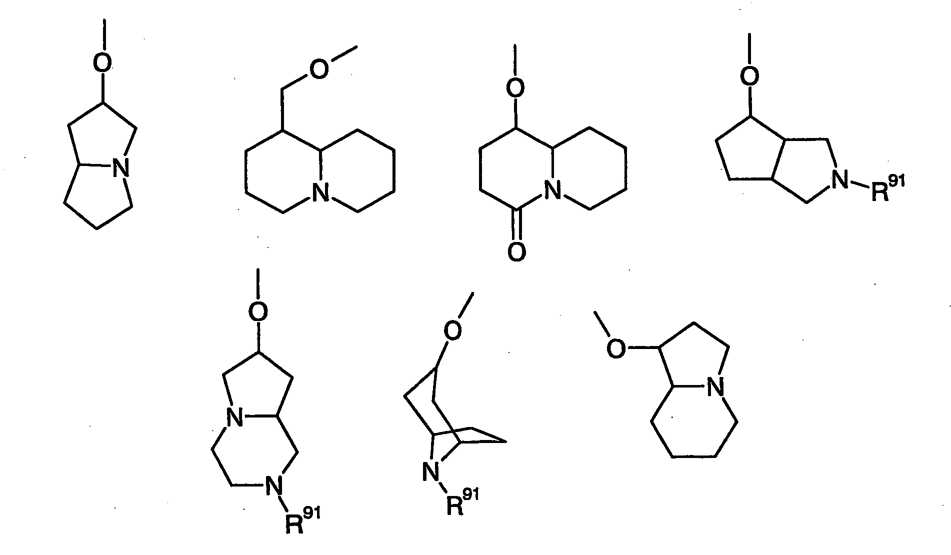 besonders bevorzugt 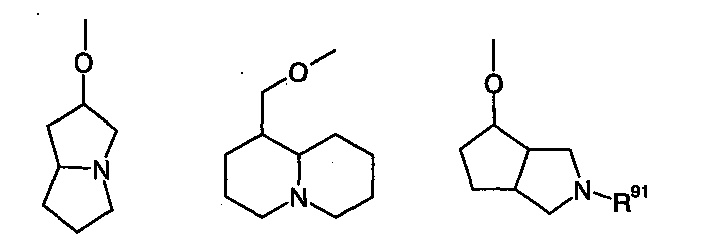 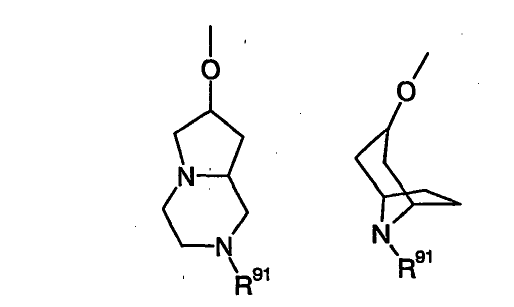 ganz besonders bevorzugt 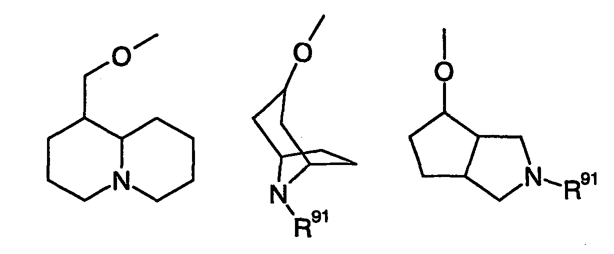
- R91: H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96; bevorzugt H, (C₁-C₆)-Alkyl, (CR93R94)ₒ,-R95;
- R92: H, (C₁-C₈)-Alkyl;
- R93, R94: unabhängig voneinander H, (C₁-C₈)-Alkyl, OH, (C₃-C₈)-Cycloalkyl, (C₁-C₄)-Alkoxy-(C₁-C₄)-alkyl, bevorzugt H, (C₁-C₈)-Alkyl;
- o', p': unabhängig voneinander 0, 1, 2,3,4,5,6, bevorzugt 0,1,2,3;
- R95, R96: unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH; bevorzugt unabhängig voneinander OH, O-(C₁-C₈)-Alkyl, CON(R97)(R98), ein 4-6 gliedriges Ringsystem mit 0 bis 2 Heteroatomen ausgewählt aus der Gruppe N und O, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH, wobei das Ringsystem besonders bevorzugt unsubstituiert ist;
- R97, R98, R99, R100, R103, R104: unabhängig voneinander H, (C₁-C₈)-Alkyl;
oder
- R97 und R98: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R101, R102: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
- R105, R119 R106, R107, R108, R109, R110, R112, R113, R114, R115, R116, R117, R118,: unabhängig voneinander H, (C₁-C₈)-Alkyl;
oder
- R117 und R118: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q', r': unabhängig voneinander 0,1, 2, 3, 4, 5, 6;
- R108, R111: unabhängig voneinander OH, O-(C₁-C₈)-Alkyl, CON(R120)(R121), N(R122)CO(R123), N(R124)(R125), CO₂(R126), SO₂Me CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
- R120, R121, R122, R123, R124, R125, R126, R127: unabhängig voneinander H, (C₁-C₈)-Alkyl;
oder
- R120 und R121, R124 und R125: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

In einer besonders bevorzugten Ausführungsform der vorliegenden Erfindung bedeutet die Gruppe L-Q in den Verbindungen der Formel I

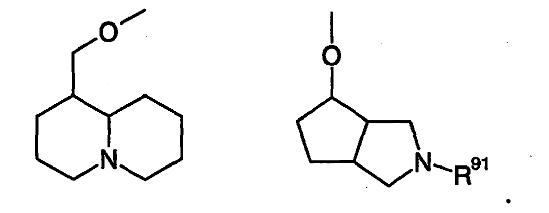

In einer weiteren bevorzugten Ausführungsform bedeutet die Gruppe L-Q in den Verbindungen der Formel I 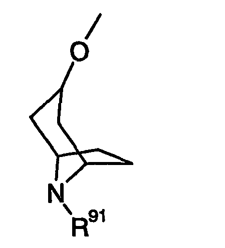 wobei R91 die vorstehend genannten Bedeutungen aufweist.

Des Weiteren betrifft die vorliegende Erfindung Verbindungen der Formel I worin
- A,B,D,G: unabhängig voneinander N oder C(R3) bedeuten und die Gesamtzahl der Stickstoffatome in diesem Ring 0-2, bevorzugt 0 oder 1, besonders bevorzugt 0 beträgt;
wobei die weiteren Symbole in Formel I bereits vorstehend definiert wurden.

In einer bevorzugten Ausführungsform betrifft die vorliegende Anmeldung Verbindungen der Formel I
worin bedeuten
- A, B, D, G: C(R3);
- R3: H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀ - C₈)-Alkylen-aryl, N(R4)(R5), SO₂-CH₃ CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)(R14))ₓ-O(R15); ganz besonders bevorzugt H, F, (C₁-C₆)-Alkyl; weiter bevorzugt H, F, CH₃; insbesondere bevorzugt H;
- R4, R5, R6, R7, R8: unabhängig voneinander H, (C₁-C₈)-Alkyl;
oder
- R4 und R5, R6 und R7: bilden unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
- R9, R12: unabhängig voneinander H, (C₁-C₈)-Alkyl;
- R13, R14: H;
- R15: H, (C₁-C₆)-Alkyl;
- x: 0, 1, 2, bevorzugt 0, 1, besonders bevorzugt 1.

In einer weiteren bevorzugten Ausführungsform bedeuten A, B, G und D in den Verbindungen der Formel I CH.

R2 ist bevorzugt ausgewählt aus der Gruppe bestehend aus:
n-Propyl, n-Butyl, iso-Butyl, iso-Pentyl, Cyclopropyl, Cyclobutyl, Cyclopentyl, Cyclohexyl, Cyclohex-(1)-enyl, Phenyl, p-Fluorophenyl, p-Chlorophenyl, p-Bromophenyl, p-Tolyl, p-Methoxyphenyl, p-Trifluoromethylphenyl, 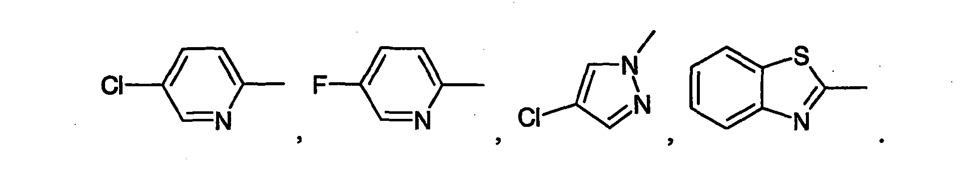 K ist bevorzugt ausgewählt aus der Gruppe bestehend aus:
O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂, bevorzugt OCH₂, CH₂O, N(R36)CO, CON(R37), (C(R38)(R39))₂, (R31)C=C(R32), C=C, SCH₂, SO₂CH₂, besonders bevorzugt OCH₂, CH₂O, CON(R37), C≡C, SCH₂; wobei
- v: 1, 2, 3, bevorzugt 2;
- R31, R32, R35, R36, R37, R38, R39: unabhängig voneinander H, (C₁-C₈)-Alkyl bedeuten

X ist bevorzugt ausgewählt aus der Gruppe bestehend aus Bindung und N(R16) bedeutet, worin R16 H oder (C₁-C₈)-Alkyl bedeutet, besonders bevorzugt Bindung und NH.

Die Gruppe E in den Verbindungen der Formel I ist vorstehend definiert. Gemäß den vorstehenden Definitionen für E kann E z.B. ein fünf- oder sechsgliedriger Ring sein. Handelt es sich bei der Gruppe E um einen fünfgliedrigen Ring, so sind die Gruppen K und X in den Verbindungen der Formel I in einer bevorzugten Ausführungsform in der 1- und 3-Position des fünfgliedrigen Rings angeordnet. Handelt es sich bei der Gruppe E um einen sechsgliedrigen Ring, so sind die Gruppen K und X in einer bevorzugten Ausführungsform in der 1- und 4-Position (d.h. in para-Stellung zueinander) des sechsgliedrigen Rings angeordnet.

E ist besonders bevorzugt ausgewählt aus der Gruppe bestehend aus:

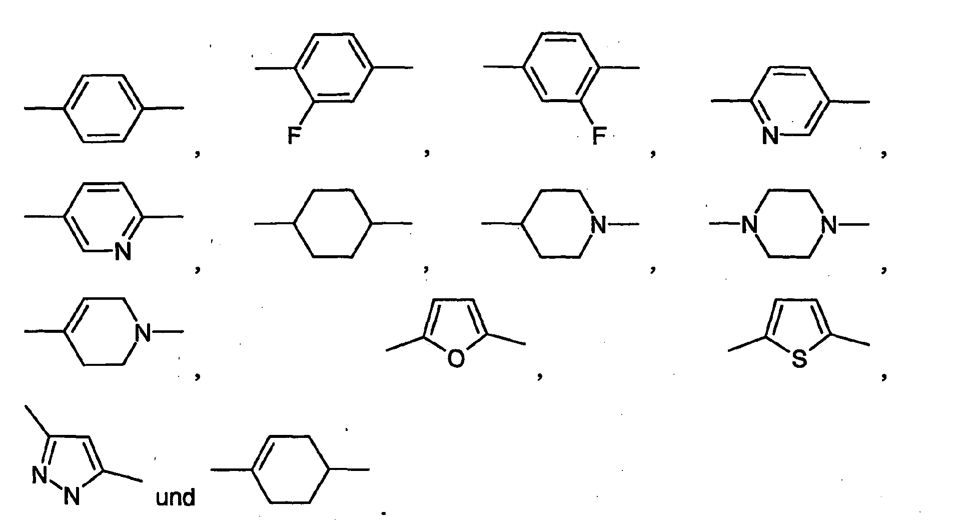

Diese Erfindung bezieht sich weiterhin auf die Verwendung von Verbindungen der Formel I und ihren pharmazeutischen Zusammensetzungen als MCH-Rezeptor-Liganden. Die erfindungsgemäßen MCH-Rezeptor-Liganden eignen sich insbesondere als Modulatoren der Aktivität des MCH1R.
Die Rolle von MCH in der Regulation des Energiehaushalts ist inzwischen gut dokumentiert (Qu, D. et al.; Nature 1996, 380, 243-7; Shimada, M. et al. Nature 1998, 396, 670-4; Chen, Y. et al. Endocrinology 2002, 143, 2469-77; Endocrinology 2003,144,4831-40; Übersicht: G. Hervieu, Expert Opin. Ther. Targets 2003,7,495-511).
Auch gibt es Hinweise, dass MCH-Antagonisten zentral bedingte Störungen wie z.B. Depressionen günstig beeinflussen können (Borowsky, B. et al.; Nature Medicine 2002, 8, 825-30; Übersicht: G. Hervieu, Expert Opin. Ther. Targets 2003, 7, 495-511).
Besonders geeignet sind solche Verbindungen zur Behandlung und/oder Prävention von
1. Obesitas
2. Diabetes mellitus, insbesondere Typ 2 Diabetes einschließlich der Verhinderung der damit verbundenen Folgeerkrankungen.

Besondere Aspekte sind dabei die
- Hyperglykämie,
- Verbesserung der Insulinresistenz,
- Verbesserung der Glukose-Toleranz,
- Schutz der β-Zellen der Bauchspeicheldrüse
- Verhinderung makro- und mikrovaskulärer Erkrankungen

3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid- Konzentrationen
   - niedrige HDL-Cholesterin Konzentration
4. Verschiedene andere Zuständen, die mit dem Metabolischen Syndrom assoziert sein können, sind wie:
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt , hypertensive Herzerkrankung oder Kardiomyopathie
5. Psychiatrische Indikationen wie
   - Depressionen
   - Angstzustände
   - Störungen des zirkadianen Rhythmus
   - Affektionsstörungen
   - Schizophrenie
   - Suchtkrankheiten

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg bis50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich durch ihre geringe Toxizität und ihre geringen Nebenwirkungen aus. Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I in Kombination mit Insulin verabreicht.

Bei einer Ausführungsform der Erfindung werden die Verbindungen der Formel I in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188).

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid. Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel I in Kombination mit einem PPARgamma Agonist, wie z.B. Rosiglitazon, Pioglitazon, verabreicht.

Bei einer Aufführungsform werden die Verbindungen der Formel I in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin.
Bei noch einer Ausführungsform ist der weitere Wirkstoff Sibutramin.
Bei einer anderen Ausführungsform ist der weitere Wirkstoff Rimonabant.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo- 2,3,3a,4,6,7-hexahydro-pyrazolo[4,3-c]pyridin-5-yl)-1-(4-chloro-phenyl)-2-oxo-ethyl]- amid; (WO 01/91752)) , Orexin-Antagonisten (z.B. 1-(2-Methyl-benzoxazol-6-yl)-3-[1,5]naphthyridin-4-yl-harnstoff; hydrochloride (SB-334867-A)), CB1-Antagonisten / Inversen Agonisten, H3-Antagonisten / Inversen Agonisten (z.B. 3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethylphenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino)-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), BRS3-Agonisten, Galanin-Antagonisten, Ghrelin-Antagonisten, MCH-Antagonisten, mGluR5-Antagonisten, Opioid-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), CNTF, CNTF-Derivaten (z.B. Axokine), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin. Bei einer Ausführungsform werden die Verbindungen der Formel I in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

### Die Wirksamkeit der Verbindungen wurde wie folgt getestet:

Die Klonierung der cDNA für den humanen MCH-Rezeptor, Herstellung einer rekombinanten HEK293-Zellinie, welche den humanen MCH-Rezeptor exprimiert, sowie funktionelle Messungen mit der rekombinanten Zellinie erfolgten sinngemäß wie von Audinot et al. (J. Biol. Chem. 276, 13554-13562, 2001) beschrieben. Im Unterschied zur Literaturstelle wurde jedoch für die Konstruktion des Expressionsvektors das Plasmid pEAK8 der Fa. EDGE Biosystems (USA) verwendet. Als Wirt für die Transfektion diente eine transformierte HEK-Zellinie namens "PEAK Stable Cells" (ebenfalls von EDGE Biosystems). Die funktionellen Messungen des zellulären Calciumflusses nach Agonistenzugabe (MCH) in Gegenwart von erfindungsgemäßem Ligand erfolgten mit Hilfe des FLIPR-Gerätes der Fa. Molecular Devices (USA), unter Verwendung von Vorschriften des Geräteherstellers.

### Biologisches Prüfmodell

Die Prüfung der anorektischen Wirkung erfolgte an weiblichen NMRI Mäusen. Nach 17stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit den Vehikel-behandelten Kontrolltieren verglichen.

**Tabelle 1: Anorektische Wirkung betreffend Verbindungen der Formel I gemessen als Reduktion des kumulierten Milchkonsums behandelter im Vergleich zu Kontrolltieren.**

| Beispiel | Orale Dosis [mg/kg] | Reduktion des kumulierten Milchkonsums in % der Kontrolle |
|---|---|---|
| 2 | 30 | 73 |
| 37 | 30 | 24 |

Die nachfolgend aufgeführten Beispiele und Herstellungsmethoden dienen zur Erläuterung der Erfindung, ohne diese jedoch einzuschränken.

### Herstellungsmethoden

Die erfindungsgemäßen Verbindungen der Formel I können mit Hilfe von im Prinzip bekannten Reaktionen hergestellt werden. Beispielsweise wurden die Verbindungen nach folgenden allgemeinen Reaktionsschemata erhalten.

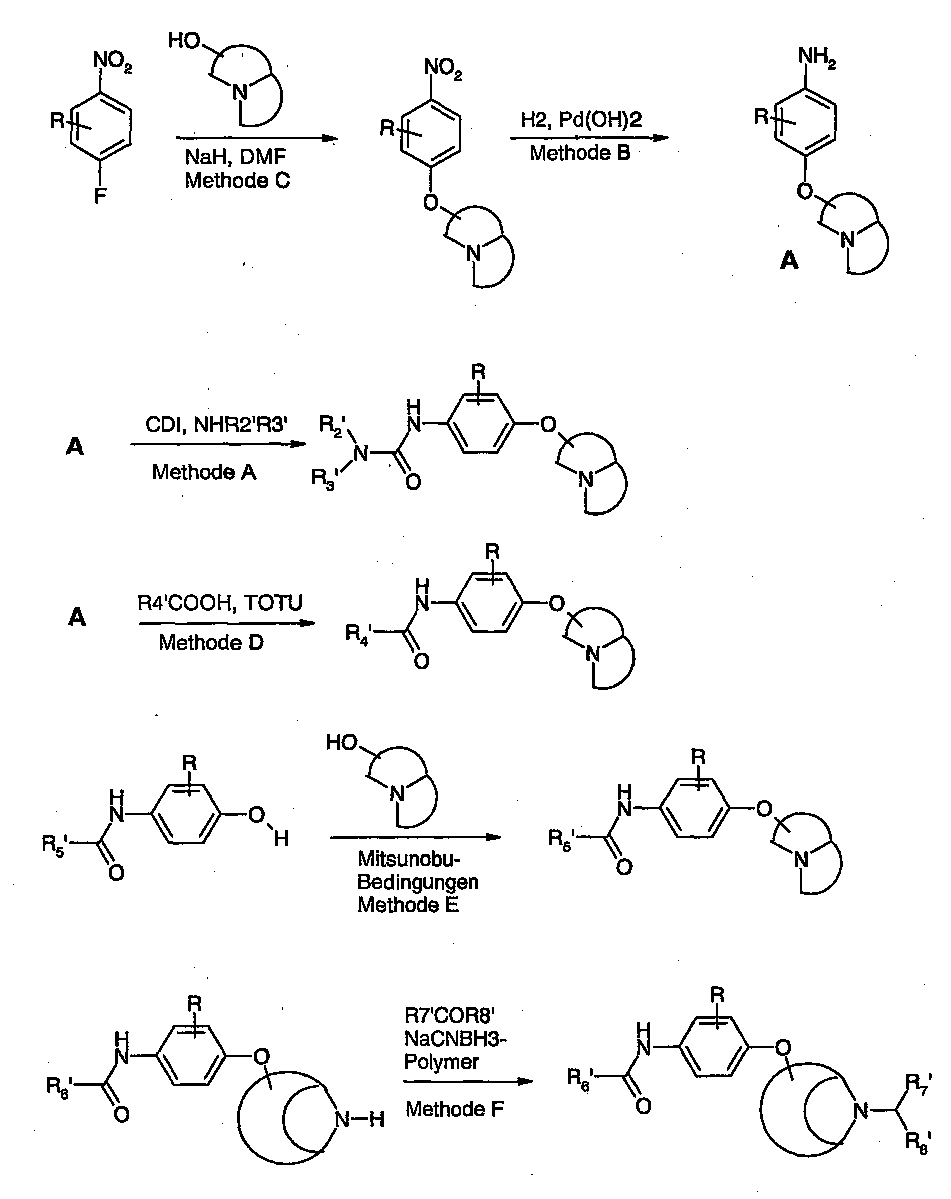

Beschreibungen der verwendeten allgemeinen Methoden finden sich exemplarisch an folgenden Stellen:
Methode A, B und C im Beispiel 1;
Methode D im Beispiel 2;
Methode E im Beispiel 5;
Methode F im Beispiel 78.

### Beispiele

### Allgemeine Erläuterungen

### a) Zeichenweise der Strukturformeln

In den Strukturformeln der gegebenen Beispiele sind zur Übersichtlichkeit nur Nicht-Wasserstoffatome dargestellt.

### b) Salzformen

Viele der erfindungsgemäßen Verbindungen sind Basen und können mit entsprechend starken Säuren Salze bilden. Insbesondere können die Verbindungen nach HPLCchromatographischer Reinigung unter Verwendung eines Trifluoressigsäure enthaltenden Laufmittels als Hydrotrifluoracetate vorliegen. Diese können durch einfaches Behandeln einer Lösung der Salze z. B. mit Natriumcarbonatlösung in die gezeigten freien Basen überführt werden.

### c) Einheiten der Charakterisierungsdaten

Die Einheit der angegebenen Molekulargewichte ist "g/mol". Beobachtete Peaks im Massenspektrum sind angegeben als ganzzahliger Quotient der molaren Molekülionmasse und der Ladung des Molekülions (m/z).

### Beispiel 1

### 1-{4-[(1R,9aR)-1-(Octahydro-quinolizin-1-yl)methoxy]-phenyl}-3-(4-phenoxy-phenyl)-urea

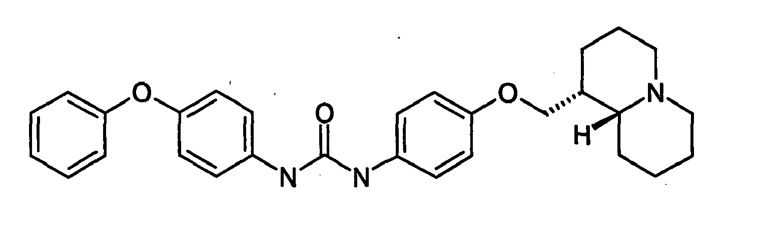

### Methode A

Zu einer auf 0 °C gekühlten Lösung von Carbonyldiimidazol (162 mg) in DMF (1 mL) wurde eine Lösung von 4-Phenoxy-anilin (185 mg) in DMF (1 mL) getropft. Nach 30 Minuten wurde 4-[(lR,9aR)-1-(Octahydro-quinolizin-1-yl)methoxy]-phenylamine (289 mg) in DMF (1 mL) zugetropft. Die Reaktionslösung wurde zunächst für 2 Stunden bei Raumtemperatur und dann für 30 Minuten bei 80°C gehalten. Die Mischung wurde in Wasser (20 mL) eingetropft und der entstandene Niederschlag abgesaugt und mit Wasser gewaschen. Alternativ kann das Produkt auch mit Ethylacetat extrahiert und nach dem Einengen durch Chromatographie gereinigt werden. Man erhielt so das Produkt mit dem Molekulargewicht 471,60 (C29H33N3O3); MS (ESI): 472 (M+H+).

### 4-[(1R,9aR)-1-(Octahydro-quinolizin-1-yl)methoxy]-phenylamine

### Methode B

Eine Suspension von (1R,9aR)-1-(4-Nitro-phenoxymethyl)-octahydro-quinolizine (800 mg) und Palladium(II)-hydroxid (20%ig auf Kohle; 0,15 g) in Ethanol (30 mL) wurde unter Wasserstoffatmosphäre (Normaldruck) für 3 Stunden heftig gerührt. Dann wurde der Katalysator durch Filtration entfernt und das Filtrat eingeengt. Man erhielt so das Produkt mit dem Molekulargewicht 260,38 (C16H24N2O); MS (ESI): 261 (M+H+).

### (1R,9aR)-1-(4-Nitro-phenoxymethyl)-octahydro-quinolizine

### Methode C

Eine Suspension von Natriumhydrid (60% in Öl, 710 mg) in DMF (20 mL) wurde mit (1R,9aR)-1-(Octahydro-quinolizin-1-yl)-methanol (2,5 g) versetzt und nach beendeter Gasentwicklung 4-Fluor-nitrobenzol (2,51 g) zugegeben. Nach 2 Stunden wurde die Reaktionsmischung mit Wasser hydrolysiert und anschließend zwischen Ethylacetat und verdünnter Salzsäure verteilt. Die wässrige Phase wurde mit Natronlauge alkalisch gestellt und zweimal mit Ethylacetat extrahiert. Die zwei Ethylacetatphasen wurden vereinigt, über Magnesiumsulfat getrocknet und eingeengt. Man erhielt so das Produkt mit dem Molekulargewicht 290,37 (C16H22N2O3); MS (ESI): 291 (M+H+).

### Beispiel 2

### 4-Butoxy-N-{4-[(1R,9aR)-1-(octahydro-quinolizin-1-yl)methoxy]-phenyl}-benzamide

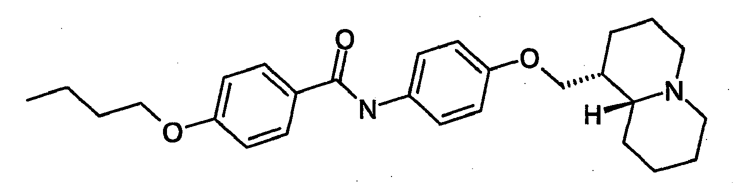

### Methode D

Eine Lösung von 4-Butoxy-benzoic acid (46,4 mg) in DMF (2 mL) wurde bei 0 °C mit TOTU (78 mg) und Ethyldiisopropylamin (31 mg) gefolgt von 4-[(1R,9aR)-1-(Octahydroquinolizin-1-yl)methoxy]-phenylamine (62 mg) versetzt. Nach drei Stunden Reaktionszeit bei Raumtemperatur wurde das Gemisch mit Natriumhydrogencarbonatlösung und Ethylacetat verdünnt. Nach der Trennung der Phasen wurde die wässrige Phase mit Ethylacetat extrahiert und die vereinigten organischen Phasen über Magnesiumsulfat getrocknet und eingeengt. Der Rückstand wurde durch präparative HPLC gereinigt. Man erhielt so das Produkt mit dem Molekulargewicht 436,60 (C27H36N2O3); MS (ESI): 437 (M+H+).

### Beispiel 3

### 1-[4-((1S*,3R*,5R*)-8-Methyl-8-aza-bicyclo[3.2.1]oct-3-yloxy)-phenyl]-3-(4-phenoxyphenyl)-urea

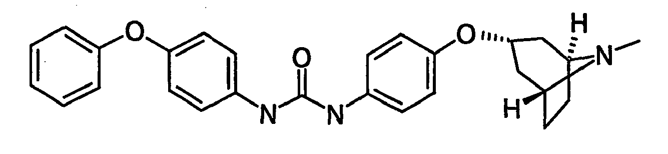

Nach Methode A wurde 4-Phenoxy-aniline mit 4-((1S*,3R*,5R*)-8-Methyl-8-azabicyclo[3.2.1]oct-3-yloxy)-phenylamine umgesetzt. Man erhielt so das Produkt mit dem Molekulargewicht 443,55 (C27H29N3O3); MS (ESI): 444 (M+H+).

4-((1S*,3R*,5R*)-8-Methyl-8-aza-bicyclo[3.2.1]oct-3-yloxy)-phenylamine (1S*,3R*,5R*)-8-Methyl-8-aza-bicyclo[3.2.1]octan-3-ol wurde nach Methode C und B mit 4-Fluornitrobenzol umgesetzt und anschließend hydriert. Man erhielt so das Produkt mit dem Molekulargewicht 232,33 (C14H20N2O); MS (ESI): 233 (M+H+).

### Beispiel 4

### 4-Butoxy-N-[4-((1S*,3R*,5R*)-8-methyl-8-aza-bicyclo[3.2.1]oct-3-yloxy)-phenyl]-benzamide

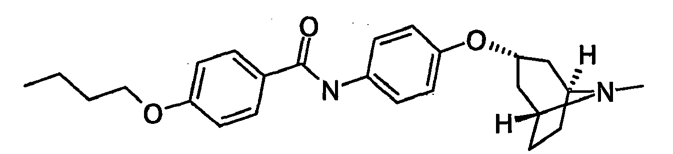

Nach Methode D wurde 4-Butoxy-benzoic acid mit 4-((1S*,3R*,5R*)-8-Methyl-8-aza-bicyclo[3.2.1]oct-3-yloxy)-phenylamine umgesetzt. Man erhielt so das Produkt mit dem Molekulargewicht 408,55 (C25H32N2O3); MS (ESI): 409 (M+H+).

### Beispiel 5

### 4-(4-Chloro-phenyl)-piperidine-1-carboxylic acid [4-((1S*,3S*,5R*)-8-methyl-8-aza-bicyclo[3.2.1]oct-3-yloxy)-phenyl]-amide

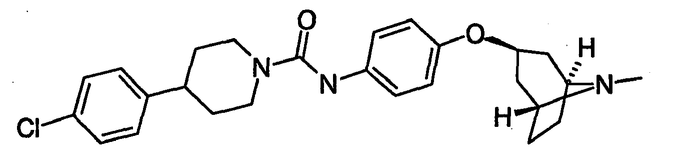

### Methode E

Zu einer gerührten Suspension von Triphenylphosphin (Polymer, 80 mg) in Dichlormethan (3 mL) bei 0°C wurde Di-tert.-butyl azodicarboxylate (69,5 mg) gegeben und nach 5 Minuten eine Mischung aus Tropin, 4-(4-Chloro-phenyl)-piperidine-1-carboxylic acid (4-hydroxy-phenyl)-amide (100 mg) und Dichlormethan (3 mL) zugesetzt. Nach 12 Stunden bei Raumtemperatur wurde vom Polymer abgesaugt und das Filtrat eingeengt. Der Rückstand wurde durch präparative HPLC gereinigt. Man erhielt so das Produkt mit dem Molekulargewicht 454,02 (C26H32C1N3O2); MS (ESI): 454 (M+H+).

### 4-(4-Chloro-phenyl)-piperidine-1-carboxylic acid (4-hydroxy-phenyl)-amide

Eine Lösung von 4-Aminophenol (1,0 g) in DMF (10 mL) wurde bei 0°C mit Carbonyldiimidazol (1,48 g) versetzt. Nach 10 Minuten wurde eine Mischung von 4-(4-Chloro-phenyl)-piperidine (Hydrochlorid; 2,13 g), Hünig-Base (1,18 g) und DMF (10 mL) zugesetzt. Nach zwei Stunden wurde die Reaktionsmischung mit Wasser verdünnt und mit Ethylacetat extrahiert. Die organische Phase wurde mit Wasser gewaschen, über Magnesiumsulfat getrocknet, filtriert und eingeengt. Man erhielt so das Produkt mit dem Molekulargewicht 330,82 (C18H19C1N2O2); MS (ESI): 331 (M+H+).

### Beispiel 6

### 4-(4-Chloro-phenyl)-piperidine-1-carboxylic acid [4-((1S*,3R*,5R*)-8-methyl-8-aza-bicyclo[3.2.1]oct-3-yloxy)-phenyl]-amide

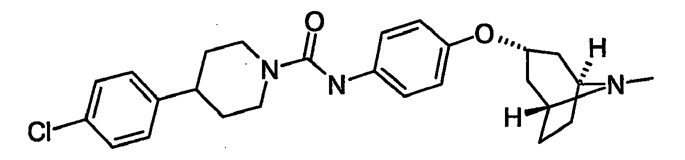

Nach Methode A wurde 4-(4-Chloro-phenyl)-piperidine mit 4-((1S*,3R*,5R*)-8-Methyl-8-aza-bicyclo[3.2.1]oct-3-yloxy)-phenylamine umgesetzt. Man erhielt so das Produkt mit dem Molekulargewicht 454,02 (C26H32ClN3O2); MS (ESI): 454 (M+H+).

### Beispiel 7

### 4-(4-Chloro-phenyl)-piperidine-1-carboxylic acid [4-(4-oxo-octahydro-quinolizin-1-yloxy)-phenyl]-amide

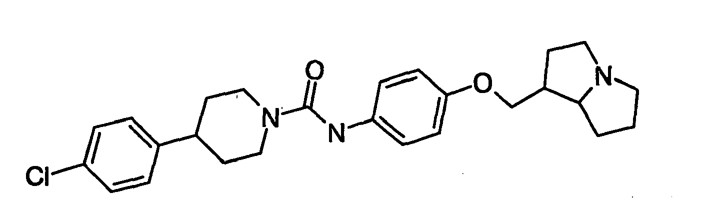

Nach Methode E wurde 4-(4-Chloro-phenyl)-piperidine mit (Hexahydro-pyrrolizin-1-yl)-methanol umgesetzt. Man erhielt so das Produkt mit dem Molekulargewicht 454,02 (C26H32C1N302); MS (ESI): 454 (M+H+).

### (Hexahydro-pyrrolizin-1-yl)-methanol

Eine Lösung von Hexahydro-pyrrolizine-1-carboxylic acid ethyl ester (0,97 g) in THF (3 mL) wurde mit Lithiumaluminiumhydrid (0,3 g) versetzt. Nach 12 Stunden wurde die Reaktionsmischung mit Diethylether verdünnt und durch vorsichtige Zugabe von Wasser (0,7 mL) hydrolysiert. Der entstandene Niederschlag wurde abfiltriert und das Filtrat eingeengt. Man erhielt so das Produkt mit dem Molekulargewicht 141,21 (C8H15NO); MS (ESI): 142 (M+H+).

### Hexahydro-pyrrolizine-1-carboxylic acid ethyl ester

Eine Mischung von rac-Prolin (1,15 g), Ethylacrylat (1,20 g), Toluol (80 mL) und Paraformaldehyd (3,6 g) wurde für 3 Stunden zum Rückfluss erhitzt. Die Mischung wurde eingeengt und der Rückstand über Kieselgel filtriert. Man erhielt so das Produkt mit dem Molekulargewicht 183,25 (C10H17NO2); MS (ESI): 184 (M+H+).

Nach Methode A (für Harnstoffe) oder Methode D (für Amide) wurden die Verbindungen in Tabelle 2a und Tabelle 2b erhalten.

**Tabelle 2a.**

| Bsp. No. | Struktur | Summenformel | Molekulargewicht | ESI-MS [M+H]⁺ |
|---|---|---|---|---|
| 8 | 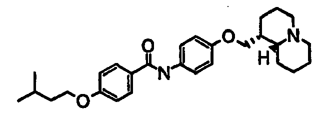 | C28H38N2O3 | 450.63 | 451 |
| 9 | 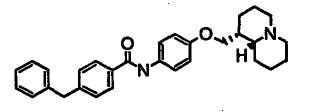 | C30H34N2O2 | 454.62 | 455 |
| 10 | 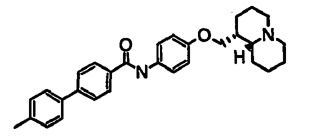 | C30H34N2O2 | 454.62 | 455 |
| 11 | 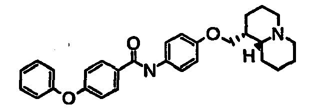 | C29H32N2O3 | 456.59 | 457 |
| 12 | 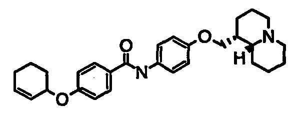 | C29H36N2O3 | 460.62 | 461 |
| 13 | 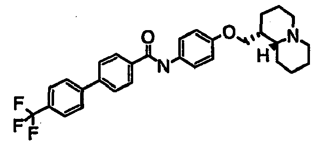 | C30H31F3N2O2 | 508.59 | 509 |
| 14 | 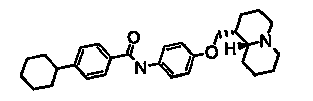 | C29H38N2O2 | 446.64 | 447 |
| 15 | 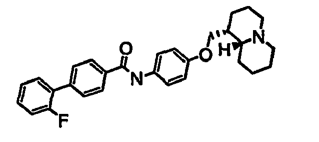 | C29H31FN2O2 | 458.58 | 459 |
| 16 | 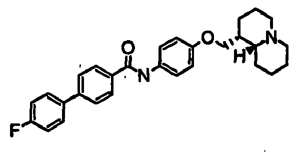 | C29H31FN2O2 | 458.58 | 459 |
| 17 | 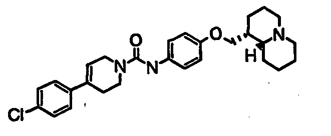 | C28H34C1N3O2 | 480.06 | 480 |
| 18 | 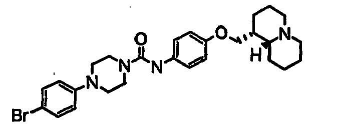 | C27H35BrN4O2 | 527.51 | 528 |
| 19 | 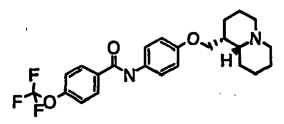 | C24H27F3N2O3 | 448.49 | 449 |
| 20 | 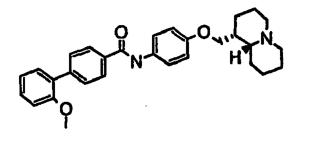 | C30H34N2O3 | 470.62 | 471 |
| 21 | 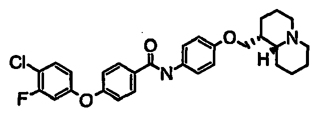 | C29H30ClFN2O3 | 509.03 | 509 |
| 22 | 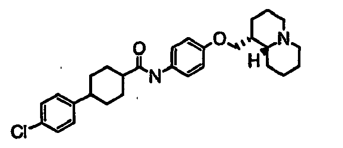 | C29H37ClN2O2 | 481.08 | 481 |
| 23 | 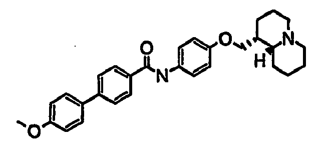 | C30H34N2O3 | 470.62 | 471 |
| 24 | 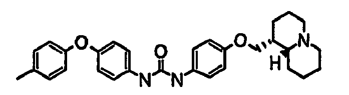 | C30H35N3O3 | 485.63 | 486 |
| 25 | 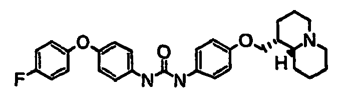 | C29H32FN3O3 | 489.60 | 490 |
| 26 | 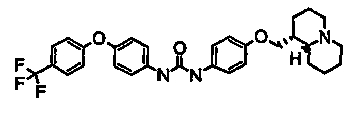 | C30H32F3N3O3 | 539.60 | 540 |
| 27 | 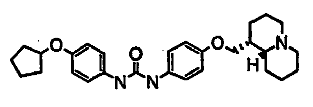 | C28H37N3O3 | 463.63 | 464 |
| 28 | 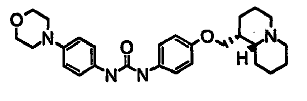 | C27H36N4O3 | 464.61 | 465 |
| 29 | 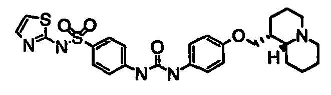 | C26H31N5O4S2 | 541.70 | 542 |
| 30 | 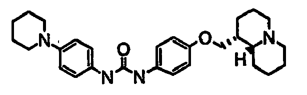 | C28H38N4O2 | 462.64 | 463 |
| 31 | 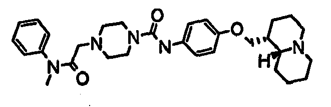 | C30H41N5O3 | 519.69 | 520 |
| 32 | 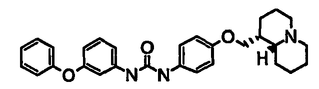 | C29H33N3O3 | 471.60 | 472 |
| 33 | 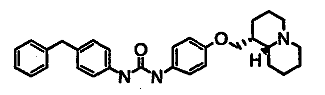 | C30H35N3O2 | 469.63 | 470 |
| 34 | 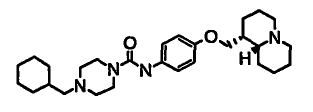 | C28H44N4O2 | 468.69 | 469 |
| 35 | 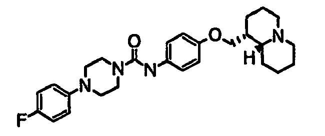 | C27H35FN4O2 | 466.60 | 467 |
| 36 | 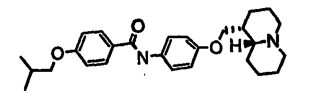 | C27H36N2O3 | 436.60 | 437 |
| 37 | 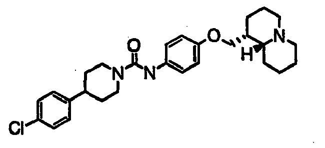 | C28H36ClN3O2 | 482.07 | 482 |
| 38 | 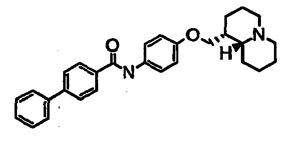 | C29H32N2O2 | 440.59 | 441 |
| 39 | 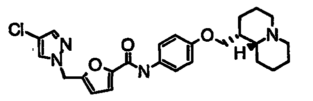 | C25H29ClN4O3 | 468.99 | 469 |
| 40 | 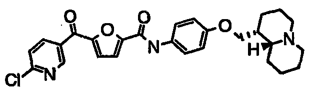 | C27H28ClN3O4 | 493.99 | 494 |
| 41 | 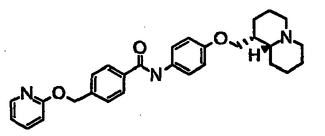 | C29H33N3O3 | 471.60 | 472 |
| 42 | 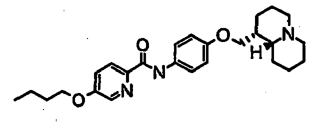 | C26H35N3O3 | 437.59 | 438 |
| 43 | 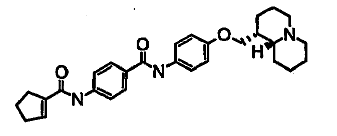 | C29H35N3O3 | 473.62 | 474 |
| 44 | 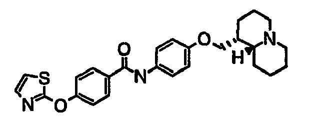 | C26H29N3O3S | 463.60 | 464 |
| 45 | 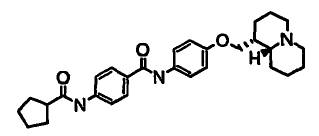 | C29H37N3O3 | 475.64 | 476 |
| 46 | 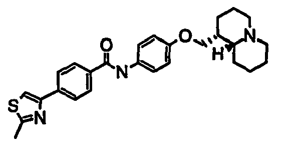 | C27H31N3O2S | 461.63 | 462 |
| 47 | 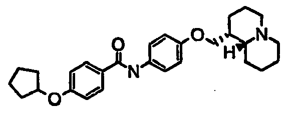 | C28H36N2O3 | 448.61 | 449 |
| 48 | 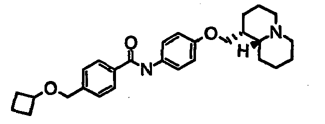 | C28H36N2O3 | 448.61 | 449 |
| 49 | 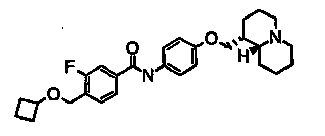 | C28H35FN2O3 | 466.60 | 467 |
| 50 | 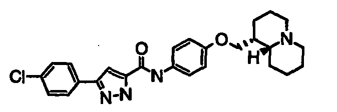 | C26H29ClN4O2 | 465.00 | 465 |
| 51 | 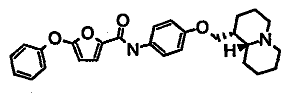 | C27H30N2O4 | 446.55 | 447 |
| 52 | 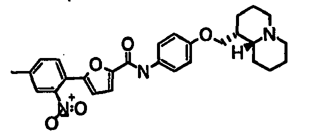 | C28H31N3O5 | 489.58 | 490 |
| 53 | 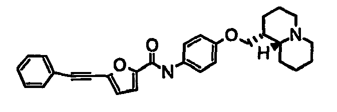 | C29H30N203 | 454.57 | 455 |
| 54 | 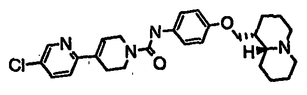 | C27H33CIN4O2 | 481.04 | 482 |
| 55 | 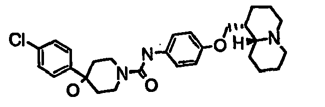 | C28H36CIN3O3 | 498.07 | 498 |

**Tabelle 2b.**

| Bsp. No. | Struktur | Summenformel | Molekulargewicht | ESI-MS [M+H]⁺ |
|---|---|---|---|---|
| 56 | 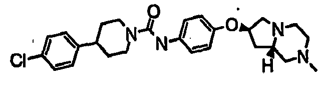 | C26H33 C1N4O2 | 469,03 | 469 |
| 57 | 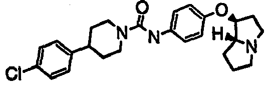 | C25H30ClN3O2 | 439,99 | 440 |
| 58 | 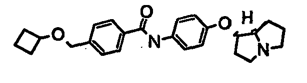 | C25H30N2O3 | 406,53 | 407 |
| 59 | 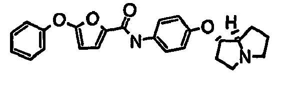 | C24H24N2O4 | 404,47 | 405 |
| 60 | 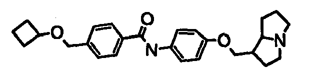 | C26H32N2O3 | 420,56 | 421 |
| 61 | 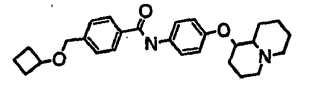 | C27H34N2O3 | 434,58 | 435 |
| 62 | 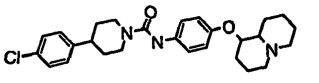 | C27H34ClN3O2 | 468,04 | 468 |
| 63 | 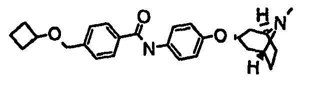 | C26H32N2O3 | 420,56 | 421 |
| 64 | 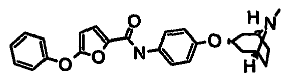 | C25H26N2O4 | 418,50 | 419 |
| 65 | 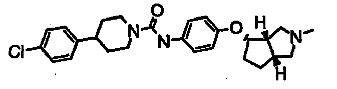 | C26H32ClN3O2 | 454,02 | 454 |
| 66 | 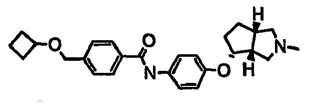 | C26H32N2O3 | 420,56 | 421 |
| 67 | 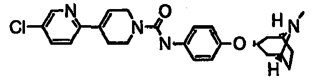 | C25H29ClN4O2 | 452,99 | 453 |
| 68 | 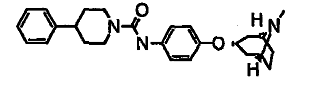 | C26H33N3O2 | 419,57 | 420 |
| 69 | 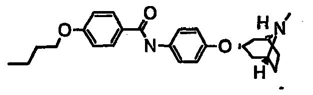 | C25H32N2O3 | 408,55 | 409 |
| 70 | 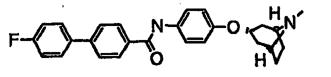 | C27H27FN2O2 | 430,53 | 431 |
| 71 | 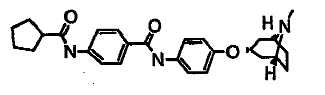 | C27H33N3O3 | 447,58 | 448 |
| 72 | 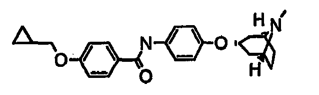 | C25H30N2O3 | 406,53 | 407 |
| 73 | 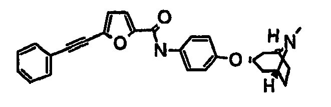 | C27H26N2O3 | 426,52 | 427 |
| 74 | 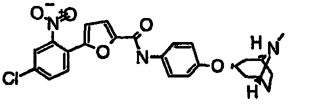 | C25H24C1N3O5 | 481,94 | 482 |
| 75 | 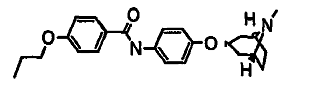 | C24H30N2O3 | 394,52 | 395 |
| 76 | 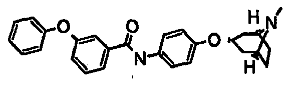 | C27H28N2O3 | 428,54 | 429 |
| 77 | 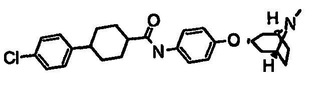 | C27H33C1N2O2 | 453,03 | 453 |

### Beispiel 78

### 4-(4-Chloro-phenyl)-piperidine-1-carboxylic acid {4-((1S*,3R*,5R*)-[8-(tetrahydro-furan-3-ylmethyl)-8- aza-bicyclo[3.2.1]oct-3-yloxy]-phenyl}-amide

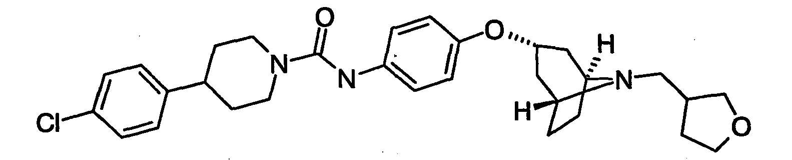

### Methode F

Eine Mischung aus 4-(4-Chloro-phenyl)-piperidine-1-carboxylic acid [4-((1S*,3R*,5R*)-8-aza-bicyclo[3.2.1]oct-3-yloxy)-phenyl]-amide (50 mg), Tetrahydro-furan-3-carbaldehyde (10 mg), Essigsäure (7 mg), THF (2 mL) wurde mit Natriumcyanoborhydrid (Polymergebunden; 0,12 mmol) versetzt und 12 h gerührt. Es wurde vom Polymer abgesaugt und das Filtrat eingeengt. Der Rückstand wurde durch präparative HPLC gereinigt. Man erhielt so das Produkt mit dem Molekulargewicht 524,11 (C30H38C1N3O3); MS (ESI): 524 (M+H+).

### 4-(4-Chloro-phenyl)-piperidine-1-carboxylic acid [4-((1S*,3R*,5R*)-8-aza-bicyclo[3.2.1]oct-3-yloxy)-phenyl]-amide

(1S*,3R*,5R*)-8-Aza- bicyclo[3.2.1]octan-3-ol wurde nach einem Standardverfahren (Di-tert.-butyldicarbonate, Natriumhydroxid, THF/Wasser) geschützt und das erhaltene Carbamat nach Methode C und B zunächst mit 4-Fluor-nitrobenzol umgesetzt und dann hydriert. Das erhaltene Anilin ((1S,3R,5R)-3-(4-Amino-phenoxy)-8-aza-bicyclo[3.2.1]octane-8-carboxylic acid tert-butyl ester) wurde nach Methode A mit 4-(4-Chloro-phenyl)-piperidine umgesetzt und abschließend mit Trifluoressigsäure in Dichlormethan das Amin freigesetzt. Man erhielt so das Produkt mit dem Molekulargewicht 439,99 (C25H30CIN3O2); MS (ESI): 440 (M+H+).

Durch Umsetzung von 4-(4-Chloro-phenyl)-piperidine-1-carboxylic acid [4-((1S*,3R*,5R*)-8-aza-bicyclo[3.2.1]oct-3-yloxy)-phenyl]-amide mit den entsprechenden Carbonylverbindungen nach Methode F wurden die Beispiele in Tabelle 3 erhalten.

**Tabelle 3.**

| Bsp. No. | Struktur | Summenformel | Molekulargewicht | ESI-MS [M+H]⁺ |
|---|---|---|---|---|
| 79 | 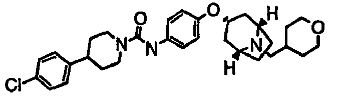 | C31H40ClN3O3 | 538,14 | 538 |
| 80 | 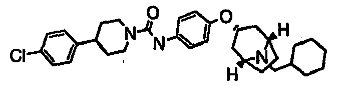 | C32H42ClN3O2 | 536,16 | 536 |
| 81 | 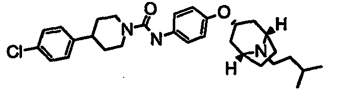 | C30H40ClN3O2 | 510,13 | 510 |
| 82 | 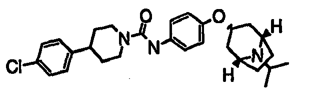 | C28H36ClN3O2 | 482,07 | 482 |
| 83 | 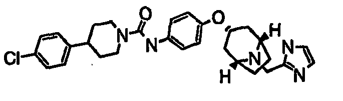 | C29H34ClN5O2 | 520,08 | 520 |
| 84 | 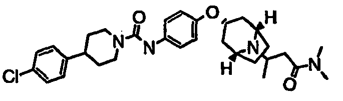 | C31H41ClN4O3 | 553,15 | 553 |
| 85 | 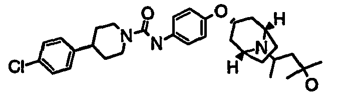 | C31H42ClN3O3 | 540,15 | 540 |
| 86 | 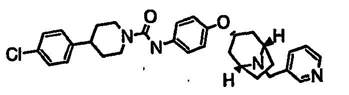 | C31H35C1N4O2 | 531,10 | 531 |
| 87 | 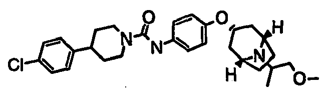 | C29H38ClN3O3 | 512,10 | 512 |
| 88 | 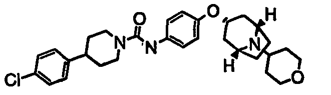 | C30H38ClN3O3 | 524,11 | 524 |

### Synthese nicht käuflich erhaltener Ausgangsmaterialien

### 4-(Cyclopentanecarbonyl-amino)-benzoic acid

4-Amino-benzoic acid ethyl ester wurde nach Methode E mit Cyclopentanecarboxylic acid umgesetzt und der erhaltene Ester durch Kochen mit Natriumhydroxid in wässrigem Ethanol verseift. Man erhielt so das Produkt mit dem Molekulargewicht 233,27 (C13H15N03); MS (ESI): 234 (M+H+).
Analog kann 4-[(Cyclopent-1-enecarbonyl)-amino]-benzoic acid erhalten werden.

### 4-Cyclobutoxymethyl-benzoic acid

Eine Lösung von Cyclobutanol (0,7 g) in DMF (8 mL) wurde vorsichtig mit Natriumhydrid (50% in Öl; 0,42g) versetzt. Nach beenderter Gasentwicklung wurde 4-Brommethyl-benzoesäure methyl ester (1,0 g) zugesetzt. Nach 4 Stunden wurde die Mischung zwischen Wasser und Ethylacetat verteilt. Die organische Phase wurde über Magnesiumsulfat getrocknet und eingeengt. Der als Rohprodukt erhaltene Ester wurde durch Kochen mit Natriumhydroxid in wässrigem Ethanol verseift. Man erhielt so das Produkt mit dem Molekulargewicht 206,24 (C12H14O3); MS (ESI): 207 (M+H+).

### 4-Cyclobutoxymethyl-3-fluoro-benzoic acid

Eine Lösung von Cyclobutanol (2,7 g) in DMF (12 mL) wurde vorsichtig mit Natriumhydrid (50% in Öl; 1,63 g) versetzt. Nach beendeter Gasentwicklung wurde 4-Bromo-1-bromomethyl-2-fluoro-benzene (10 g) bei 0°C zugesetzt. Nach 6 Stunden bei Raumtemperatur wurde die Mischung nach vorsichtiger Hydrolyse zwischen Wasser und Ethylacetat verteilt. Die organische Phase wurde über Magnesiumsulfat getrocknet und eingeengt. Man erhielt so 4-Bromo-l-cyclobutoxymethyl-2-fluoro-benzene als Rohprodukt.
Eine Lösung von 4-Bromo-1-cyclobutoxymethyl-2-fluoro-benzene (2,6 g) in THF (50 mL) wurde bei -78°C mit n-Butyllithium (1,6 M in Hexan; 7,0 mL) versetzt. Nach 15 Minuten wurde Trockeneis (4,4 g) zugesetzt. Nach dem Erwärmen auf Raumtemperatur wurde die Mischung mit Wasser verdünnt und mit Ethylacetat extrahiert. Die wässrige Phase wurde sauer gestellt und erneut mit Ethylacetat extrahiert. Die organische Phase wurde über Magnesiumsulfat getrocknet, filtriert und eingeengt. Man erhielt so das Produkt mit dem Molekulargewicht 224,23 (C12H13F03); MS (ESI): 225 (M+H+).

### 4-(Pyridin-2-yloxymethyl)-benzoic acid

Eine Mischung aus 2-Fluorpyridin (1,6 g), 4-Brombenzylalkohol (3,08 g), Kalium-tert.-butoxid (2,03 g) und N-Methylpyrrolidon (12,8 mL) wurde für eine Minute durch Mikrowellenbestrahlung auf 100°C erhitzt. Die Mischung wurde mit Wasser verdünnt und mit Ethylacetat extrahiert. Die organische Phase wurde über Magnesiumsulfat getrocknet, filtriert und eingeengt. Man erhielt so 2-(4-Bromo-benzyloxy)-pyridine.
Eine Lösung von 2-(4-Bromo-benzyloxy)-pyridine (4,2 g) in THF (120 mL) wurde bei - 78°C mit n-Butyllithium (1,6 M in Hexan; 11,4 mL) versetzt. Nach 15 Minuten wurde Trockeneis (7 g) zugesetzt. Nach dem Erwärmen auf Raumtemperatur wurde die Mischung mit Wasser verdünnt und mit Ethylacetat extrahiert. Die wässrige Phase wurde sauer gestellt und erneut mit Ethylacetat extrahiert. Die organische Phase wurde über Magnesiumsulfat getrocknet, filtriert und eingeengt. Man erhielt so das Produkt mit dem Molekulargewicht 229,24 (C13H11NO3); MS (ESI): 230 (M+H+).

### 5-Butoxy-pyridine-2-carboxylic acid

5-Hydroxy-pyridin-2-carbonsäure benzhydryl ester (2.0 g) gelöst in DMF (20 mL) wurde mit Natriumhydrid (50% in Öl, 250 mg) versetzt und nach beendeter Gasentwicklung 1-Brombutan (0,72 g) zugesetzt. Die Mischung wurde für 6 Stunden auf 90 °C erwärmt. Es wurde mit Wasser verdünnt und mit Ethylacetat extrahiert. Die organische Phase wurde über Magnesiumsulfat getrocknet und eingeengt. Der Rückstand wurde analog der Methode B hydriert. Man erhielt so das Produkt mit dem Molekulargewicht 195,22 (C10H13NO3); MS (ESI): 196 (M+H+).

### 5-Chloro-1',2',3',6'-tetrahydro-[2,4']bipyridinyl

Eine Lösung von 2-Brom-5-chlorpyridin (2,0 g) in Diethylether (50 mL) wurde bei -78°C tropfenweise mit Butyllithium (15% in Hexan; 7,6 mL) versetzt und nach einer Stunde eine Lösung von N-tert.-Butoxycarbonyl-4-piperidinon (2,1 g) in Diethylether (10 mL) zugetropft. Nach 30 Minuten wurde vorsichtig Wasser zugesetzt und die Mischung mit Ethylacetat extrahiert. Die organische Phase wurde über Natriumsulfat getrocknet, filtriert und eingeengt. Der Rückstand wurde für 24 Stunden mit Thionylchlorid (3 g) behandelt und die eingeengte Reaktionslösung durch präparative HPLC gereinigt. Man erhielt so das Produkt mit dem Molekulargewicht 194,67 (C10H11ClN2); MS(ESI): 195 (M+H+).

### 4-Cyclopentyloxy-anilin

Eine Mischung von 4-Nitrophenol (63,7 g), Bromcyclopentan (68,2 g), Kaliumcarbonat (63,3 g) und DMF (300 mL) wurde für 24 Stunden auf 80 °C erwärmt. Nach dem Abkühlen wurde mit Wasser verdünnt und mit Ethylacetat extrahiert. Die organische Phase wurde mit Wasser gewaschen, über Magnesiumsulfat getrocknet und eingeengt. Der Rückstand wurde nach Methode B hydriert. Man erhielt so das Produkt mit dem Molekulargewicht 177,25 (C11H15NO); MS (ESI): 178 (M+H+).

Ausgehend von den entsprechenden cyclischen Aminoalkoholen wurden durch Umsetzung mit 4-Fluor-nitrobenzol nach Methode C und anschließende Hydrierung die folgenden Aniline erhalten:
4-((S)-2-Benzyl-2-aza-bicyclo[2.2.2]oct-6-yloxy)-phenylamine;
4-((7R,8aS)-2-Methyl-octahydro-pyrrolo[1,2-a]pyrazin-7-yloxy)-phenylamine;
4-[(4R*,5S*)-(1-Aza-bicyclo[3.3.1]non-4-yl)oxy]-phenylamine;
4-[(1R*,7aS*)-(Hexahydro-pyrrolizin-1-yl)oxy]-phenylamine;
4-(Octahydro-quinolizin-1-yloxy)-phenylamine und
4-((3aS*,4S*,6aR*)-2-Methyl-octahydro-cyclopenta[c]pyrrol-4-yloxy)-phenylamine.

Die benötigten cyclischen Aminoalkohole wurden nach Literaturmethoden erhalten:
(7R,8aS)-2-Methyl-octahydro-pyrrolo[1,2-a]pyrazin-7-ol (J. Heterocyclic Chem. 1999, 27, 2181);
(4R*,5S*)-1-Aza-bicyclo[3.3.1]nonan-4-ol (Lithiumaluminiumhydridreduktion von 1-Aza-bicyclo[3.3.1]nonan-4-one (J. Med. Chem. 2003,46,2216));
(1R*,7aS*)-Hexahydro-pyrrolizin-1-ol (Tetrahedron Lett. 1996,52,3757);
Octahydro-quinolizin-1-ol (J. Org. Chem. 1964, 29, 2248) und
(3aS*,4S*,6aR*)-2-Methyl-octahydro-cyclopenta[c]pyrrol-4-ol ((3aS*,4S*,6aR*)-2-Trityl-octahydro-cyclopenta[c]pyrrol-4-ol (Eur. J. Med. Chem. 1991, 26, 889) wurde in einem Gemisch aus Ameisensäure und wässrigem Formaldehyd erhitzt).

## Patentansprüche

1. Verbindungen der Formel I, 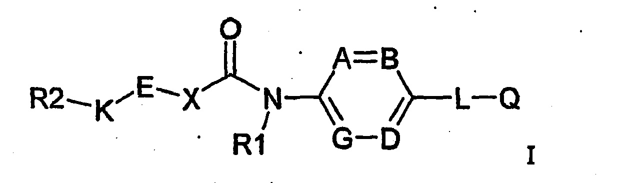 worin bedeuten
A,B,D,G unabhängig voneinander N, C(R3);
oder
die Gruppen A und B oder die Gruppen D und G sind jeweils C(R3) und bilden gemeinsam einen 5- oder 6-gliedrigen carbocyclischen oder heterocyclischen Rest, so dass sich insgesamt ein bicyclisches System ergibt;
R3 H, F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, (C₂-C₆)-Alkinyl, (C₀-C₈)-Alkylen-aryl, O-(C₀-C₈)-Alkylen-aryl, S-Aryl, N(R4)(R5), SO₂-CH₃, COOH, COO-(C₁-C₆)-Alkyl, CON(R6)(R7), N(R8)CO(R9), N(R10)SO₂(R11), CO(R12), (CR13R14)ₓ-O(R15);
R4, R5, R6, R7, R8, R10 unabhängig voneinander H, (C₁-C₈)-Alkyl;
oder
R4 und R5, R6 und R7 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R9, R11, R12 unabhängig voneinander H, (C₁-C₈)-Alkyl, Aryl;
R13, R14 unabhängig voneinander H, (C₁-C₈)-Alkyl;
R15 H, (C₁-C₆)-Alkyl, Aryl;
x 0, 1, 2, 3, 4, 5, 6;
R1 H, (C₁-C₈)-Alkyl, (C₃-C₆)-Alkenyl, (C₃-C₆)-Alkinyl;
X N(R16), O eine Bindung, (R17)C=C(R18), C≡C, eine Gruppe der Formel (CR19R20)_{y}, worin eine oder zwei Gruppen (CR19R20), durch Y ersetzt sein können, wobei sich ein chemisch sinnvoller Rest ergibt;
Y O, S, N(R21), C=O;
R16, R17, R18 unabhängig voneinander H, (C₁-C₈)-Alkyl;
R19, R20 unabhängig voneinander H, (C₁-C₄)-Alkyl, wobei R19 und R20 in den y Gruppen jeweils die gleichen oder verschiedene Bedeutungen aufweisen können;
y 1, 2, 3, 4, 5, 6;
R21 H, (C₁-C₈)-Alkyl;
E 3-14 gliedrige bivalente carbo- oder heterocyclische Ringstruktur mit 0-4 Heteroatomen aus der Gruppe N, O und S, die optional Substituenten aus der Gruppe H, F, Cl, Br, I, OH, CF₃, CN, OCF₃, Oxo, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, (C₀-C₈)-Alkylen-aryl, O-(C₀-C₈)-Alkylen-aryl, S-Aryl, N(R22)(R23), SO₂-CH₃, CON(R24)(R25), N(R26)CO(R27), N(R28)SO₂(R29), CO(R30) tragen und mono- oder bicyclisch sein kann, wobei E keine Tetrazol-5-yl-Gruppe ist;
R22, R23, R24, R25, R26, R28 unabhängig voneinander H, (C₁-C₈)-Alkyl, Aryl;
oder
R22 und R23, R24 und R25 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R27, R29, R30 unabhängig voneinander H, (C₁-C₈)-Alkyl, Aryl;
K eine Bindung, C≡C, (R31)C=C(R32), eine Gruppe der Formel (CR33R34)_{z}, worin eine oder mehrere Gruppen (CR33R34) durch Z ersetzt sein können, wobei sich ein chemisch sinnvoller Rest ergibt, bevorzugt eine Bindung, O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂;
v 1,2,3,4;
R31, R32, R35, R36, R37, R38, R39 unabhängig voneinander H, (C₁-C₈)-Alkyl;
Z O, S, N(R40), CO, SO, SO₂;
R33, R34 unabhängig voneinander H, (C₁-C₈)-Alkyl, Hydroxy-(C₁-C₄)-Alkyl, Hydroxy, (C₁-C₄)-Alkoxy-(C₁-C₄)-alkyl, wobei R38 und R39 in den z Gruppen jeweils die gleichen oder verschiedene Bedeutungen aufweisen können;
z 1, 2, 3, 4, 5, 6;
R40 H, (C₁-C₈)-Alkyl;
R2 H; (C₁-C₈)-Alkyl, (C₁-C₈)-Alkoxy-(C₁-C₄)-alkyl, (C₃-C₈)-Alkenyl, (C₃-C₈)-Alkinyl, ein 3 bis 10-gliedriger mono-, bi-, tri- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R41), CON(R42)(R43), Hydroxy, COO(R44), N(R45)CO(C₁-C₆)-Alkyl, N(R46)(R47), SO₂CH₃, SCF₃ oder S-(C₁-C₆)-Alkyl, wobei R2 keine Tetrazol-5-yl-Gruppe ist;
R41, R42, R43, R44, R45, R46, R47 unabhängig voneinander H, (C₁-C₈)-Alkyl;
oder
R42 und R43, R46 und R47 bilden unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
E, K und R2 zusammen einen Tricyclus bilden, wobei die Ringe unabhängig voneinander gesättigt, teilgesättigt oder ungesättigt und jeweils 3 - 8 Ringatome enthalten können;
L eine Gruppe der Formel (CR48R49)ₘ, worin eine oder mehrere Gruppen (CR48R49) durch M ersetzt sein können, wobei sich ein chemisch sinnvoller Rest ergibt, wobei L in dem Fall, dass Q 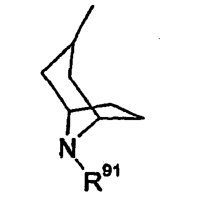 bedeutet, O ist, wobei R91 nachstehend definiert ist;
M O;
m 1, 2, 3, 4, 5, 6, bevorzugt 1, 2, 3, 4, besonders bevorzugt 1, 2;
R48, R49, R50 unabhängig voneinander H, (C₁-C₆)-Alkyl, Aryl;
Q 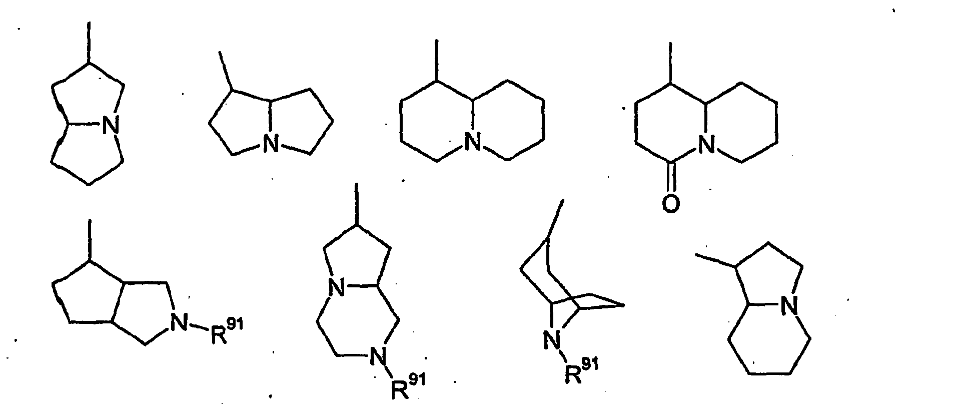
R91 H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96;
R92 H, (C₁-C₈)-Alkyl;
R93, R94 unabhängig voneinander H, (C₁-C₈)-Alkyl, OH, (C₃-C₈)-Cycloalkyl, (C₁-C₄)-Alkoxy-(C₁-C₄)-alkyl;
o', p' unabhängig voneinander 0, 1, 2, 3, 4, 5, 6;
R95, R96 unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH;
R97, R98, R99, R100, R103, R104 unabhängig voneinander H, (C₁-C₈)-Alkyl;
oder
R97 und R98 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R101, R102 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 unabhängig voneinander H, (C₁-C₈)-Alkyl;
oder
R117 und R118 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q', r' unabhängig voneinander 0, 1, 2, 3, 4, 5, 6;
R108,R111 unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
R120, R121, R122, R123, R124, R125, R126, R127 unabhängig voneinander H, (C₁-C₈)-Alkyl;
oder
R120 und R121, R124 und R125 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
deren N-Oxide sowie deren physiologisch verträgliche Salze.

2. Verbindungen der Formel I nach Anspruch 1, worin bedeuten
L eine Gruppe der Formel (CR48R49)ₘ, worin eine oder mehrere Gruppen (CR48R49) durch M ersetzt sein können, wobei sich ein chemisch sinnvoller Rest ergibt, wobei L in dem Fall, dass Q 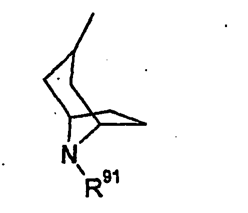 bedeutet, O ist,
M O;
m 1, 2, 3, 4, 5, 6, bevorzugt 1, 2, 3, 4, besonders bevorzugt 1, 2;
R48, R49, R50. unabhängig voneinander H, (C₁-C₆)-Alkyl, Aryl, bevorzugt H, (C₁-C₆)-Alkyl, besonders bevorzugt H.

3. Verbindungen der Formel I nach Anspruch 1 oder 2,
worin bedeuten
A, B, D, G unabhängig voneinander N, C(R3) oder die Gruppen A und B oder D und G sind jeweils C(R3) und bilden gemeinsam eine ortho-Phenyleneinheit, so dass sich insgesamt ein 1,4-bisubstitüiertes Naphthalinsystem ergibt; bevorzugt unabhängig voneinander N oder C(R3), wobei die Gesamtzahl der Stickstoffatome in dem Ring 0 - 2, bevorzugt 0 oder 1 beträgt, besonders bevorzugt sind A, B, D und G C(R3);
R3 H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀ - C₈)-Alkylen-aryl; N(R4)(R5), SO₂-CH₃, CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)(R14)ₓ-O(R15); ganz besonders bevorzugt H, F, (C₁-C₆)-Alkyl; weiter bevorzugt H, F, CH₃; insbesondere bevorzugt H;
R4, R5, R6, R7, R8 unabhängig voneinander H, (C₁-C₈)-Alkyl;
oder
R4 und R5, R6 und R7 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R9, R12 unabhängig voneinander H, (C₁-C₈)-Alkyl;
R13, R14 H;
R15 H, (C₁-C₆)-Alkyl;
x 0, 1, 2, bevorzugt 0, 1, besonders bevorzugt 1;
R1 H, (C₁-C₈)-Alkyl;
X N(R16), eine Bindung, (R17)C=C(R18), C≡C, CH₂-CH₂, YCH₂, CH₂Y, bevorzugt N(R16), eine Bindung;
Y O, S, N(R21);
R16, R17, R18 unabhängig voneinander H, (C₁-C₈)-Alkyl;
R21 H, (C₁-C₈)-Alkyl;
E 3-8 gliedrige bivalente carbo- oder heterocyclische Ringstruktur mit 0-4 Heteroatomen aus .der Gruppe N, O und S, die optional Substituenten aus der Gruppe H, F, Cl, Br, OH, CF₃, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, O-(C₃-C₈)-Cycloalkyl, (C₃-C₈)-Cycloalkenyl, (C₂-C₆)-Alkinyl, (C₀-C₈)-Alkylen-aryl, O-(C₀-C₈)-Alkylen-aryl, S-Aryl, N(R22)(R23), SO₂-CH₃, N(R26)CO(R27), N(R28)SO₂(R29), CO(R30) tragen und mono- oder bicyclisch sein kann, wobei E keine Tetrazol-5-yl-Gruppe ist; bevorzugt 5-7 gliedrige bivalente carbo- oder heterocyclische Ringstruktur mit 0-3 Heteroatomen aus der Gruppe N, O und S, die optional Substituenten aus der Gruppe H, F, Cl, Br, OH, CF₃, CN, OCF₃, O-(C₁-C₆)-Alkyl, S-(C₁-C₆)-Alkyl, (C₁-C₆)-Alkyl, (C₂-C₆)-Alkenyl, O-(C₀-C₈)-Alkylen-aryl, S-Aryl, N(R22)(R23), SO₂-CH₃, N(R26)CO(R27), CO(R30) tragen und mono- oder bicyclisch sein kann;
besonders bevorzugt 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl tragen kann
z.B. ist E ausgewählt aus der Gruppe bestehend aus 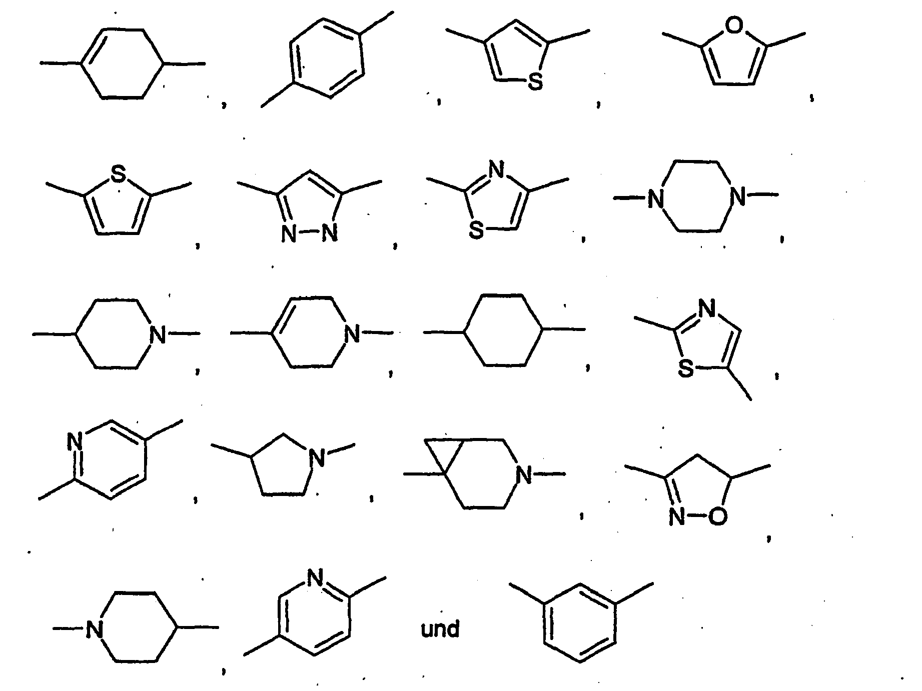 die optional Substituenten aus der Gruppe H, F, Cl, Br, OH, CF₃, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl tragen können;
bevorzugt 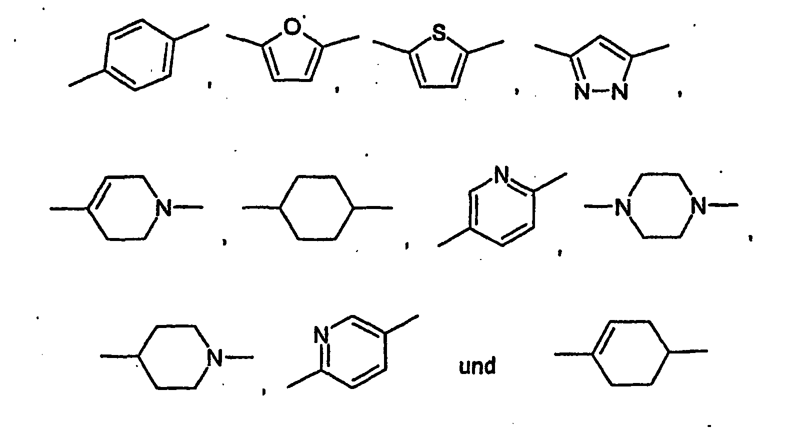 die optional die vorstehend genannten Substituenten tragen können;
R22, R23, R26, R28 unabhängig voneinander H, (C₁-C₈)-Alkyl;
oder
R22 und R23 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R27, R29, R30 unabhängig voneinander H, (C₁-C₈)-Alkyl;
K eine Bindung, O, OCH₂, CH₂O, S, SO, SO₂, N(R35), N(R36)CO, N-SO₂, CON(R37), (C(R38)(R39))ᵥ, CO, (R31)C=C(R32), C≡C, SCH₂, SO₂CH₂, bevorzugt eine Bindung, O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂, besonders bevorzugt OCH₂, CH₂O, N(R36)CO, CON(R37), (C(R38)(R39))₂, (R31)C=C(R32), C≡C, SCH₂, SO₂CH₂, ganz besonders: bevorzugt OCH₂, CH₂O, CON(R37), C≡C, SCH₂;
v 1, 2, 3, bevorzugt 2;
R31, R32, R35, R36, R37, R38, R39 unabhängig voneinander H, (C₁-C₈)-Alkyl;
R2 (C₁-C₈)-Alkyl, (C₁-C₄)-Alkoxy-(C₁-C₄)-alkyl, ein 3 bis 10-gliedriger mono-, bi-, tri- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, (C₀-C₂)-Alkylen-aryl, Oxo, CO(R41), CON(R42)(R43), Hydroxy, N(R45)CO(C₁-C₆)-Alkyl, N(R46)(R47) oder SO₂CH₃; bevorzugt (C₁-C₈)-Alkyl, (C₁-C₄)-Alkoxy-(C₁-C₄)-alkyl, ein 3 bis 10-gliedriger mono- oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, Oxo, CO(R41), CON(R42)(R43), N(R45)CO(C₁-C₆)-Alkyl oder SO₂CH₃;
R41, R42, R43, R45, R46, R47 unabhängig voneinander H, (C₁-C₈)-Alkyl;
oder
R42 und R43, R46 und R47 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
L eine Gruppe der Formel (C(R48)(R49))ₘ, in der 0 oder 1 Glied ersetzt sein kann durch O, wobei L in dem Fall, dass Q 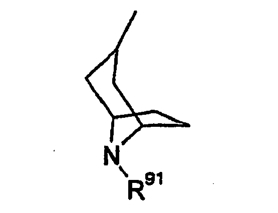 bedeutet, O ist,
m 1, 2, 3, 4, bevorzugt 1 oder 2;
R48, R49 H;
R50 H, (C₁-C₈)-Alkyl, bevorzugt H;
Q 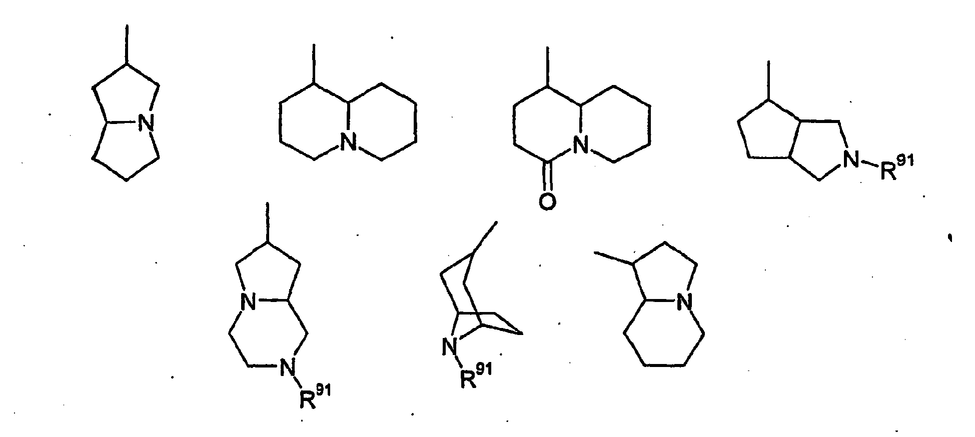
R91 H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96; bevorzugt H, (C₁-C₆)-Alkyl, (CR93R94)_{o'}-R95;
R92 H, (C₁-C₈)-Alkyl;
R93, R94 unabhängig voneinander H, (C₁-C₈)-Alkyl, OH, (C₃-C₈)-Cycloalkyl, (C₁-C₄)-Alkoxy-(C₁-C₄)-alkyl, bevorzugt H, (C₁-C₈)-Alkyl;
o', p' unabhängig voneinander 0, 1, 2, 3, 4, 5, 6, bevorzugt 0, 1, 2, 3;
R95, R96 unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104); Oxo, OH; bevorzugt unabhängig voneinander OH, O-(C₁-C₈)-Alkyl, CON(R97)(R98), ein 4-6 gliedriges Ringsystem mit 0 bis 2 Heteroatomen ausgewählt aus der Gruppe N und O, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH, wobei das Ringsystem besonders bevorzugt unsubstituiert ist;
R97, R98, R99, R100, R103, R104 unabhängig voneinander H, (C₁-C₈)-Alkyl;
oder
R97 und R98 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R101, R102 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 unabhängig voneinander H, (C₁-C₈)-Alkyl;
oder
R117 und R118 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q', r' unabhängig voneinander 0, 1, 2, 3, 4, 5, 6;
R108, R111 unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
R120, R121, R122, R123, R124, R125, R126, R127 unabhängig voneinander H, (C₁-C₈)-Alkyl;
oder
R120 und R121, R124 und R125 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
deren N-Oxide sowie deren physiologisch verträgliche Salze.

4. Verbindungen nach Anspruch 3, **dadurch gekennzeichnet, dass** Q die folgenden Bedeutungen aufweist 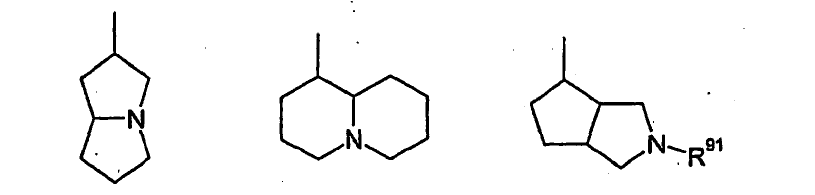 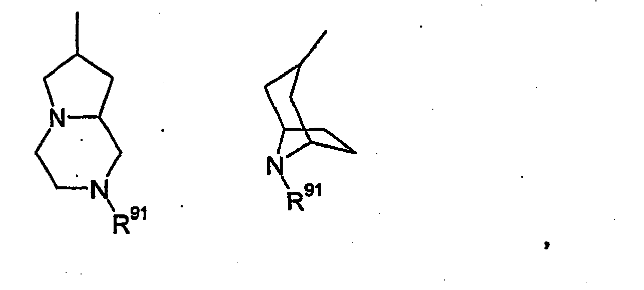 bevorzugt 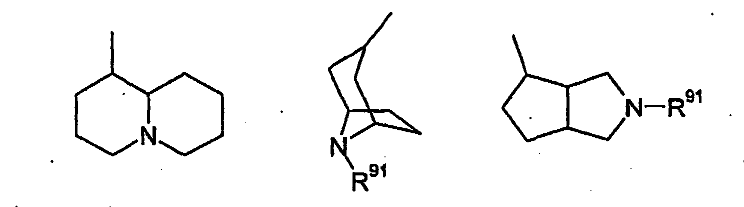
R91 H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96; bevorzugt H, (C₁-C₆)-Alkyl, (CR93R94)_{o'}-R95;
R92 H, (C₁-C₈)-Alkyl;
R93, R94 unabhängig voneinander H, (C₁-C₈)-Alkyl, OH, (C₃-C₈)-Cycloalkyl, (C₁-C₄)-Alkoxy-(C₁-C₄)-alkyl, bevorzugt H, (C₁-C₈)-Alkyl;
o', p' unabhängig voneinander 0, 1, 2, 3, 4, 5, 6, bevorzugt 0, 1, 2, 3;
R95, R96 unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH; bevorzugt unabhängig voneinander OH, O-(C₁-C₈)-Alkyl, CON(R97)(R98), ein 4-6 gliedriges Ringsystem mit 0 bis 2 Heteroatomen ausgewählt aus der Gruppe N und O, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH, wobei das Ringsystem besonders bevorzugt unsubstituiert ist;
R97, R98, R99, R100, R103, R104 unabhängig voneinander H, (C₁-C₈)-Alkyl;
oder
R97 und R98 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R101, R102 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(RI16), N(R117)(R118), CO₂(R119), SO₂Me;
R105, R106, R107, R108, R109, R110, R112, R113, R114, R115, R116, R117, R118, R119 unabhängig voneinander H, (C₁-C₈)-Alkyl;
oder
R117 und R118 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q', r' unabhängig voneinander 0, 1, 2, 3, 4, 5, 6;
R108, R111 unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
R120, R121, R122, R123, R124, R125, R126, R127 unabhängig voneinander H, (C₁-C₈)-Alkyl;
oder
R120 und R121, R124 und R125 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

5. Verbindungen nach Anspruch 4, **dadurch gekennzeichnet, dass** Q die folgenden Bedeutungen aufweist

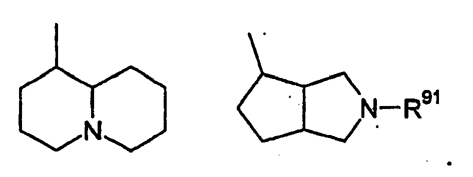

6. Verbindungen nach Anspruch 4, worin Q die folgende Bedeutung aufweist 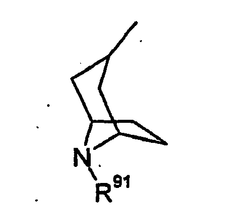
R91 H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96; bevorzugt H, (C₁-C₆)-Alkyl, (CR93R94)_{o'}-R95;
R92 H, (C₁-C₈)-Alkyl;
R93, R94 unabhängig voneinander H, (C₁-C₈)-Alkyl, OH, (C₃-C₈)-Cycloalkyl, (C₁-C₄)-Alkoxy-(C₁-C₄)-alkyl, bevorzugt H, (C₁-C₈)-Alkyl;
o', p' unabhängig voneinander 0, 1, 2, 3, 4, 5, 6, bevorzugt 0, 1,2,3;
R95, R96 unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH; bevorzugt unabhängig voneinander OH, O-(C₁-C₈)-Alkyl, CON(R97)(R98), ein 4-6 gliedriges Ringsystem mit 0 bis 2 Heteroatomen ausgewählt aus der Gruppe N und O das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH, wobei das Ringsystem besonders bevorzugt unsubstituiert ist;
R97, R98, R99, R100, R103, R104 unabhängig voneinander H, (C₁-C₈)-Alkyl;
oder
R97 und R98 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R101, R102 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 unabhängig voneinander H, (C₁-C₈)-Alkyl;
oder
R117 und R118 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q', r' unabhängig voneinander 0, 1, 2, 3, 4, 5, 6;
R108,R111 unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
R120, R121, R122, R123, R124; R125, R126, R127 unabhängig voneinander H, (C₁-C₈)-Alkyl;
oder
R120 und R121, R124 und R125 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
wobei in den Verbindungen der Formel I
L O bedeutet.

7. Verbindungen nach einem der Ansprüche 1 bis 5, **dadurch gekennzeichnet, dass** L ausgewählt ist aus O und CH₂O, bevorzugt O, wobei wobei L in dem Fall, dass Q 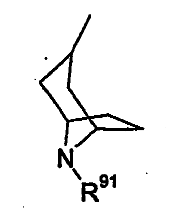 bedeutet, O ist.

8. Verbindungen nach Anspruch 7, worin die Gruppe L-Q die folgenden Bedeutungen aufweist: 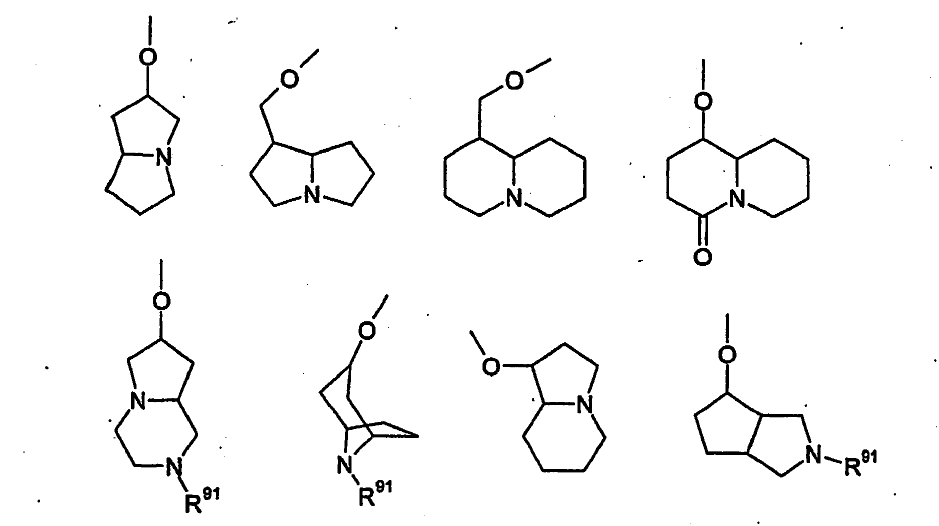 bevorzugt 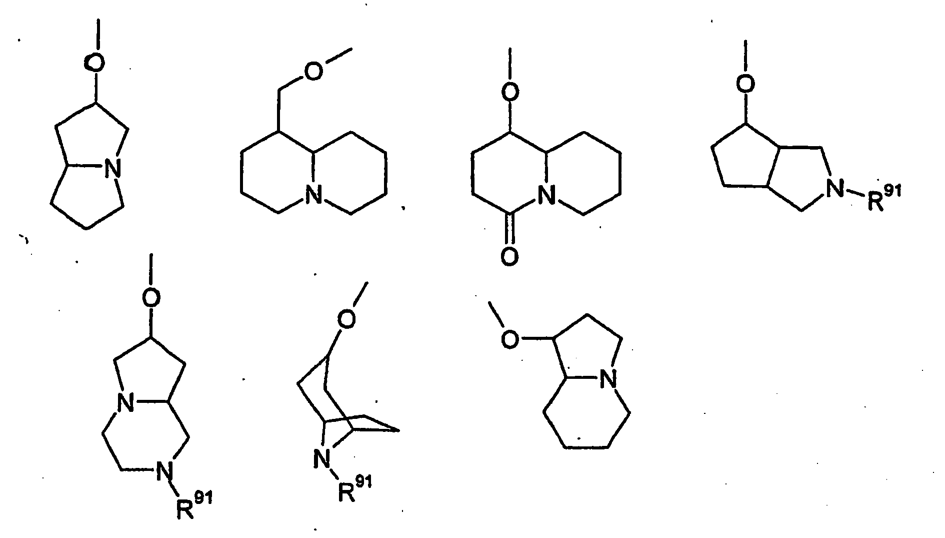 besonders bevorzugt 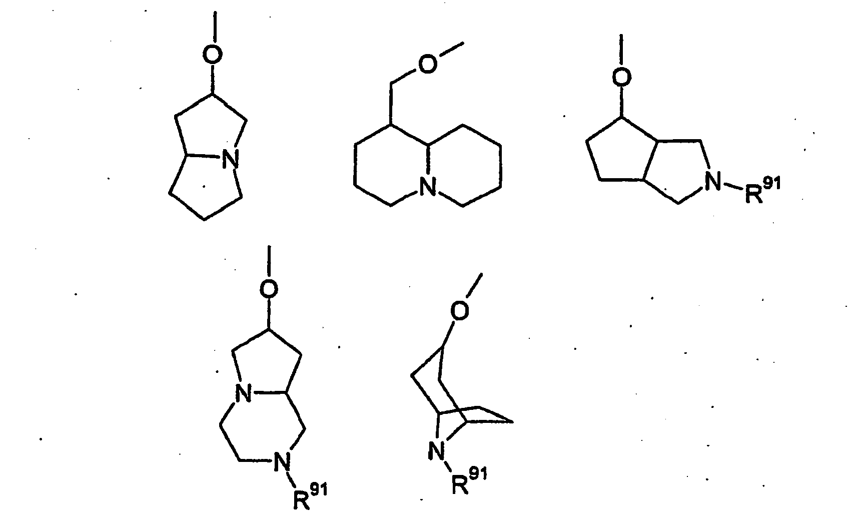 ganz besonders bevorzugt 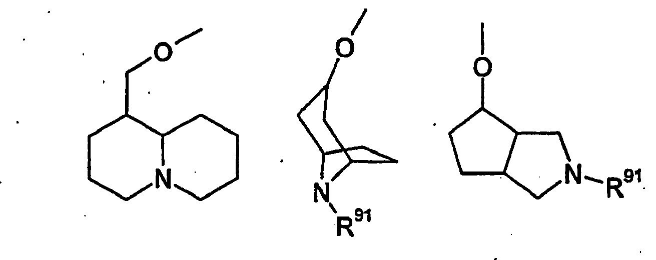
R91 H, (C₁-C₄)-Alkoxy-(C₁-C₄)-alkyl, (C₁-C₆)-Alkyl, (C₂-C₆)-Alkenyl, (C₂-C₆)-Alkinyl, CO(R92), (CR93R94)_{o'}-R95, CO(CR93R94)_{p'}-R96; bevorzugt H, (C₁-C₆)-Alkyl, (CR93R94)_{o'}-R95;
R92 H, (C₁-C₈)-Alkyl;
R93, R94 unabhängig voneinander H, (C₁-C₈)-Alkyl, OH, (C₃-C₈)-Cycloalkyl, (C₁-C₄)-Alkoxy-(C₁-C₄)-alkyl, bevorzugt H, (C₁-C₈)-Alkyl;
o', p' unabhängig voneinander 0, 1, 2, 3, 4, 5, 6, bevorzugt 0,1,2,3;
R95, R96 unabhängig voneinander OH, O-(C₁-C₈)-Alkyl, CON(R97)(R98), N(R99)CO(R100), N(R101)(R102), CO₂(R103), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH; bevorzugt unabhängig voneinander OH, O-(C₁-C₈)-Alkyl, CON(R97)(R98), ein 4-6 gliedriges Ringsystem mit 0 bis 2 Heteroatomen ausgewählt aus der Gruppe N und O, das substituiert sein kann mit einem oder mehreren der folgenden Substituenten: F, Cl, Br, CF₃, (C₁-C₈)-Alkyl, O-(C₁-C₈)-Alkyl, CO(R104), Oxo, OH, wobei das Ringsystem besonders bevorzugt unsubstituiert ist;
R97, R98, R99, R100, R103, R104 unabhängig voneinander H, (C₁-C₈)-Alkyl;
oder
R97 und R98 bilden optional zusammen mit dem Stickstoff atom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R101, R102 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R105), (CR106R107)_{q'}-R108, CO(CR109R110)_{r'}-R111; oder R101 und R102 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R112), Oxo, OH, (C₁-C₄)-Alkoxy-(C₁-C₄)-alkyl, Hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 unabhängig voneinander H, (C₁-C₈)-Alkyl;
oder
R117 und R118 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q', r' unabhängig voneinander 0, 1, 2, 3, 4, 5, 6;
R108, R111 unabhängig voneinander OH, O-(C₁-C₈)-Alkyl, CON(R120)(R121), N(R122)CO(R123), N(R124)(R125), CO₂(R126), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R127), Oxo, OH;
R120, R121, R122, R123, R124, R125, R126, R127 unabhängig voneinander H, (C₁-C₈)-Alkyl;
oder
R120 und R121, R124 und R125 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

9. Verbindungen der Formel I gemäß einem der Ansprüche 1 bis 8, **dadurch gekennzeichnet, dass**
A,B,D,G unabhängig voneinander N oder C(R3) bedeuten und die Gesamtzahl der Stickstoffatome in diesem Ring 0-2, bevorzugt 0 oder 1, besonders bevorzugt 0 beträgt.

10. Verbindungen der Formel I, gemäß einem der Ansprüche 1 bis 8,
worin bedeuten
K O OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂, bevorzugt OCH₂, CH₂O, N(R36)CO, CON(R37), (C(R38)(R39))₂, (R31)C=C(R32), C≡C, SCH₂, SO₂CH₂, besonders bevorzugt OCH₂, CH₂O, CON(R37), C≡C, SCH₂; wobei
v 1,2,3, bevorzugt 2;
R31, R32, R35, R36, R37, R38, R39 unabhängig voneinander H, (C₁-C₈)-Alkyl bedeuten.

11. Verbindungen nach einem der Ansprüche 1 bis 9,
worin bedeuten
A,B,D,G C(R3);
R3 H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀ - C₈)-Alkylen-aryl, N(R4)(R5), SO₂-CH₃, CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)(R14))ₓ-O(R15); ganz besonders bevorzugt H, F, (C₁-C₆)-Alkyl; weiter bevorzugt H, F, CH₃; insbesondere bevorzugt H;
R4, R5, R6, R7, R8 unabhängig voneinander H, (C₁-C₈)-Alkyl;
oder
R4 und R5, R6 und R7 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R9, R12 unabhängig voneinander H, (C₁-C₈)-Alkyl;
R13, R14 H;
R15 H, (C₁-C₆)-Alkyl;
x 0, 1, 2, bevorzugt 0, 1, besonders bevorzugt 1.

12. Verbindungen nach einem der Ansprüche 1 bis 11, **dadurch gekennzeichnet, dass** X Bindung oder N(R16) bedeutet, worin R16 H oder (C₁-C₈)-Alkyl bedeutet.

13. Arzneimittel enthaltend eine oder mehrere der Verbindungen der Formel I gemäß einem oder mehreren der Ansprüche 1 bis 12.

14. Arzneimittel enthaltend eine oder mehrere der Verbindungen der Formel I gemäß einem oder mehreren der Ansprüche 1 bis 12 und einen oder mehrere Wirkstoffe, die günstige Wirkungen auf Stoffwechselstörungen oder damit assozierte Erkrankungen haben.

15. Arzneimittel enthaltend eine oder mehrere der Verbindungen der Formel I gemäß einem oder mehreren der Ansprüche 1 bis 12 und ein oder mehrere Antidiabetika.

16. Arzneimittel enthaltend eine oder mehrere der Verbindungen der Formel I gemäß einem oder mehreren der Ansprüche 1 bis 12 und einen oder mehrere Lipidmodulatoren.

17. Verwendung der Verbindungen der Formel I gemäß einem oder mehreren der Ansprüche 1 bis 12 zur Herstellung eines Arzneimittels zur Behandlung und/oder Prävention von Störungen des Fettsäurestoffwechsels und Glucoseverwertungsstörungen.

18. Verwendung der Verbindungen der Formel I gemäß einem oder mehreren der Ansprüche 1 bis 12 zur Herstellung eines Arzneimittels zur Behandlung und/oder Prävention von Störungen, bei denen Insulin Resistenz eine Rolle spielt

19. Verwendung der Verbindungen der Formel I gemäß einem oder mehreren der Ansprüche 1 bis 12 zur Herstellung eines Arzneimittels zur Behandlung und/oder Prävention von Diabetes mellitus und der damit verbundenen Folgeerkrankungen.

20. Verwendung der Verbindungen der Formel I gemäß einem oder mehreren der Ansprüche 1 bis 12 zur Herstellung eines Arzneimittels zur Behandlung und/oder Prävention von Dyslipidämien und deren Folgen.

21. Verwendung der Verbindungen der Formel I gemäß einem oder mehreren der Ansprüche 1 bis 12 zur Herstellung eines Arzneimittels zur Behandlung und/oder Prävention von Zuständen, die mit dem Metabolischen Syndrom assoziert sind.

22. Verwendung der Verbindungen der Formel I gemäß einem oder mehreren der Ansprüche 1 bis 12 zur Herstellung eines Arzneimittels zur Behandlung und/oder Prävention von Obesitas und damit verbundenen Folgeerkrankungen.

23. Verwendung der Verbindungen gemäß einem oder mehreren der Ansprüche 1 bis 12 in Kombination mit mindestens einem weiteren Wirkstoff zur Herstellung eines Arzneimittels zur Behandlung und/oder Prävention von Störungen des Fettsäurestoffwechsels und Glucoseverwertungsstörungen.

24. Verwendung der Verbindungen -gemäß einem oder mehreren der Ansprüche 1 bis 12 in Kombination mit mindestens einem weiteren Wirkstoff zur Herstellung eines Arzneimittels zur Behandlung und/oder Prävention von Störungen, bei denen Insulin Resistenz eine Rolle spielt.

25. Verfahren zur Herstellung eines Arzneimittels enthaltend eine oder mehrere der Verbindungen gemäß einem oder mehreren der Ansprüche 1 bis 12, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 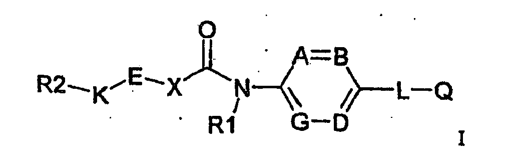 in which the meanings are
A, B, D, G independently of one another N, C(R3);
or
groups A and B or groups D and G are in each case C(R3) and form together a 5- or 6-membered carbocyclic or heterocyclic radical to result overall in a bicyclic system;
R3 H, F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, (C₂-C₆)-alkynyl, (C₀-C₈)-alkylene-aryl, O-(C₀-C₈)-alkylene-aryl, S-aryl, N(R4)(R5), SO₂-CH₃, COOH, COO-(C₁-C₆)-alkyl, CON(R6)(R7), N(R8)CO(R9), N(R10)SO₂(R11), CO(R12), (CR13R14)ₓ-O(R15);
R4, R5, R6, R7, R8, R10 or independently of one another H, (C₁-C₈)-alkyl;
R4 and R5, R6 and R7 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R9, R11, R12 independently of one another H, (C₁-C₈)-alkyl, aryl;
R13, R14 independently of one another H, (C₁-C₈)-alkyl;
R15 H, (C₁-C₆)-alkyl, aryl;
x 0, 1, 2, 3, 4, 5, 6;
R1 H, (C₁-C₈)-alkyl, (C₃-C₆)-alkenyl, (C₃-C₆)-alkynyl;
X N(R16), O, a bond, (R17)C=C(R18), C≡C, a group of the formula (CR19R20)_{y}, in which one or two (CR19R20) groups may be replaced by Y to result in a chemically reasonable radical;
Y O, S, N(R21), C=O;
R16, R17, R18 independently of one another H, (C₁-C₈)-alkyl;
R19, R20 independently of one another H, (C₁-C₄)-alkyl, where R19 and R20 in the y groups may in each case have identical or different meanings;
y 1, 2, 3, 4, 5, 6;
R21 H, (C₁-C₈)-alkyl;
E 3-14 membered bivalent carbo- or heterocyclic ring structure having 0-4 heteroatoms from the group of N, O and S, which may optionally have substituents from the group of H, F, Cl, Br, I, OH, CF₃, CN, OCF₃, oxo, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cydoalkyl, O-(C₃-C₈)-cydoalkyl, (C₃-C₈)-cycloalkenyl, O-(C₃-C₈)-cycloalkenyl, (C₂-C₆)-alkynyl, (C₀-C₈)-alkylene-aryl, O-(C₀-C₈)-alkylene-aryl, S-aryl, N(R22)(R23), SO₂-CH₃, CON(R24)(R25), N(R26)CO(R27), N(R28)SO₂(R29), CO(R30) and be mono- or bicyclic, E not being a tetrazol-5-yl group;
R22,R23,R24,R25,R26,R28 independently of one another H, (C₁-C₈)-alkyl, aryl;
or
R22 and R23, R24 and R25 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R27, R29, R30 independently of one another H, (C₁-C₈)-alkyl, aryl;
K a bond, C≡C, (R31)C=C(R32), a group of the formula (CR33R34)_{z} in which one or more (CR33R34) groups may be replaced by Z to result in a chemically reasonable radical, preferably a bond, O, OCH₂, CH₂O, S, SO, SO₂, N(R35), N(R36)CO, CON(R37), (C(R38)(R39))ᵥ, CO, (R31)C=C(R32), C ≡C, SCH₂, SO₂CH₂;
v 1, 2, 3, 4;
R31, R32, R35, R36, R37, R38, R39 independently of one another H, (C₁-C₈)-alkyl;
Z O, S, N(R40), CO, SO, SO₂;
R33, R34 independently of one another H, (C₁-C₈)-alkyl, hydroxy-(C₁-C₄)-alkyl, hydroxy, (C₁-C₄)-alkoxy-(C₁-C₄)-alkyl, where R38 and R39 in the z groups may in each case have identical or different meanings;
z 1, 2, 3, 4, 5, 6;
R40 H, (C₁-C₈)-alkyl;
R2 H, (C₁-C₈)-alkyl, (C₁-C₈)-alkoxy-(C₁-C₄)-alkyl, (C₃-C₈)-alkenyl, (C₃-C₈)-alkynyl, a 3 to 10-membered mono-, bi-, tri- or spirocyclic ring which may include 0 to 4 heteroatoms selected from the group of oxygen, nitrogen and sulfur, where the ring system may additionally be substituted by one or more of the following substituents: F, Cl, Br, CF₃, NO₂, CN, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R41), CON(R42)(R43), hydroxy, COO(R44), N(R45)CO(C₁-C₆)-alkyl, N(R46)(R47), SO₂CH₃, SCF₃ or S-(C₁-C₆)-alkyl, R2 not being a tetrazol-5-yl group;
R41, R42, R43, R44, R45, R46, R47 independently of one another H, (C₁-C₈)-alkyl;
or
R42 and R43, R46 and R47 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
E, K and R2 together form a tricyclic system where the rings may independently of one another be saturated, partially saturated or unsaturated and in each case comprise 3 - 8 ring atoms;
L a group of the formula (CR48R49)ₘ, in which one or more (CR48R49) groups may be replaced by M to result in a chemically reasonable radical, where L is O when Q is 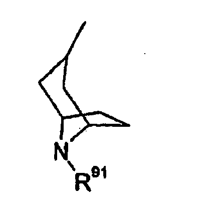 where R91 is defined below;
M O;
m 1, 2, 3, 4, 5, 6, preferably 1, 2, 3, 4, particularly preferably 1, 2;
R48, R49, R50 independently of one another H, (C₁-C₆)-alkyl, aryl;
Q 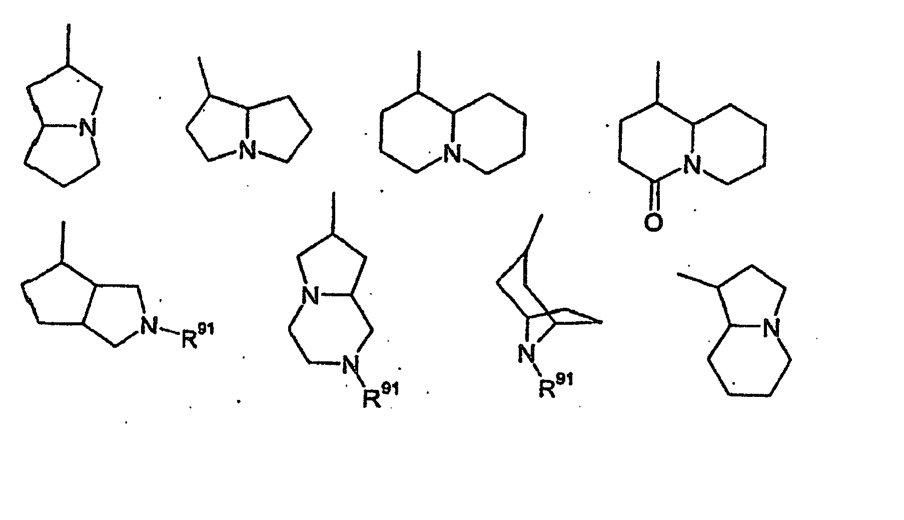
R91 H, (C₁-C₄)-alkoxy-(C₁-C₄)-alkyl, (C₁-C₆)-alkyl, (C₂-C₆)-alkenyl, (C₂-C₆)-alkynyl, CO(R92), (CR93R94)_{o'}-R95, CO(CR93R94)_{p'}-R96;
R92 H, (C₁-C₈)-alkyl;
R93, R94 independently of one another H, (C₁-C₈)-alkyl, OH, (C₃-C₈)-cycloalkyl, (C₁-C₄)-alkoxy-(C₁-C₄)-alkyl;
o', p' independently of one another 0, 1, 2, 3, 4, 5, 6;
R95, R96 independently of one another OH, O-(C₁-C₈)-alkyl, CON(R97)(R98), N(R99)CO(R100), N(R101)(R1 02), CO₂(R103), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R104), oxo, OH;
R97, R98, R99, R100, R103, R104 independently of one another H, (C₁-C₈)-alkyl;
or
R97 and R98 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R101, R102 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R105), (CR106R107)_{q'}-R108, CO(CR109R110)_{r'}-R111; or R101 and R102 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R112), oxo, OH, (C₁-C₄)-alkoxy-(C₁-C₄)-alkyl, hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 independently of one another H, (C₁-C₈)-alkyl;
or
R117 and R118 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q', r' independently of one another 0, 1, 2, 3, 4, 5, 6;
R108, R111 independently of one another OH, O-(C₁-C₈)-alkyl, CON(R120)(R121), N(R122)CO(R123), N(R124)(R125), CO₂(R126), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R127), oxo, OH;
R120, R121, R122, R123, R124, R125, R126, R127 independently of one another H, (C₁-C₈)-alkyl;
or
R120 and R121, R124 and R125 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
the N-oxides thereof and the physiologically tolerated salts thereof.

2. A compound of the formula I as claimed in claim 1, in which the meanings are
L a group of the formula (CR48R49)ₘ, in which one or more (CR48R49) groups may be replaced by M to result in a chemically reasonable radical, where L is O when Q is 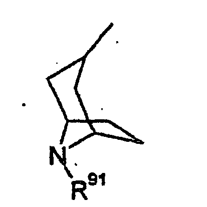
M O;
m 1, 2, 3, 4, 5, 6, preferably 1, 2, 3, 4, particularly preferably 1, 2;
R48, R49, R50 independently of one another H, (C₁-C₆)-alkyl, aryl, preferably H, (C₁-C₆)-alkyl, particularly preferably H.

3. A compound of the formula I as claimed in claim 1 or 2,
in which the meanings are
A,B,D,G independently of one another N, C(R3) or groups A and B or D and G are each C(R3) and form together an ortho-phenylene unit to result overall in a 1,4-disubstituted naphthalene system; preferably independently of one another N or C(R3), where the total number of nitrogen atoms in the ring is 0 - 2, preferably 0 or 1, and particularly preferably A, B, D and G are C(R3);
R3 H, F, Cl, Br, CF₃, CN, O-(C₁-C₆)-alkyl, O-(C₁-C₄)-alkoxy-(C₁-C₄)-alkyl, S-(C₁-C₆)-alkyl, (C₁-C₆)-alkyl, (C₀-C₈)-alkylene-aryl, O-(C₀ - C₈)-alkylene-aryl, N(R4)(R5), SO₂-CH₃, CON(R6)(R7), N(R8)CO(R9), CO(R12), (CR13R14)ₓ-O(R15); preferably H, F, Cl, Br, CF₃, CN, O-(C₁-C₆)-alkyl, (C₁-C₆)-alkyl, SO₂-CH₃, CON(R6)(R7), N(R8)CO(R9), CO(R12), (CR13R14)ₓ-O(R15), particularly preferably H, F, Cl, CF₃, CN, (C₁-C₆)-alkyl, (C(R13)(R14))ₓ-O(R15); very particularly preferably H, F, Cl, (C₁-C₆)-alkyl; more preferably H, F, CH₃; especially preferably H;
R4, R5, R6, R7, R8 independently of one another H, (C₁-C₈)-alkyl;
or
R4 and R5, R6 and R7 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R9, R12 independently of one another H, (C₁-C₈)-alkyl;
R13, R14 H;
R15 H, (C₁-C₆)-alkyl;
x 0, 1, 2, preferably 0, 1, particularly preferably 1;
R1 H, (C₁-C₈)-alkyl;
X N(R16), a bond, (R17)C=C(R18), C≡C, CH₂-CH₂, YCH₂, CH₂Y, preferably N(R16), a bond;
Y O, S, N(R21);
R16, R17, R18 independently of one another H, (C₁-C₈)-alkyl;
R21 H, (C₁-C₈)-alkyl;
E 3-8 membered bivalent carbo- or heterocyclic ring structure having 0-4 heteroatoms from the group of N, O and S, which may optionally have substituents from the group of H, F, Cl, Br, OH, CF₃, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, O-(C₃-C₈)-cycloalkyl, (C₃-C₈)-cydoalkenyl, (C₂-C₆)-alkynyl, (C₀-C₈)-alkylene-aryl, O-(C₀-C₈)-alkylene-aryl, S-aryl, N(R22)(R23), SO₂-CH₃, N(R26)CO(R27), N(R28)SO₂(R29), CO(R30) and be mono- or bicyclic, E not being a tetrazol-5-yl group;
preferably 5-7 membered bivalent carbo- or heterocyclic ring structure having 0-3 heteroatoms from the group of N, O and S, which may optionally have substituents from the group of H, F, Cl, Br, OH, CF₃, CN, OCF₃, O-(C₁-C₆)-alkyl, S-(C₁-C₆)-alkyl, (C₁-C₆)-alkyl, (C₂-C₆)-alkenyl, O-(C₀-C₈)-alkylene-aryl, S-aryl, N(R22)(R23), SO₂-CH₃, N(R26)CO(R27), CO(R30) and be mono-orbicyclic;
particularly preferably 5-7 membered bivalent carbo- or heterocyclic ring structure having 0-2 heteroatoms from the group of N, O and S, which may optionally have substituents from the group of H, F, Cl, Br, OH, CF₃, OCF₃, O-(C₁-C₆)-alkyl, (C₁-C₆)-alkyl, (C₂-C₆)-alkenyl, N(R22)(R23), SO₂-CH₃, CO(R30), preferably H, F, Cl, Br, OH, CF₃, (C₁-C₆)-alkyl, O-(C₁-C₆)-alkyl,
e.g. E is selected from the group consisting of 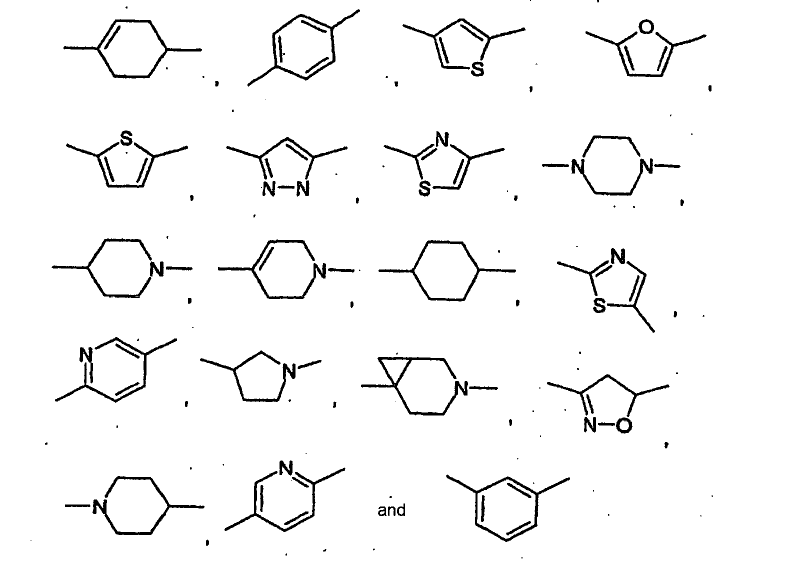 which may optionally have substituents from the group of H, F, Cl, Br, OH, CF₃, OCF₃, O-(C₁-C₆)-alkyl, (C₁-C₆)-alkyl, (C₂-C₆)-alkenyl, N(R22)(R23), SO₂-CH₃, CO(R30), preferably H, F, Cl, Br, OH, CF₃, (C₁-C₆)-alkyl, O-(C₁-C₆)-alkyl;
preferably 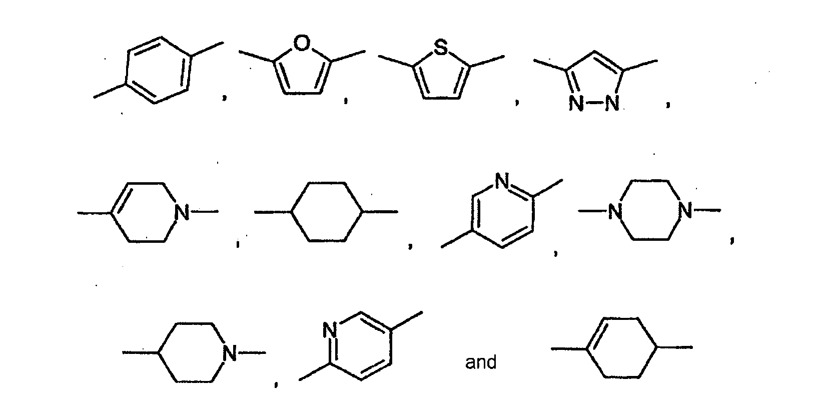 which may optionally have the aforementioned substituents;
R22,R23,R26,R28 or independently of one another H, (C₁-C₈)-alkyl;
R22 and R23 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R27,R29,R30 independently of one another H, (C₁-C₈)-alkyl;
K a bond, O, OCH₂, CH₂O, S, SO, SO₂, N(R35), N(R36)CO, N-SO₂, CON(R37), (C(R38)(R39))ᵥ, CO, (R31)C=C(R32), C≡C, SCH₂, SO₂CH₂, preferably a bond, O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂, particularly preferably OCH₂, CH₂O, N(R36)CO, CON(R37), (C(R38)(R39))₂, (R31)C=C(R32), C≡C, SCH₂, SO₂CH₂, very particularly preferably OCH₂, CH₂O, CON(R37), C≡C, SCH₂;
v 1, 2, 3, preferably 2;
R31, R32, R35, R36, R37, R38, R39 independently of one another H, (C₁-C₈)-alkyl;
R2 (C₁-C₈)-alkyl, (C₁-C₄)-alkoxy-(C₁-C₄)-alkyl, a 3 to 10-membered mono-, bi-, tri- or spirocyclic ring which may include 0 to 3 heteroatoms selected from the group of oxygen, nitrogen and sulfur, where the ring system may additionally be substituted by one or more of the following substituents: F, Cl, Br, CF₃, CN, (C₁-C₆)-alkyl, O-(C₁-C₈)-alkyl, (C₀-C₂)-alkylene-aryl, oxo, CO(R41), CON(R42)(R43), hydroxy, N(R45)CO(C₁-C₆)-alkyl, N(R46)(R47) or SO₂CH₃; preferably (C₁-C₈)-alkyl, (C₁-C₄)-alkoxy-(C₁-C₄)-alkyl, a 3 to 10-membered mono- or bicyclic ring which may include 0 to 2 heteroatoms selected from the group of oxygen, nitrogen and sulfur, where the ring system may additionally be substituted by F, Cl, Br, CF₃, CN, (C₁-C₆)-alkyl, O-(C₁-C₈)-alkyl, oxo, CO(R41), CON(R42)(R43), N(R45)CO(C₁-C₆)-alkyl or SO₂CH₃;
R41, R42, R43, R45, R46, R47 independently of one another H, (C₁-C₈)-alkyl;
or
R42 and R43, R46 and R47 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
L a group of the formula (C(R48)(R49))ₘ, in which 0 or 1 member may be replaced by O, where L is O when Q is 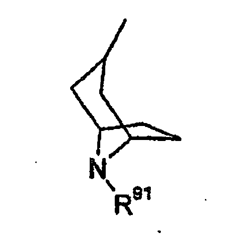
m 1, 2, 3, 4, preferably 1 or 2;
R48, R49 H;
R50 H, (C₁-C₈)-alkyl, preferably H;
Q 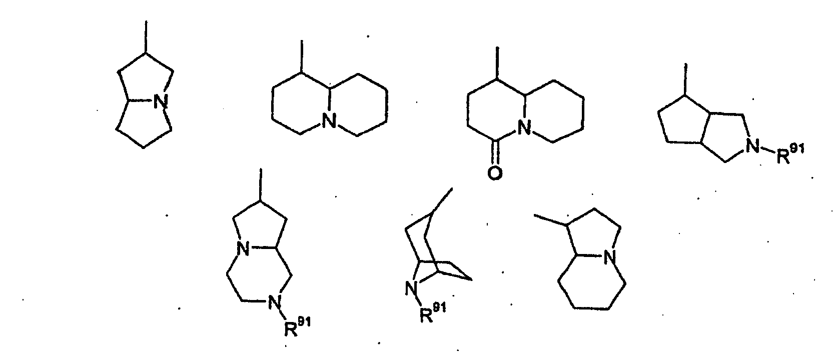
R91 H, (C₁-C₄)-alkoxy-(C₁-C₄)-alkyl, (C₁-C₆)-alkyl, (C₂-C₆)-alkenyl, (C₂-C₆)-alkynyl, CO(R92), (CR93R94)_{o'}-R95, CO(CR93R94)_{p'}-R96; preferably H, (C₁-C₆)-alkyl, (CR93R94)_{o'}-R95;
R92 H, (C₁-C₈)-alkyl;
R93, R94 independently of one another H, (C₁-C₈)-alkyl, OH, (C₃-C₈)-cycloalkyl, (C₁-C₄)-alkoxy-(C₁-C₄)-alkyl, preferably H, (C₁-C₈)-alkyl;
o', p' independently of one another 0, 1, 2, 3, 4, 5, 6, preferably 0, 1, 2, 3;
R95, R96 independently of one another OH, O-(C₁-C₈)-alkyl, CON(R97)(R98), N(R99)CO(R100), N(R101)(R102), CO₂(R103), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R104), oxo, OH; preferably independently of one another OH, O-(C₁-C₈)-alkyl, CON(R97)(R98), a 4-6 membered ring system having 0 to 2 heteroatoms selected from the group of N and O, which may be substituted by one or more of the following substituents: F, Cl, Br, CF₃, (C₁-C₈)-alkyl, O-(C₁-C₈)-alkyl, CO(R104), oxo, OH, the ring system being particularly preferably unsubstituted;
R97, R98, R99, R100, R103, R104 independently of one another H, (C₁-C₈)-alkyl;
or
R97 and R98 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R101, R102 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R105), (CR106R107)_{q'}-R108, CO(CR109R110)_{r'}-R111; or R101 and R102 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R112), oxo, OH, (C₁-C₄)-alkoxy-(C₁-C₄)-alkyl, hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116,R117,R118,R119 independently of one another H, (C₁-C₈)-alkyl;
or
R117 and R118 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q', r' independently of one another 0, 1, 2, 3, 4, 5, 6;
R108, R111 independently of one another OH, O-(C₁-C₈)-alkyl, CON(R120)(R121), N(R122)CO(R123), N(R124)(R125), CO₂(R126), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, 0-(C₁-C₈)-alkyl, CO(R127), oxo, OH;
R120, R121, R122, R123, R124, R125, R126, R127 independently of one another H, (C₁-C₈)-alkyl;
or
R120 and R121, R124 and R125 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
the N-oxides thereof and the physiologically tolerated salts thereof.

4. A compound as claimed in claim 3, wherein Q has the following meanings: 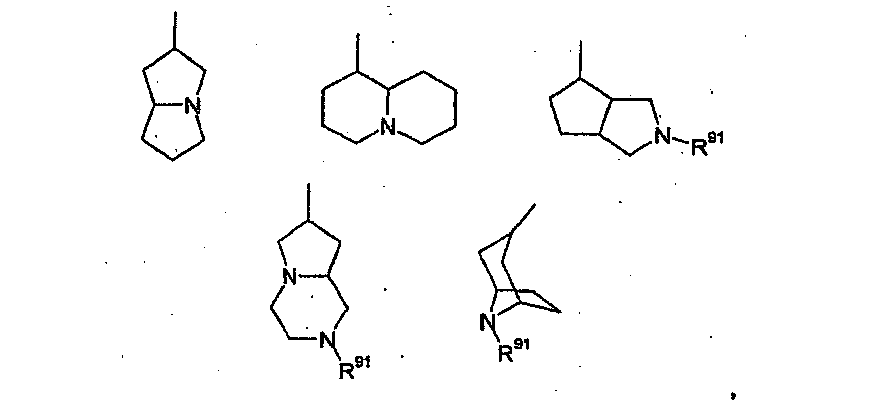 preferably 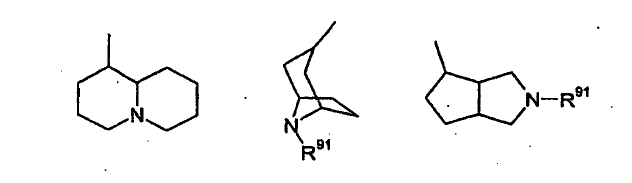
R91 H, (C₁-C₄)-alkoxy-(C₁-C₄)-alkyl; (C₁-C₆)-alkyl, (C₂-C₆)-alkenyl, (C₂-C₆)-alkynyl, CO(R92), (CR93R94)_{o'}-R95, CO(CR93R94)_{p'}-R96; preferably H, (C₁-C₆)-alkyl, (CR93R94)_{o'}-R95;
R92 H, (C₁-C₈)-alkyl;
R93, R94 independently of one another H, (C₁-C₈)-alkyl, OH, (C₃-C₈)-cycloalkyl, (C₁-C₄)-alkoxy-(C₁-C₄)-alkyl, preferably H, (C₁-C₈)-alkyl;
o', p' independently of one another 0, 1, 2, 3, 4, 5, 6, preferably 0, 1, 2, 3;
R95, R96 independently of one another OH, O-(C₁-C₈)-alkyl, CON(R97)(R98), N(R99)CO(R100), N(R101 )(R102), CO₂(R103), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R104), oxo, OH; preferably independently of one another OH, O-(C₁-C₈)-alkyl, CON(R97)(R98), a 4-6 membered ring system having 0 to 2 heteroatoms selected from the group of N and O, which may be substituted by one or more of the following substituents: F, Cl, Br, CF₃, (C₁-C₈)-alkyl, O-(C₁-C₈)-alkyl, CO(R104), oxo, OH, the ring system being particularly preferably unsubstituted;
R97, R98, R99, R100, R103, R104 independently of one another H, (C₁-C₈)-alkyl;
or
R97 and R98 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R101, R102 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R105), (CR106R107)_{q'}-R108, CO(CR109R110)_{r'}-R111; or R101 and R102 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R112), oxo, OH, (C₁-C₄)-alkoxy-(C₁-C₄)-alkyl, hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 independently of one another H, (C₁-C₈)-alkyl;
or
R117 and R118 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q', r' independently of one another 0, 1, 2, 3, 4, 5, 6;
R108, R111 independently of one another OH, O-(C₁-C₈)-alkyl, CON(R120)(R121), N(R122)CO(R123), N(R124)(R125), CO₂(R126), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R127), oxo, OH;
R120, R121, R122, R123, R124, R125, R126, R127 independently of one another H, (C₁-C₈)-alkyl;
or
R120 and R121, R124 and R125 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur.

5. A compound as claimed in claim 4, wherein Q has the following meanings

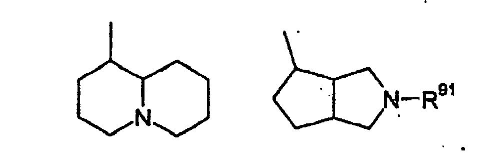

6. A compound as claimed in claim 4, in which Q has the following meaning 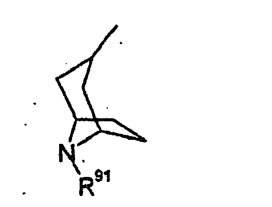
R91 H, (C₁-C₄)-alkoxy-(C₁-C₄)-alkyl, (C₁-C₆)-alkyl, (C₂-C₆)-alkenyl, (C₂-C₆)-alkynyl, CO(R92), (CR93R94)_{o'}-R95, CO(CR93R94)_{p'}-R96, preferably H, (C₁-C₆)-alkyl, (CR93R94)_{o'}-R95;
R92 H, (C₁-C₈)-alkyl;
R93, R94 independently of one another H, (C₁-C₈)-alkyl, OH, (C₃-C₈)-cycloalkyl, (C₁-C₄)-alkoxy-(C₁-C₄)-alkyl, preferably H, (C₁-C₈)-alkyl;
o', p' independently of one another 0, 1, 2, 3, 4, 5, 6, preferably 0, 1, 2, 3;
R95, R96 independently of one another OH, O-(C₁-C₈)-alkyl, CON(R97)(R98), N(R99)CO(R100), N(R101)(R102), CO₂(R103), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R104), oxo, OH; preferably independently of one another OH, O-(C₁-C₈)-alkyl, CON(R97)(R98), a 4-6 membered ring system having 0 to 2 heteroatoms selected from the group of N and O, which may be substituted by one or more of the following substituents: F, Cl, Br, CF₃, (C₁-C₈)-alkyl, O-(C₁-C₈)-alkyl, CO(R104), oxo, OH, the ring system being particularly preferably unsubstituted;
R97, R98, R99, R100, R103, R104 independently of one another H, (C₁-C₈)-alkyl;
or
R97 and R98 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R101,R102 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R105), (CR106R107)_{q'}-R108, CO(CR109R110)_{r'}-R111; or R101 and R102 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R112), oxo, OH, (C₁-C₄)-alkoxy-(C₁-C₄)-alkyl, hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 independently of one another H, (C₁-C₈)-alkyl;
or
R117 and R118 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q', r' independently of one another 0, 1, 2, 3, 4, 5, 6;
R108, R111 independently of one another OH, O-(C₁-C₈)-alkyl, CON(R120)(R121), N(R122)CO(R123), N(R124)(R125), CO₂(R126), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R127), oxo, OH;
R120, R121, R122, R123, R124, R125, R126, R127 independently of one another H, (C₁-C₈)-alkyl;
or
R120 and R121, R124 and R125 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur, in the compounds of the formula I
L being O.

7. A compound as claimed in any of claims 1 to 5, wherein L is selected from O and CH₂O, preferably O, in the case that Q is 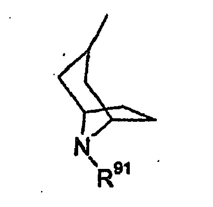
L being O.

8. A compound as claimed in claim 7 in which the group L-Q has the following meanings: 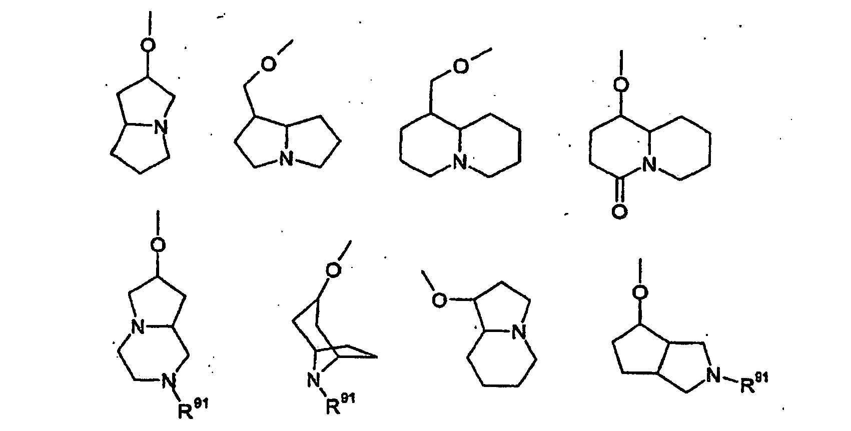 preferably 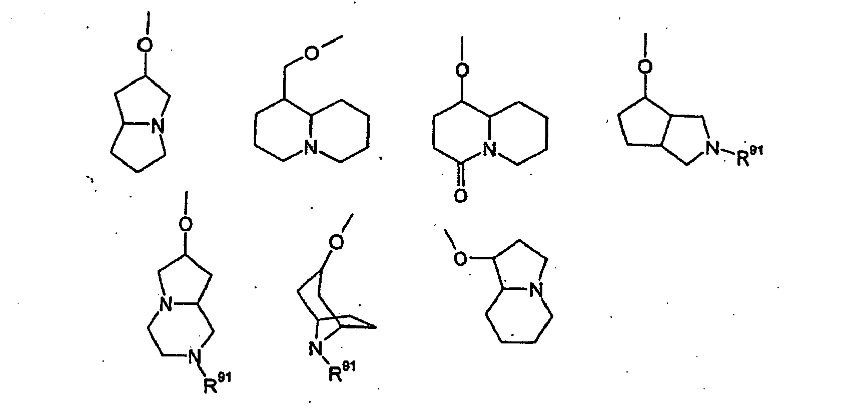 particularly preferably 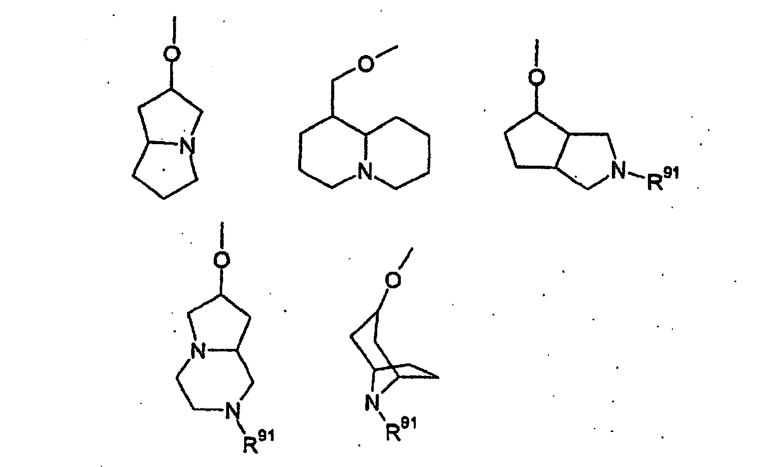 very particularly preferably 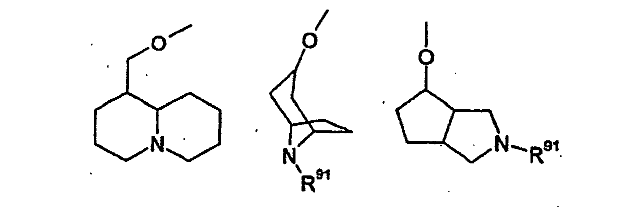
R91 H, (C₁-C₄)-alkoxy-(C₁-C₄)-alkyl, (C₁-C₆)-alkyl, (C₂-C₆)-alkenyl, (C₂-C₆)-alkynyl, CO(R92), (CR93R94)_{o'}-R95, CO(CR93R94)_{p'}-R96; preferably H, (C₁-C₆)-alkyl, (CR93R94)_{o'}-R95;
R92 H, (C₁-C₈)-alkyl;
R93, R94 independently of one another H, (C₁-C₈)-alkyl, OH, (C₃-C₈)-cycloalkyl, (C₁-C₄)-alkoxy-(C₁-C₄)-alkyl, preferably H, (C₁-C₈)-alkyl;
o', p' independently of one another 0, 1, 2, 3, 4, 5, 6, preferably 0, 1, 2, 3;
R95, R96 independently of one another OH, O-(C₁-C₈)-alkyl, CON(R97)(R98), N(R99)CO(R100), N(R101 )(R102), CO₂(R103), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R104), oxo, OH; preferably independently of one another OH, O-(C₁-C₈)-alkyl, CON(R97)(R98), a 4-6 membered ring system having 0 to 2 heteroatoms selected from the group of N and O, which may be substituted by one or more of the following subsituents: F, Cl, Br, CF₃, (C₁-C₈)-alkyl, O-(C₁-C₈)-alkyl, CO(R104), oxo, OH, the ring system being particularly preferably unsubstituted;
R97, R98, R99, R100, R103, R104 independently of one another H, (C₁-C₈)-alkyl;
or
R97 and R98 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R101, R102 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R105), (CR106R107)_{q'}-R108, CO(CR109R110)_{r'}-R111; or R101 and R102 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R112), oxo, OH, (C₁-C₄)-alkoxy-(C₁-C₄)-alkyl, hydroxy-(C₁-C₄)-alkyl, CON(R113)(R114), N(R115)CO(R116), N(R117)(R118), CO₂(R119), SO₂Me;
R105, R106, R107, R108, R109, R110, R112, R113, R114, R115, R116, R117, R118, R119 independently of one another H, (C₁-C₈)-alkyl;
or
R117 and R118 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q', r' independently of one another 0, 1, 2, 3, 4, 5, 6;
R108, R111 independently of one another OH, O-(C₁-C₈)-alkyl, CON(R120)(R121), N(R122)CO(R123), N(R124)(R125), CO₂(R126), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R127), oxo, OH;
R120, R121, R122, R123, R124, R125, R126, R127 independently of one another H, (C₁-C₈)-alkyl;
or
R120 and R121, R124 and R125 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur.

9. A compound of the formula I as claimed in any of claims 1 to 8, wherein
A,B,D,G are independently of one another N or C(R3) and the total number of nitrogen atoms in this ring is 0-2, preferably 0 or 1, particularly preferably 0.

10. A compound of the formula I as claimed in any of claims 1 to 8, in which the meanings are
K O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂, preferably OCH₂, CH₂O, N(R36)CO, CON(R37), (C(R38)(R39))₂, (R31)C=C(R32), C≡C, SCH₂, SO₂CH₂, particularly preferably OCH₂, CH₂O, CON(R37), C≡C, SCH₂; where
v is 1, 2, 3, preferably 2;
R31, R32, R35, R36, R37, R38, R39 are independently of one another H, (C₁-C₈)-alkyl.

11. A compound as claimed in any of claims 1 to 9,
in which the meanings are
A,B,D,G C(R3);
R3 H, F, Cl, Br, CF₃, CN, O-(C₁-C₆)-alkyl, O-(C₁-C₄)-alkoxy-(C₁-C₄)-alkyl, S-(C₁-C₆)-alkyl, (C₁-C₆)-alkyl, (C₀-C₈)-alkylene-aryl, O-(C₀-C₈)-alkylene-aryl, N(R4)(R5), SO₂-CH₃, CON(R6)(R7), N(R8)CO(R9), CO(R12), (CR13R14)x-OR(15); preferably H, F, Cl, Br, CF₃, CN, O-(C₁-C₆)-alkyl, (C₁-C₆)-alkyl, SO₂-CH₃, CON(R6)(R7), N(R8)CO(R9), CO(R12), (CR13R14)ₓ-O(R15), particularly preferably H, F, Cl, CF₃, CN, (C₁-C₆)-alkyl, (C(R13)(R14))ₓ O(R15); very particularly preferably H, F, (C₁-C₆)-alkyl; more preferably H, F, CH₃; especially preferably H;
R4,R5,R6,R7,R8 independently of one another H, (C₁-C₈)-alkyl;
or
R4 and R5, R6 and R7 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R9, R12 independently of one another H, (C₁-C₈)-alkyl;
R13, R14 H;
R15 H, (C₁-C₆)-alkyl;
x 0,1,2, preferably 0,1, particularly preferably 1.

12. A compound as claimed in any of claims 1 to 11, wherein X is bond or N(R16) in which R16 is H or (C₁-C₈)-alkyl.

13. A medicament comprising one or more of the compounds of the formula I as claimed in one or more of claims 1 to 12.

14. A medicament comprising one or more of the compounds of the formula I as claimed in one or more of claims 1 to 12 and one or more active ingredients which have beneficial effects on metabolic disturbances or disorders associated therewith.

15. A medicament comprising one or more of the compounds of the formula I as claimed in one or more of claims 1 to 12 and one or more antidiabetics.

16. A medicament comprising one or more of the compounds of the formula I as claimed in one or more of claims 1 to 12 and one or more lipid modulators.

17. The use of the compounds of the formula I as claimed in one or more of claims 1 to 12 for the production of a medicament for the treatment and/or prevention of disorders of fatty acid metabolism and glucose utilization disorders.

18. The use of the compounds of the formula I as claimed in one or more of claims 1 to 12 for the production of a medicament for the treatment and/or prevention of disorders in which insulin resistance is involved.

19. The use of the compounds of the formula I as claimed in one or more of claims 1 to 12 for the production of a medicament for the treatment and/or prevention of diabetes mellitus and the sequelae associated therewith.

20. The use of the compounds of the formula I as claimed in one or more of claims 1 to 12 for the production of a medicament for the treatment and/or prevention of dyslipidemias and the sequelae thereof.

21. The use of the compounds of the formula I as claimed in one or more of claims 1 to 12 for the production of a medicament for the treatment and/or prevention of conditions associated with the metabolic syndrome.

22. The use of the compounds of the formula I as claimed in one or more of claims 1 to 12 for the production of a medicament for the treatment and/or prevention of obesity and sequelae associated therewith.

23. The use of the compounds as claimed in one or more of claims 1 to 12 for the production of a medicament in combination with at least one further active ingredient for the treatment and/or prevention of disorders of fatty acid metabolism and glucose utilization disorders.

24. The use of the compounds as claimed in one or more of claims 1 to 12 for the production of a medicament in combination with at least one further active ingredient for the treatment and/or prevention of disorders in which insulin resistance is involved.

25. A process for producing a medicament comprising one or more of the compounds as claimed in one or more of claims 1 to 12, which comprises mixing the active ingredient with a pharmaceutically suitable carrier, and converting this mixture into a form suitable for administration

## Revendications

1. Composés de formule I 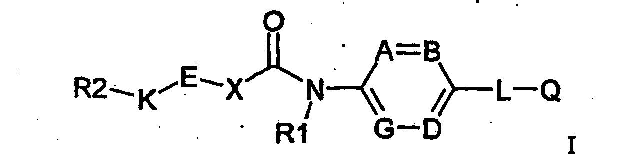 dans laquelle
A, B, D, G signifient, indépendamment l'un de 1 ' autre N, C(R3) ;
ou
les groupes A et B ou les groupes D et G représentent à chaque fois C(R3) et forment, ensemble, un radical carbocyclique ou hétérocyclique de 5 ou 6 chaînons, de telle manière qu'on obtient au total un système bicyclique ;
R3 signifie H, F, Cl, Br, I, OH, CF₃, NO₂, CN, OCF₃, O-(C₁-C₆)-alkyle, O-(C₁-C₄)-alcoxy-(C₁-C₄)-alkyle, S- (C₁-C₆)-alkyle, (C₁-C₆)-alkyle, (C₂-C₆)-alcényle, (C₃-C₈)-cycloalkyle, O-(C₃-C₈)-cycloalkyle, (C₃-C₈)-cycloalcényle, (C₂-C₆)-alcynyle, (C₀-C₈)-alkylénaryle, O-(C₀-C₈)-alkylénaryle, S-aryle, N(R4)(R5), SO₂-CH₃, COOH, COO- (C₁-C₆)-alkyle, CON(R6) (R7), N(R8)CO(R9), N(R10)SO₂(R11), CO(R12), (CR13R14)ₓ-O(R15) ;
R4, R5, R6, R7, R8, R10 signifient, indépendamment l'un de l'autre H, (C₁-C₈)-alkyle ;
ou
R4 et R5, R6 et R7 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R9, R11, R12 signifient, indépendamment l'un de l'autre H, (C₁-C₈)-alkyle, aryle ;
R13, R14 signifient, indépendamment l'un de l'autre H, (C₁-C₈)-alkyle ;
R15 signifie H, (C₁-C₆)-alkyle, aryle ;
x vaut 0, 1, 2, 3, 4, 5, 6 ;
R1 signifie H, (C₁-C₈)-alkyle, (C₃-C₆)-alcényle, (C₃-C₆)-alcynyle ;
X signifie N(R16), O, une liaison, (R17)C=C(R18), C=C, un groupe de formule (CR19R20)_{y}, où un ou deux groupes (CR19R20) peuvent être remplacés par Y, avec obtention d'un radical qui a chimiquement un sens ;
Y signifie O, S, N(R21), C=O ;
R16, R17, R18 signifient, indépendamment l'un de l'autre H, (C₁-C₈)-alkyle ;
R19, R20 signifient, indépendamment l'un de l'autre H, (C₁-C₄)-alkyle, où R19 et R20 dans les y groupes peuvent à chaque fois présenter des significations identiques ou différentes ;
y vaut 1, 2, 3, 4, 5, 6 ;
R21 signifie H, (C₁-C₈)-alkyle ;
E signifie une structure carbocyclique ou hétérocyclique divalente de 3-14 chaînons, comprenant 0-4 hétéroatomes du groupe formé par N, O et S, qui peut éventuellement porter des substituants du groupe formé par H, F, Cl, Br, I, OH, CF₃, CN, OCF₃, oxo, O- (C₁-C₆) -alkyle, O- (C₁-C₄)-alcoxy- (C₁-C₄) -alkyle, S-(C₁-C₆)-alkyle, (C₁-C₆)-alkyle, (C₂-C₆) -alcényle, (C₃-C₈) -cycloalkyle, O-(C₃-C₈)-cycloalkyle, (C₃-C₈) -cycloalcényle, O- (C₃-C₈)-cycloalcényle, (C₂-C₆) -alcynyle, (C₀-C₈)-alkylénaryle, O-(C₀-C₈)-alkylénaryle, S-aryle,N(R22) (R23) , SO₂-CH₃, CON(R24) (R25) , N(R26)CO(R27) , N(R28) SO₂ (R29) , CO(R30) et qui peut être monocyclique ou bicyclique, E n'étant pas un groupe tétrazol-5-yle ;
R22, R23, R24, R25, R26, R28 signifient, indépendamment l'un de l'autre H, (C₁-C₈)-alkyle, aryle ;
ou
R22 et R23, R24 et R25 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R27, R29, R30 signifient, indépendamment l'un de l'autre H, (C₁-C₈)-alkyle, aryle ;
K signifie une liaison, C≡C, (R31)C=C(R32), un groupe de formule (CR33R34)_{z}, dans laquelle un ou plusieurs groupes (CR33R34) peuvent être remplacés par Z, avec obtention d'un radical qui a chimiquement un sens, de préférence une liaison, O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂ ;
v vaut 1, 2, 3, 4 ;
R31, R32, R35, R36, R37, R38, R39 signifient, indépendamment l'un de l'autre H, (C₁-C₈)-alkyle ;
Z signifie O, S, N(R40), CO, SO, SO₂ ;
R33, R34 signifient, indépendamment l'un de l'autre H, (C₁-C₈)-alkyle, hydroxy-(C₁-C₄)-alkyle, hydroxy, (C₁-C₄)-alcoxy-(C₁-C₄)-alkyle, où R38 et R39 dans les z groupes peuvent présenter à chaque fois des significations identiques ou différentes ;
z vaut 1, 2, 3, 4, 5, 6 ;
R40 signifie H, (C₁-C₈)-alkyle ;
R2 signifie H, (C₁-C₈)-alkyle, (C₁-C₈)-alcoxy-(C₁-C₄)-alkyle, (C₃-C₈)-alcényle, (C₃-C₈)-alcynyle, un cycle monocyclique, bicyclique, tricyclique ou spirocyclique de 3 à 10 chaînons, qui peut comporter 0 à 4 hétéroatomes, choisis dans le groupe formé par l'oxygène, l'azote ou le soufre, le système cyclique pouvant en outre être substitué par un ou plusieurs des substituants suivants : F, Cl, Br, CF₃, NO₂, CN, (C₁-C₆)-alkyle, O- (C₁-C₈)-alkyle, (C₁-C₄) -alcoxy- (C₁-C₄) -alkyle, (C₀-C₈)-alkylénaryle, oxo, CO(R41), CON(R42)(R43), hydroxy, COO(R44), N(R45)CO(C₁-C₆)-alkyle, N(R46)(R47), SO₂CH₃, SCF₃ ou S-(C₁-C₆)-alkyle, R2 n'étant pas un groupe tétrazol-5-yle ;
R41, R42, R43, R44, R45, R46, R47 signifient, indépendamment l'un de l'autre H, (C₁-C₈)-alkyle ;
ou
R42 et R43, R46 et R47 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
E, K et R2 forment ensemble un tricycle, les cycles pouvant être, indépendamment l'un de l'autre, saturés, partiellement saturés ou insaturés et contenir à chaque fois 3-8 atomes de cycle ;
L signifie un groupe de formule (CR48R49)ₘ, où un ou plusieurs groupes (CR48R49) peuvent être remplacés par M, avec obtention d'un radical qui a chimiquement un sens, où L, dans le cas où Q représenterait 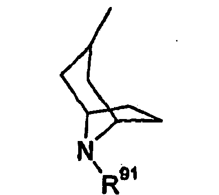 représente O, R91 étant défini ci-dessous ;
M signifie O ;
m vaut 1, 2, 3, 4, 5, 6, de préférence 1, 2, 3, 4, de manière particulièrement préférée 1, 2 ;
R48, R49, R50 signifient, indépendamment l'un de l'autre H, (C₁-C₆)-alkyle, aryle ;
Q signifie 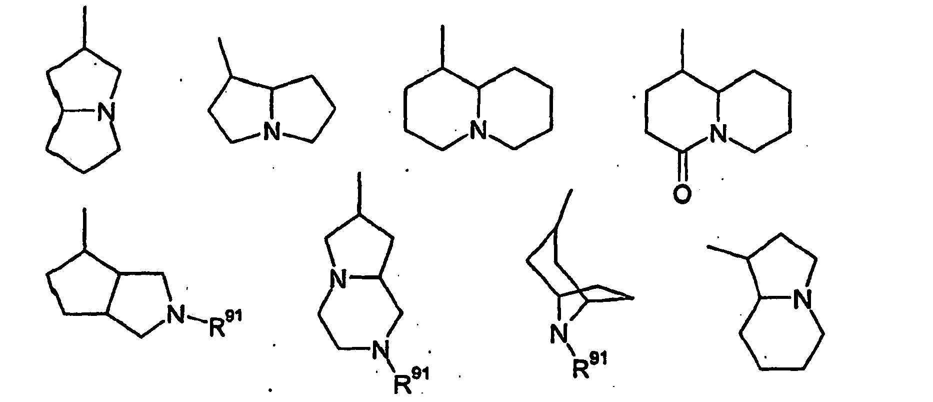
R91 signifie H, (C₁-C₄)-alcoxy-(C₁-C₄)-alkyle, (C₁-C₆)-alkyle, (C₂-C₆)-alcényle, (C₂-C₆)-alcynyle, CO(R92), (CR93R94)_{o'}-R95, CO(CR93R94)_{p'}-R96 ;
R92 signifie H, (C₁-C₈)-alkyle ;
R93, R94 signifient, indépendamment l'un de l'autre H, (C₁-C₈)-alkyle, OH, (C₃-C₈)-cycloalkyle, (C₁-C₄)-alcoxy-(C₁-C₄)-alkyle ;
o', p' valent, indépendamment l'un de l'autre 0, 1, 2, 3, 4, 5, 6 ;
R95, R96 signifient, indépendamment l'un de l'autre OH, O-(C₁-C₈)-alkyle, CON(R97)(R98), N(R99)CO(R100), N(R101)(R102), CO₂(R103), SO₂Me, CN, un système cyclique de 3-10 chaînons comprenant 0 à 3 hétéroatomes choisis dans le groupe formé par N, 0 et S, qui peut être substitué par un ou plusieurs des substituants suivants: F, Cl, Br, CF₃, (C₁-C₈)-alkyle, O-(C₁-C₈)-alkyle, CO(R104), oxo, OH ;
R97, R98, R99, R100, R103, R104 signifient, indépendamment l'un de l'autre H, (C₁-C₈)-alkyle ;
ou
R97 et R98 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R101, R102 signifient, indépendamment l'un de l'autre H, (C₁-C₆)-alkyle, (C₁-C₄)-alcoxy-(C₁-C₄)-alkyle, (C₂-C₆)-alcényle, (C₂-C₆)-alcynyle, CO(R105), (CR106R107)_{q'}-R108, CO(CR109R110)_{r'}-R111 ; ou R101 et R102 forment ensemble avec l'atome d'azote auquel ils sont liés, un cycle monocyclique, bicyclique ou spirocyclique de 4 à 10 chaînons, qui contient, outre l'atome d'azote, 0 à 3 hétéroatomes supplémentaires choisis dans le groupe formé par N, O et S et qui peut en outre être substitué par un ou plusieurs des substituants suivants: F, Cl, Br, CF₃, O-(C₁-C₈)-alkyle, (C₁-C₆)-alkyle, CO(R112), oxo, OH, (C₁-C₄)-alcoxy-(C₁-C₄)-alkyle, hydroxy- (C₁-C₄)-alkyle, CON(R113)(R114), N(R115)CO(R116), N(R117)(R118), CO₂(R119), SO₂Me ;
R105, R106, R107, R108, R109, R110, R112, R113, R114, R115, R116, R117, R118, R119 signifient, indépendamment l'un de l'autre H, (C₁-C₈)-alkyle ;
ou
R117 et R118 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
q', r' valent, indépendamment l'un de l'autre 0, 1, 2, 3, 4, 5, 6 ;
R108, R111 signifient, indépendamment l'un de l'autre OH, O- (C₁-C₈) -alkyle, CON(R120) (R121) , N(R122)CO(R123), N(R124) (R125), CO₂(R126), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈) -alkyle, O- (C₁-C₈)-alkyle, CO(R127), oxo, OH ;
R120, R121, R122, R123, R124, R125, R126, R127 signifient, indépendamment l'un de l'autre H, (C₁-C₈)-alkyle ;
ou
R120 et R121, R124 et R125 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
leurs N-oxydes ainsi que leurs sels physiologiquement acceptables.

2. Composés de formule I selon la revendication 1, dans laquelle :
L signifie un groupe de formule (CR48R49)ₘ, où un ou plusieurs groupes (CR48R49) peuvent être remplacés par M, avec obtention d'un radical qui a chimiquement un sens, où L, dans le cas où Q représenterait 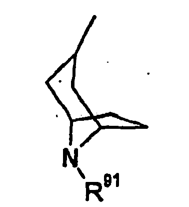 représente O,
M signifie O ;
m vaut 1, 2, 3, 4, 5, 6, de préférence 1, 2, 3, 4, de manière particulièrement préférée 1, 2 ;
R48, R49, R50 signifient, indépendamment l'un de l'autre H, (C₁-C₆)-alkyle, aryle, de préférence H, (C₁-C₆)-alkyle, de manière particulièrement préférée H.

3. Composés de formule I selon la revendication 1 ou 2, dans laquelle
A, B, D, G signifient, indépendamment l'un de l'autre N, C(R3) ou les groupes A et B ou D et G représentent à chaque fois C(R3) et forment ensemble une unité ortho-phénylène, de telle manière qu'on obtient au total un système naphtalénique 1,4-disubstitué ; de préférence, indépendamment l'un de l'autre N ou C(R3), le nombre total d'atomes d'azote dans le cycle étant 0-2, de préférence 0 ou 1, de manière particulièrement préférée A, B, D et G représentent C(R3) ;
R3 signifie H, F, Cl, Br, CF₃, CN, O-(C₁-C₆)-alkyle, O-(C₁-C₄)-alcoxy-(C₁-C₄)-alkyle, S-(C₁-C₆)-alkyle, (C₁-C₆)-alkyle, (C₀-C₈)-alkylénaryle, O-(C₀-C₈)-alkylénaryle, N(R4)(R5), SO₂-CH₃, CON(R6)(R7), N (R8) CO (R9) , CO(R12), (CR13R14)ₓ-O(R15); de préférence H, F, Cl, Br, CF₃, CN, O-(C₁-C₆)-alkyle, (C₁-C₆)-alkyle, SO₂-CH₃, CON(R6)(R7), N(R8)CO(R9), CO(R12), (CR13R14)ₓ-O(R15), de manière particulièrement préférée H, F, Cl, CF₃, CN, (C₁-C₆)-alkyle, (C(R13)(R14))ₓ-O(R15); de manière tout particulièrement préférée H, F, (C₁-C₆)-alkyle ; de manière encore plus particulièrement préférée H, F, CH₃ ; de manière particulièrement préférée H ;
R4, R5, R6, R7, R8 signifient, indépendamment l'un de l'autre H, (C₁-C₈)-alkyle ;
ou
R4 et R5, R6 et R7 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆) -alkyle, oxygène et soufre ;
R9, R12 signifient, indépendamment l'un de l'autre H, (C₁-C₈)-alkyle ;
R13, R14 signifient H ;
R15 signifie H, (C₁-C₆) -alkyle ;
x vaut 0, 1, 2, de préférence 0, 1, de manière particulièrement préférée 1 ;
R1 signifie H, (C₁-C₈)-alkyle ;
X signifie N(R16), une liaison, (R17)C=C(R18), C≡C, CH₂-CH₂, YCH₂, CH₂Y, de préférence N(R16), une liaison ;
Y signifie O, S, N(R21) ;
R16, R17, R18 signifient, indépendamment l'un de l'autre H, (C₁-C₈)-alkyle ;
R21 signifie H, (C₁-C₈)-alkyle ;
E signifie une structure divalente carbocyclique ou hétérocyclique de 3-8 chaînons comprenant 0-4 hétéroatomes du groupe N, O et S, qui peut éventuellement porter des substituants du groupe formé par H, F, Cl, Br, OH, CF₃, CN, OCF₃, O-(C₁-C₆)-alkyle, O(C₁-C₄)-alcoxy-(C₁-C₄)-alkyle, S-(C₁-C₆)-alkyle, (C₁-C₆)-alkyle, (C₂-C₆)-alcényle, O-(C₃-C₈)-cycloalkyle, (C₃-C₈) -cycloalcényle, (C₂-C₆)-alcynyle, (C₀-C₈)-alkylénaryle , O-(C₀-C₈)-alkylénaryle, S-aryle, N(R22)(R23), SO₂-CH₃, N(R26)CO(R27), N(R28)SO₂(R29), CO(R30) et qui peut être monocyclique ou bicyclique, E n'étant pas un groupe tétrazol-5-yle ; de préférence une structure carbocyclique ou hétérocyclique de 5-7 chaînons comprenant 0-3 hétéroatomes du groupe N, O et S, qui peut éventuellement porter des substituants du groupe formé par H, F, Cl, Br, OH, CF₃, CN, OCF₃, O-(C₁-C₆)-alkyle, S- (C₁-C₆) -alkyle, (C₁-C₆)-alkyle, (C₂-C₆)-alcényle, O-(C₀-C₈)-alkylénaryle, S-aryle, N(R22)(R23), SO₂-CH₃, N(R26)CO(R27), CO(R30) et qui peut être monocyclique ou bicyclique ;
de manière particulièrement préférée une structure carbocyclique ou hétérocyclique divalente de 5-7 chaînons comprenant 0-2 hétéroatomes du groupe formé par N, O et S, qui peut éventuellement porter les substituants du groupe formé par H, F, Cl, Br, OH, CF₃, OCF₃, O-(C₁-C₆)-alkyle, (C₁-C₆)-alkyle, (C₂-C₆)-alcényle, N(R22)(R23), SO₂-CH₃, CO(R30), de préférence H, F, Cl, Br, OH, CF₃, (C₁-C₆)-alkyle, O-(C₁-C₆)-alkyle
par exemple, E est choisi dans le groupe constitué par 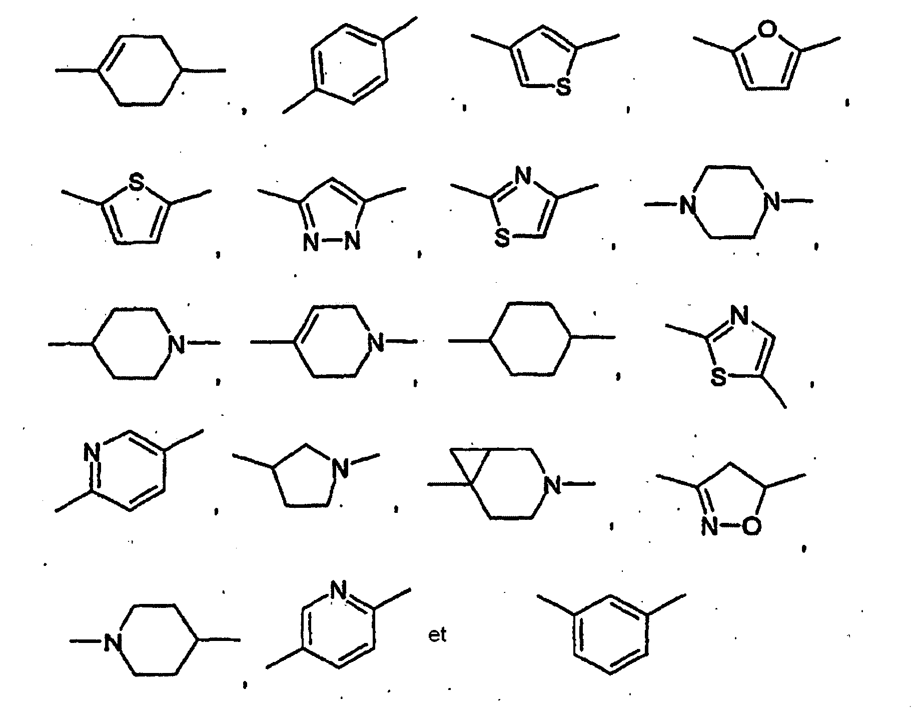 qui peuvent éventuellement porter des substituants du groupe formé par H, F, Cl, Br, OH, CF₃, OCF₃, 0-(C₁-C₆)-alkyle, (C₁-C₆)-alkyle, (C₂-C₆)-alcényle, N(R22)(R23), SO₂-CH₃, CO(R30), de préférence H, F, Cl, Br, OH, CF₃, (C₁-C₆)-alkyle, O-(C₁-C₆)-alkyle ; de préférence 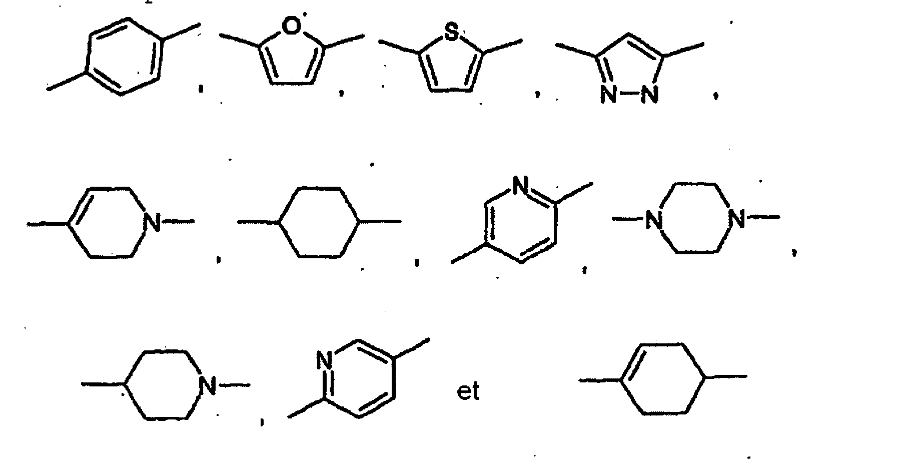 qui peuvent éventuellement porter les substituants susmentionnés ;
R22, R23, R26, R28 signifient, indépendamment l'un de l'autre H, (C₁-C₈)-alkyle ;
ou
R22 et R23 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R27, R29, R30 signifient, indépendamment l'un de l'autre H, (C₁-C₈)-alkyle ;
K signifie une liaison, O, OCH₂, CH₂O, S, SO, SO₂, N (R35) , N (R36) CO, N-SO₂, CON(R37), (C(R38)(R39))ᵥ, CO, (R31)C=C(R32), C=C, SCH₂, SO₂CH₂, de préférence une liaison, O, OCH₂, CH₂O, S, SO, SO₂, N(R35), N(R36)CO, CON(R37), (C(R38) (R39))ᵥ, CO, (R31) C=C (R32) , C=C, SCH₂, SO₂CH₂, de manière particulièrement préférée OCH₂, CH₂O, N(R36)CO, CON(R37), (C (R38) (R39))₂, (R31)C=C(R32), C≡C, SCH₂, SO₂CH₂, de manière tout particulièrement préférée OCH₂, CH₂O, CON(R37), C≡C , SCH₂ ;
v vaut 1, 2, 3, de préférence 2 ;
R31, R32, R35, R36, R37, R38, R39 signifient, indépendamment l'un de l'autre H, (C₁-C₈)-alkyle ;
R2 signifie (C₁-C₈)-alkyle, (C₁-C₄) -alcoxy- (C₁-C₄)-alkyle, un cycle monocyclique, bicyclique, tricyclique ou spirocyclique de 3 à 10 chaînons, qui peut comporter 0 à 3 hétéroatomes, choisis dans le groupe formé par l'oxygène, l'azote et le soufre, le système cyclique pouvant en outre être substitué par un ou plusieurs des substituants suivants : F, Cl, Br, CF₃, CN, (C₁-C₆)-alkyle, O-(C₁-C₈)-alkyle, (C₀-C₂)-alkylénaryle, oxo, CO (R41) , CON(R42)(R43), hydroxy, N(R45)CO (C₁-C₆)-alkyle, N(R46)(R47) ou SO₂CH₃ ; de préférence (C₁-C₈)-alkyle, (C₁-C₄) -alcoxy- (C₁-C₄) -alkyle, un cycle monocyclique ou bicyclique de 3 à 10 chaînons, qui peut comporter 0 à 2 hétéroatomes, choisis dans le groupe formé par l'oxygène, l'azote et le soufre, le système cyclique pouvant en outre être substitué par F, Cl, Br, CF₃, CN, (C₁-C₆)-alkyle, O- (C₁-C₈) -alkyle, oxo, CO(R41), CON(R42)(R43), N (R45) CO (C₁-C₆)-alkyle ou SO₂CH₃ ;
R41, R42, R43, R45, R46, R47 signifient, indépendamment l'un de l'autre H, (C₁-C₈)-alkyle ;
ou
R42 et R43, R46 et R47 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
L signifie un groupe de formule (C(R48)(R49))ₘ, dans laquelle 0 ou 1 chaînon peut être remplacé par O,
où L, dans le cas où Q signifierait 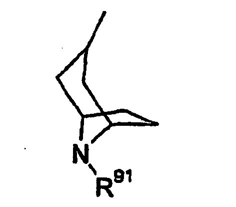 représente O,
m vaut 1, 2, 3, 4, de préférence 1 ou 2 ;
R48, R49 signifient H ;
R50 signifie H, (C₁-C₈)-alkyle, de préférence H ;
Q signifie 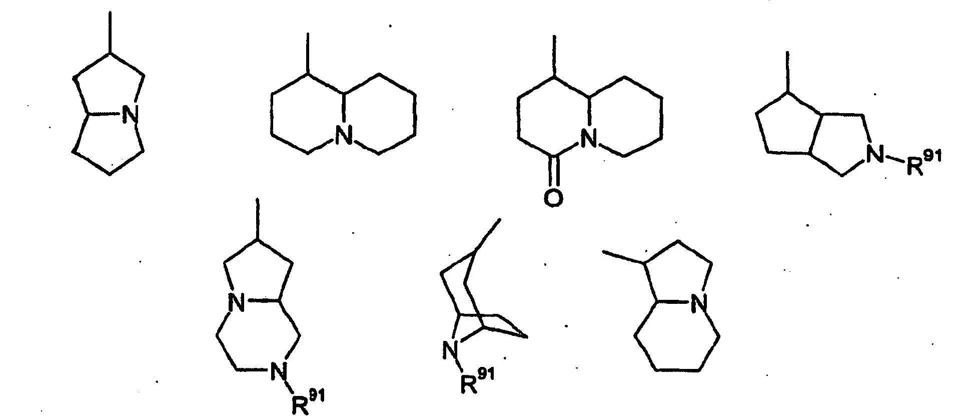
R91 signifie H, (C₁-C₄)-alcoxy-(C₁-C₄)-alkyle, (C₁-C₆)-alkyle, (C₂-C₆)-alcényle, (C₂-C₆)-alcynyle, CO(R92), (CR93R94)_{o'}-R95, CO(CR93R94)_{p'}-R96 ; de préférence H, (C₁-C₆)-alkyle, (CR93R94)_{o'}-R95 ;
R92 signifie H, (C₁-C₈)-alkyle ;
R93, R94 signifient, indépendamment l'un de l'autre H, (C₁-C₈)-alkyle, OH, (C₃-C₈) -cycloalkyle, (C₁-C₄)-alcoxy- (C₁-C₄)-alkyle, de préférence H, (C₁-C₈)-alkyle ;
o', p' valent, indépendamment l'un de l'autre 0, 1, 2, 3, 4, 5, 6, de préférence 0, 1, 2, 3 ;
R95, R96 signifient, indépendamment l'un de l'autre OH, O- (C₁-C₈) -alkyle, CON(R97) (R98) , N(R99)CO(R100) , N(R101)(R102), CO₂(R103), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈)-alkyle, O-(C₁-C₈)-alkyle, CO(R104), oxo, OH ; de préférence, indépendamment l'un de l'autre, OH, O-(C₁-C₈)-alkyle, CON(R97)(R98), un système cyclique de 4-6 chaînons comprenant 0 à 2 hétéroatomes choisis dans le groupe formé par N et O, qui peut être substitué par un ou plusieurs des substituants suivants: F, Cl, Br, CF₃, (C₁-C₈)-alkyle, O-(C₁-C₈)-alkyle, CO(R104), oxo, OH, le système cyclique étant de manière particulièrement préférée non substitué ;
R97, R98, R99, R100, R103, R104 signifient, indépendamment l'un de l'autre H, (C₁-C₈)-alkyle ;
ou
R97 et R98 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R101, R102 signifient, indépendamment l'un de l'autre H, (C₁-C₆)-alkyle, (C₁-C₄)-alcoxy- (C₁-C₄)-alkyle, (C₂-C₆)-alcényle, (C₂-C₆)-alcynyle, CO(R105), (CR106R107)_{q'}-R108, CO(CR109R110)_{r'}-R111 ; ou R101 et R102 forment ensemble avec l'atome d'azote auquel ils sont liés, un cycle monocyclique, bicyclique ou spirocyclique de 4 à 10 chaînons, qui contient, outre l'atome d'azote, 0 à 3 hétéroatomes supplémentaires choisis dans le groupe formé par N, O et S et qui peut en outre être substitué par un ou plusieurs des substituants suivants : F, Cl, Br, CF₃, O-(C₁-C₈)-alkyle, (C₁-C₆)-alkyle, CO (R112) , oxo, OH, (C₁-C₄)-alcoxy- (C₁-C₄) -alkyle, hydroxy- (C₁-C₄) -alkyle, CON(R113) (R114) , N(R115)CO(R116), N(Rl17) (Rl18), CO₂-(R119), SO₂Me ;
R105, R106, R107, R108, R109, R110, R112, R113, R114, R115, R116, R117, R118, R119 signifient, indépendamment l'un de l'autre H, (C₁-C₈)-alkyle ;
ou
R117 et R118 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
q', r' valent, indépendamment l'un de l'autre 0, 1, 2, 3, 4, 5, 6 ;
R108, R111 signifient, indépendamment l'un de l'autre OH, O-(C₁-C₈)-alkyle, CON(R120)(R121), N(R122)CO(R123), N(R124)(R125), CO₂(R126), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈)-alkyle, O-(C₁-C₈)-alkyle, CO(R127), oxo, OH ;
R120, R121, R122, R123, R124, R125, R126, R127 signifient, indépendamment l'un de l'autre H, (C₁-C₈)-alkyle ;
ou
R120 et R121, R124 et R125 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
leurs N-oxydes ainsi que leurs sels physiologiquement acceptables.

4. Composés selon la revendication 3, **caractérisés en ce que** Q présente les significations suivantes 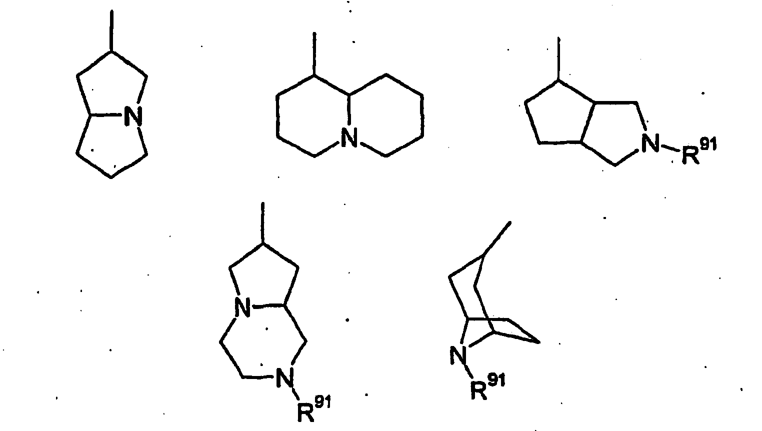 de préférence
R91 signifie H, (C₁-C₄)-alcoxy-(C₁-C₄)-alkyle, (C₁-C₆)-alkyle, (C₂-C₆)-alcényle, (C₂-C₆)-alcynyle, CO(R92), (CR93R94)_{o'}-R95, CO(CR93R94)_{p'}-R96 ; de préférence H, (C₁-C₆)-alkyle, (CR93R94)_{o'}-R95 ;
R92 signifie H, (C₁-C₈)-alkyle ;
R93, R94 signifient, indépendamment l'un de l'autre H, (C₁-C₈)-alkyle, OH, (C₃-C₈)-cycloalkyle, (C₁-C₄)-alcoxy- (C₁-C₄)-alkyle, de préférence H, (C₁-C₈)-alkyle ;
o', p' valent, indépendamment l'un de l'autre 0, 1, 2, 3, 4, 5, 6, de préférence 0, 1, 2, 3 ;
R95, R96 signifient, indépendamment l'un de l'autre OH, O- (C₁-C₈) -alkyle, CON(R97)(R98), N(R99)CO(R100), N(R101)(R102), CO₂(R103), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈)-alkyle, O-(C₁-C₈)-alkyle, CO(R104), oxo, OH ; de préférence, indépendamment l'un de l'autre, OH, O-(C₁-C₈)-alkyle, CON(R97)(R98), un système cyclique de 4-6 chaînons comprenant 0 à 2 hétéroatomes choisis dans le groupe formé par N et O, qui peut être substitué par un ou plusieurs des substituants suivants : F, Cl, Br, CF₃, (C₁-C₈)-alkyle, O- (C₁-C₈)-alkyle, CO(R104), oxo, OH, le système cyclique étant de manière particulièrement préférée non substitué ;
R97, R98, R99, R100, R103, R104 signifient, indépendamment l'un de l'autre H, (C₁-C₈)-alkyle ;
ou
R97 et R98 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R101, R102 signifient, indépendamment l'un de l'autre H, (C₁-C₆)-alkyle, (C₁-C₄)-alcoxy-(C₁-C₄)-alkyle, (C₂-C₆) -alcényle, (C₂-C₆) -alcynyle, CO(R105), (CR106R107)_{q'}-R108, CO(CR109R110)_{r'}-R111 ; ou R101 et R102 forment ensemble avec l'atome d'azote auquel ils sont liés, un cycle monocyclique, bicyclique ou spirocyclique de 4 à 10 chaînons, qui contient, outre l'atome d'azote, 0 à 3 hétéroatomes supplémentaires choisis dans le groupe formé par N, O et S et qui peut en outre être substitué par un ou plusieurs des substituants suivants : F, Cl, Br, CF₃, O- (C₁-C₈)-alkyle, (C₁-C₆)-alkyle, CO (R112) , oxo, OH, (C₁-C₄)-alcoxy- (C₁-C₄) -alkyle, hydroxy-(C₁-C₄)-alkyle, CON(R113)(R114), N(R115)CO(R116), N(R117)(R118), CO₂(R119), SO₂Me ;
R105, R106, R107, R108, R109, R110, R112, R113, R114, R115, R116, R117, R118, R119 signifient, indépendamment l'un de l'autre H, (C₁-C₈)-alkyle ;
ou
R117 et R118 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
q', r' valent indépendamment l'un de l'autre 0, 1, 2, 3, 4, 5, 6 ;
R108, R111 signifient, indépendamment l'un de l'autre OH, 0- (C₁-C₈) -alkyle, CON(R120) (R121) , N(R122)CO(R123) , N(R124) (R125) , CO₂(R126), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈)-alkyle, O-(C₁-C₈)-alkyle, CO(R127), oxo, OH ;
R120, R121, R122, R123, R124, R125, R126, R127 signifient, indépendamment l'un de l'autre H, (C₁-C₈)-alkyle ;
ou
R120 et R121, R124 et R125 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre.

5. Composés selon la revendication 4, **caractérisés que en ce** Q présente les significations suivantes

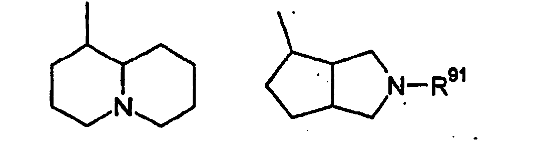

6. Composés selon la revendication 4, où Q présente la signification suivante 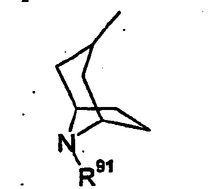
R91 signifie H, (C₁-C₄)-alcoxy-(C₁-C₄)-alkyle, (C₁-C₆)-alkyle, (C₂-C₆)-alcényle, (C₂-C₆)-alcynyle, CO(R92), (CR93R94)_{o'}-R95, CO(CR93R94)_{p'}-R96 ; de préférence H, (C₁-C₆)-alkyle, (CR93R94)_{o'}-R95 ;
R92 signifie H, (C₁-C₈)-alkyle ;
R93, R94 signifient, indépendamment l'un de l'autre H, (C₁-C₈)-alkyle, OH, (C₃-C₈)-cycloalkyle, (C₁-C₄)-alcoxy- (C₁-C₄) -alkyle, de préférence H, (C₁-C₈)-alkyle ;
o', p' valent, indépendamment l'un de l'autre 0, 1, 2, 3, 4, 5, 6, de préférence 0, 1, 2, 3 ;
R95, R96 signifient, indépendamment l'un de l'autre OH, O- (C₁-C₈) -alkyle, CON(R97) (R98) , N(R99)CO(R100), N(R101)(R102), CO₂(R103), SO₂Me, CN, un système cyclique de 3-10 chaînons comprenant 0 à 3 hétéroatomes choisis dans le groupe formé par N, 0 et S, qui peut être substitué par un ou plusieurs des substituants suivants : F, Cl, Br, CF₃, (C₁-C₈)-alkyle, O-(C₁-C₈)-alkyle, CO(R104), oxo, OH ; de préférence, indépendamment l'un de l'autre, OH, O-(C₁-C₈)-alkyle, CON(R97)(R98), un système cyclique de 4-6 chaînons comprenant 0 à 2 hétéroatomes choisis dans le groupe formé par N et O, qui peut être substitué par un ou plusieurs des substituants suivants : F, Cl, Br, CF₃, (C₁-C₈)-alkyle, O-(C₁-C₈)-alkyle, CO(R104), oxo, OH, le système cyclique étant de manière particulièrement préférée non substitué ;
R97, R98, R99, R100, R103, R104 signifient, indépendamment l'un de l'autre H, (C₁-C₈)-alkyle ;
ou
R97 et R98 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R101, R102 signifient, indépendamment l'un de l'autre H, (C₁-C₆)-alkyle, (C₁-C₄)-alcoxy-(C₁-C₄)-alkyle, (C₂-C₆)-alcényle, (C₂-C₆)-alcynyle, CO(R105), (CR106R107)_{q'}-R108, CO(CR109R110)_{r'}-R111; ou R101 et R102 forment ensemble avec l'atome d'azote auquel ils sont liés, un cycle monocyclique, bicyclique ou spirocyclique de 4 à 10 chaînons, qui contient, outre l'atome d'azote, 0 à 3 hétéroatomes supplémentaires choisis dans le groupe formé par N, O et S et qui peut en outre être substitué par un ou plusieurs des substituants suivants : F, Cl, Br, CF₃, O-(C₁-C₈)-alkyle, (C₁-C₆)-alkyle, CO(R112) , oxo, OH, (C₁-C₄)-alcoxy-(C₁-C₄)-alkyle, hydroxy- (C₁-C₄) -alkyle, CON(R113) (R114) , N(R115)CO(R116) , N(R117)(R118), CO₂(R119), SO₂Me ;
R105, R106, R107, R108, R109, R110, R112, R113, R114, R115, R116, R117, R118, R119 signifient, indépendamment l'un de l'autre H, (C₁-C₈)-alkyle ;
ou
R117 et R118 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
q', r' valent, indépendamment l'un de l'autre 0, 1, 2, 3, 4, 5, 6 ;
R108, R111 signifient, indépendamment l'un de l'autre OH, O- (C₁-C₈) -alkyle, CON(R120)(R121), N(R122)CO(R123), N(R124)(R125), CO₂(R126), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈)-alkyle, O-(C₁-C₈)-alkyle, CO(R127), oxo, OH ;
R120, R121, R122, R123, R124, R125, R126, R127 signifient, indépendamment l'un de l'autre H, (C₁-C₈)-alkyle ;
ou
R120 et R121, R124 et R125 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre;
où, dans les composés de formule I
L signifie O.

7. Composés selon l'une quelconque des revendications 1 à 5, **caractérisés en ce que** L est choisi parmi O et CH₂O, de préférence O, où L, dans le cas où Q signifierait 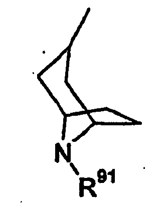 représente O.

8. Composés selon la revendication 7, où le groupe L-Q présente les significations suivantes 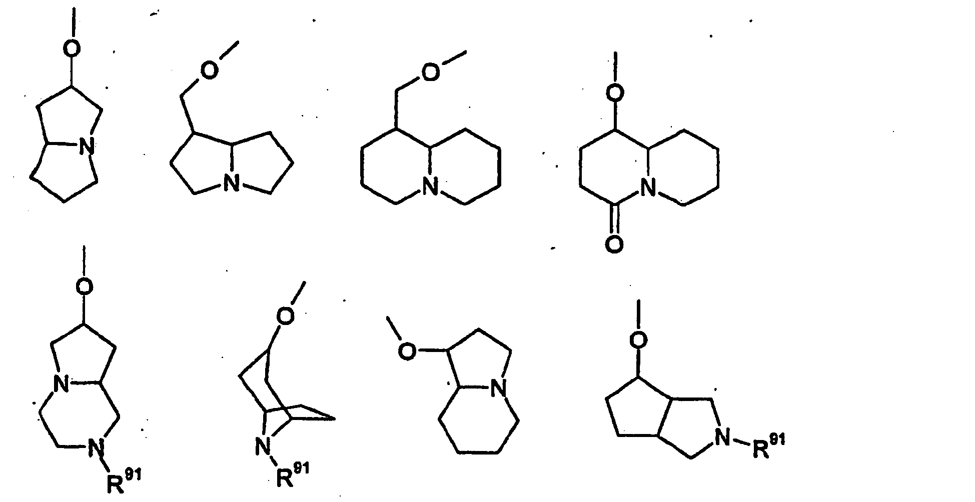 de préférence 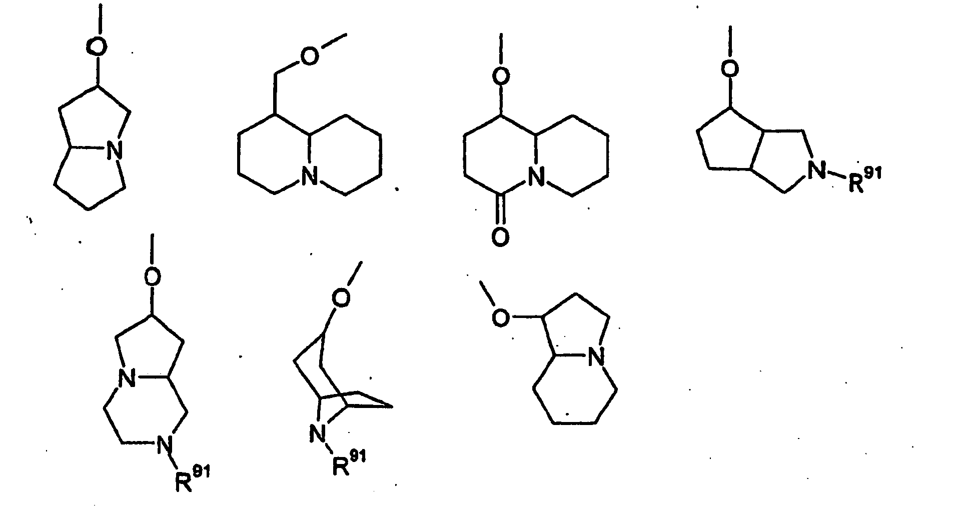 de manière particulièrement préférée 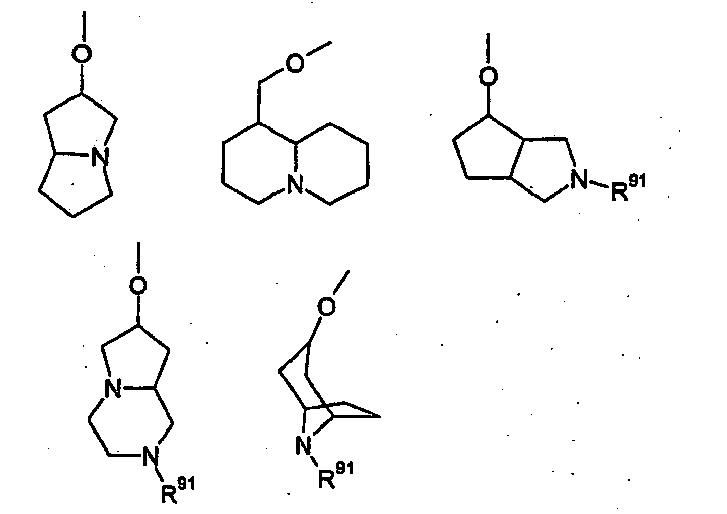 de manière tout particulièrement préférée 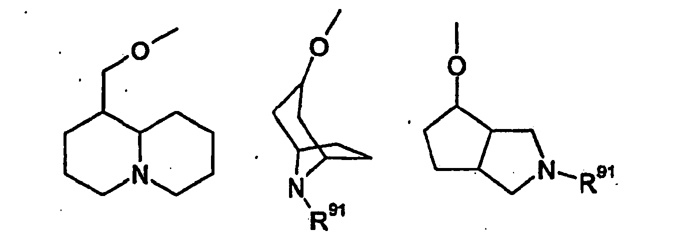
R91 signifie H, (C₁-C₄)-alcoxy-(C₁-C₄)-alkyle, (C₁-C₆)-alkyle, (C₂-C₆) -alcényle, (C₂-C₆)-alcynyle, CO (R92 ) , (CR93R94)_{o'}-R95, CO(CR93R94)_{p'}-R96 ; de préférence H, (C₁-C₆)-alkyle, (CR93R94)_{o'}-R95 ;
R92 signifie H, (C₁-C₈)-alkyle ;
R93, R94 signifient, indépendamment l'un de l'autre H, (C₁-C₈)-alkyle, OH, (C₃-C₈)-cycloalkyle, (C₁-C₄)-alcoxy-(C₁-C₄)-alkyle, de préférence H, (C₁-C₈)-alkyle;
o', p' valent, indépendamment l'un de l'autre 0, 1, 2, 3, 4, 5, 6, de préférence 0, 1, 2, 3 ;
R95, R96 signifient, indépendamment l'un de l'autre OH, O- (C₁-C₈) -alkyle, CON(R97) (R98) , N(R99)CO(R100), N(R101) (R102), CO₂(R103), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈) -alkyle, O- (C₁-C₈)-alkyle, CO(R104), oxo, OH ; de préférence, indépendamment l'un de l'autre, OH, O-(C₁-C₈)-alkyle, CON(R97)(R98), un système cyclique de 4-6 chaînons comprenant 0 à 2 hétéroatomes choisis dans le groupe formé par N et O, qui peut être substitué par un ou plusieurs des substituants suivants : F, Cl, Br, CF₃, (C₁-C₈)-alkyle, O- (C₁-C₈)-alkyle, CO(R104), oxo, OH, le système cyclique étant de manière particulièrement préférée non substitué ;
R97, R98, R99, R100, R103, R104 signifient, indépendamment l'un de l'autre H, (C₁-C₈)-alkyle ;
ou
R97 et R98 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R101, R102 signifient, indépendamment l'un de l'autre H, (C₁-C₆)-alkyle, (C₁-C₄)-alcoxy-(C₁-C₄)-alkyle, (C₂-C₆)-alcényle, (C₂-C₆)-alcynyle, CO(R105), (CR106R107)_{q'}-R108, CO(CR109R110)_{r'}-R111 ; ou R101 et R102 forment ensemble avec l'atome d'azote auquel ils sont liés, un cycle monocyclique, bicyclique ou spirocyclique de 4 à 10 chaînons, qui contient, outre l'atome d'azote, 0 à 3 hétéroatomes supplémentaires choisis dans le groupe formé par N, O et S et qui peut en outre être substitué par un ou plusieurs des substituants suivants : F, Cl, Br, CF₃, O-(C₁-C₈)-alkyle, (C₁-C₆)-alkyle, CO(R112), oxo, OH, (C₁-C₄)-alcoxy- (C₁-C₄)-alkyle, hydroxy-(C₁-C₄)-alkyle, CON(R113)(R114), N(R115)CO(R116), N(R117)(R118), CO₂(R119), SO₂Me ;
R105, R106, R107, R108, R109, R110, R112, R113, R114, R115, R116, R117, R118, R119 signifient, indépendamment l'un de l'autre H, (C₁-C₈)-alkyle ;
ou
R117 et R118 forment éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
q', r' valent, indépendamment l'un de l'autre 0, 1, 2, 3, 4, 5, 6 ;
R108, R111 signifient indépendamment l'un de l'autre OH, O-(C₁-C₈)-alkyle, CON(R120) (R121), N(R122)CO(R123), N(R124)(R125), CO₂(R126), SO₂Me, CN, un système cyclique de 3-10 chaînons comprenant 0 à 3 hétéroatomes choisis dans le groupe formé par N, O et S, qui peut être substitué par un ou plusieurs des substituants suivants : F, Cl, Br, CF₃, (C₁-C₈)-alkyle, O-(C₁-C₈)-alkyle, CO(R127), oxo, OH ;
R120, R121, R122, R123, R124, R125, R126, R127 signifient, indépendamment l'un de l'autre H, (C₁-C₈)-alkyle ;
ou
R120 et R121, R124 et R125 forment indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre.

9. Composés de formule I selon l'une quelconque des revendications 1 à 8, **caractérisés en ce que**
A, B, D, G signifient, indépendamment l'un de l'autre N ou C(R3) et le nombre total d'atomes d'azote dans ce cycle est de 0-2, de préférence 0
ou 1, de manière particulièrement préférée 0.

10. Composés de formule I selon l'une quelconque des revendications 1 à 8, où
K signifie O, OCH₂, CH₂O, S, SO, SO₂, N (R35) , N(R36)CO, CON(R37), (C(R38) (R39))ᵥ, CO, (R31)C=C(R32), C=C, SCH₂, SO₂CH₂, de préférence OCH₂, CH₂O, N (R36) CO, CON (R37) , (C(R38)(R39))₂, (R31)C=C(R32), C≡C, SCH₂, SO₂CH₂, de manière particulièrement préférée OCH₂, CH₂O, CON(R37), C=C, SCH₂ ; où
v vaut 1, 2, 3, de préférence 2 ;
R31, R32, R35, R36, R37, R38, R39 signifient, indépendamment l'un de l'autre H, (C₁-C₈)-alkyle.

11. Composés selon l'une quelconque des revendications 1 à 9, où
A, B, D, G
signifient C(R3) ;
R3 signifie H, F, Cl, Br, CF₃, CN, O-(C₁-C₆)-alkyle, O-(C₁-C₄)-alcoxy-(C₁-C₄)-alkyle, S-(C₁-C₆)-alkyle, (C₁-C₆)-alkyle, (C₀-C₈)-alkylénaryle, O-(C₀-C₈)-alkylénaryle, N(R4)(R5), SO₂-CH₃, CON(R6)(R7), N(R8)CO(R9), CO(R12), (CR13R14)ₓ-O(R15) ; de préférence H, F, C1, Br, CF₃, CN, O-(C₁-C₆)-alkyle, (C₁-C₆) -alkyle, SO₂-CH₃, CON(R6) (R7), N(R8)CO(R9), CO(R12), (CR13R14)ₓ-O(R15), de manière particulièrement préférée H, F, Cl, CF₃, CN, (C₁-C₆)-alkyle, (C(R13)(R14))ₓ-O(R15) ; de manière tout particulièrement préférée H, F, (C₁-C₆)-alkyle ; de manière encore plus particulièrement préférée H, F, CH₃ ; de manière particulièrement préférée H ;
R4, R5, R6, R7, R8 signifient, indépendamment l'un de l'autre H, (C₁-C₈)-alkyle ;
ou
R4 et R5, R6 et R7 forment, indépendamment l'un de l'autre, éventuellement, ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R9, R12 signifient, indépendamment l'un de l'autre H, (C₁-C₈)-alkyle ;
R13, R14 signifient H ;
R15 signifie H, (C₁-C₆)-alkyle ;
x vaut 0, 1, 2, de préférence 0, 1, de manière particulièrement préférée 1.

12. Composés selon l'une quelconque des revendications 1 à 11, **caractérisés en ce que** X signifie une liaison ou N(R16), où R16 signifie H ou (C₁-C₈)-alkyle.

13. Médicament contenant un ou plusieurs des composés de formule I selon l'une ou plusieurs des revendications 1 à 12.

14. Médicament contenant un ou plusieurs des composés de formule I selon l'une ou plusieurs des revendications 1 à 12 et une ou plusieurs substances actives, qui ont des effets favorables sur les troubles du métabolisme ou les maladies qui y sont associées.

15. Médicament contenant un ou plusieurs des composés de formule I selon l'une ou plusieurs des revendications 1 à 12 et un ou plusieurs antidiabétiques.

16. Médicament contenant un ou plusieurs des composés de formule I selon l'une ou plusieurs des revendications 1 à 12 et un ou plusieurs modulateurs de lipides.

17. Utilisation des composés de formule I selon l'une ou plusieurs des revendications 1 à 12 pour la préparation d'un médicament destiné au traitement et/ou à la prévention de troubles du métabolisme d'acides gras et de troubles de l'utilisation du glucose.

18. Utilisation des composés de formule I selon l'une ou plusieurs des revendications 1 à 12 pour la préparation d'un médicament destiné au traitement et/ou à la prévention de troubles dans lesquels la résistance à l'insuline joue un rôle.

19. Utilisation des composés de formule I selon l'une ou plusieurs des revendications 1 à 12 pour la préparation d'un médicament destiné au traitement et/ou à la prévention du diabète sucré et des maladies secondaires qui y sont liées.

20. Utilisation des composés de formule I selon l'une ou plusieurs des revendications 1 à 12 pour la préparation d'un médicament destiné au traitement et/ou à la prévention de dyslipidémies et de ses conséquences.

21. Utilisation des composés de formule I selon l'une ou plusieurs des revendications 1 à 12 pour la préparation d'un médicament destiné au traitement et/ou à la prévention d'états qui sont associés au syndrome métabolique.

22. Utilisation des composés de formule I selon l'une ou plusieurs des revendications 1 à 12 pour la préparation d'un médicament destiné au traitement et/ou à la prévention de l'obésité et des maladies secondaires qui y sont liées.

23. Utilisation des composés selon l'une ou plusieurs des revendications 1 à 12 en combinaison avec au moins une autre substance active pour la préparation d'un médicament destiné au traitement et/ou à la prévention de troubles du métabolisme des acides gras et de troubles de l'utilisation du glucose.

24. Utilisation des composés selon l'une ou plusieurs des revendications 1 à 12 en combinaison avec au moins une autre substance active pour la préparation d'un médicament destiné au traitement et/ou à la prévention de troubles dans lesquels la résistance à l'insuline joue un rôle.

25. Procédé pour la préparation d'un médicament contenant un ou plusieurs des composés selon l'une ou plusieurs des revendications 1 à 12, **caractérisé en ce que** la substance active est mélangée avec un support pharmaceutiquement approprié et ce mélange est amené dans une forme appropriée pour l'administration.
